# EUROPEAN PATENT APPLICATION

(11) **EP 4 424 670 A1**
(43) Date of publication of application: **04.09.2024**
(21) Application number: 22910116.7
(22) Date of filing: 22.12.2022
(51) Int. Cl.: C07D 211/48, C07D 309/14, A61K 9/50, A61K 9/51, A61K 47/18

(54) **LIPID COMPOUND AND LIPID NANOPARTICLE COMPOSITION**

(30) Priority: 23.12.2021 CN 202111593164
(71) Applicant: Suzhou Abogen Biosciences Co., Ltd., Suzhou, Jiangsu 215123 (CN)
(72) Inventor: WANG, Xiulian, SUZHOU Jiangsu 215123 (CN)
(74) Representative: Prüfer & Partner mbB Patentanwälte · Rechtsanwälte
(86) International application number: PCT/CN2022/140890
(87) International publication number: WO 2023/116804

(57) **Abstract**

The present invention relates to a lipid compound and a lipid nanoparticle composition, and specifically relates to a lipid composition which can be used in combination with other lipid components, such as neutral lipids, cholesterol, and polymer-conjugated lipids, to form lipid nanoparticles for delivery of therapeutic agents, such as nucleic acid molecules, for therapeutic or prophylactic purposes. The present invention further provides a lipid nanoparticle composition comprising the lipid compound.

## Description

### TECHNICAL FIELD

The present invention relates generally to a lipid compound that can be used in combination with other lipid components (e.g., neutral lipids, cholesterol, and polymer-conjugated lipids) to form lipid nanoparticles that are used for delivery of therapeutic agents (e.g., nucleic acid molecules, including nucleic acid mimetics such as Locked Nucleic Acids (LNA), Peptide Nucleic Acids (PNA), and morpholino oligonucleotides) both intracellular and extracellular, for therapeutic or prophylactic purposes, including vaccination.

### BACKGROUND ART

Therapeutic nucleic acids have the potential to completely change vaccination, gene therapy, protein replacement therapy, and other treatments for genetic diseases. Since the first clinical studies on therapeutic nucleic acids began in 2000s, significant progress has been made through the design of nucleic acid molecules and improvements in their delivery methods. However, nucleic acid therapeutic agents still face several challenges, including low cell permeability, and high sensitivity to degradation of certain nucleic acid molecules (including RNA). Therefore, there is a need to develop new nucleic acid molecules, as well as methods and compositions related thereto, to facilitate their extracellular or intracellular delivery for therapeutic and/or prophylactic purposes.

### SUMMARY

In one embodiment, the present invention provides a lipid compound, including a pharmaceutically acceptable salt or stereoisomer thereof, that can be used alone or in combination with other lipid components such as neutral lipid, charged lipid, steroid (including, for example, all steroids) and/or analogs thereof, and/or lipid conjugated to a polymer, and/or the polymers, to form lipid nanoparticles for use in the delivery of a therapeutic agent (e.g., nucleic acid molecules, including, for example, nucleic acid mimetics such as Locked Nucleic Acids (LNA), Peptide Nucleic Acids (PNA), and morpholine ring oligonucleotides). In some cases, the lipid nanoparticles are used for delivering nucleic acids, such as antisense and/or messenger RNA. The present invention also provides a method of using such lipid nanoparticles to treat various diseases or conditions (such as diseases or conditions caused by infectious entity and/or protein deficiencies).

In one embodiment, the present invention provides a compound represented by a Formula (I): 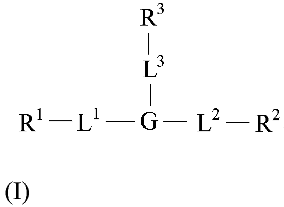 or a pharmaceutically acceptable salt or stereoisomer thereof, wherein G, L¹, L², L³, R¹, R² and R³ are as defined herein or elsewhere.

In one embodiment, the present invention provides a nanoparticle composition including the compound provided herein and a therapeutic or prophylactic agent. In some embodiment, the therapeutic or prophylactic agent includes at least one mRNA encoding an antigen or fragment or epitope thereof.

It is obvious to those skilled in the art that the present invention makes a detailed description of the specific embodiments. However, it is to be understood that it is made only in an illustrative and not restrictive manner. Various changes and modifications within the scope of the present invention will be obvious to those skilled in the art.

### DETAILED DESCRIPTION

### General techniques

The technologies and methods described or cited in the present invention include conventional methods that are generally easily understood or commonly used by those skilled in the art, such as methods described in Molecular Cloning: A Laboratory Manual (3d ed. 2001); Current Protocols in Molecular Biology (Ausubel et al. eds., 2003).

### Terms

Unless otherwise stated, all technical and scientific terms used herein have the same meanings as are generally understood by those of ordinary skill in the art. For the purposes of this specification, the following terms will be used for describing and, where appropriate, the terms used in the singular will also include the plural and vice versa. The public contents of all patents and other publications cited herein are incorporated herein by reference in their entirety. In the event of a conflict between any description of the terms herein and any document incorporated herein by reference, the description and interpretation of the terms set forth below shall prevail.

Unless otherwise stated herein, the term "lipid" refers to a group of organic compounds that include, but are not limited to, esters of fatty acids and are characterized by poor solubility in water, but being soluble in many non-polar organic compounds. Although lipid generally has poor solubility in water, certain classes of lipids (e.g., lipids modified by polar groups such as DMG-PEG2000) have limited water solubility and can be dissolved in water under certain conditions. Known types of lipids include biomolecules, such as fatty acids, waxes, sterols, fat-soluble vitamins, monoglycerides, diglycerides, triglycerides, and phospholipids. Lipids can generally be divided into at least three categories: (1) "simple lipids", including fats and oils and waxes; (2) "compound lipids", including phospholipids and glycolipids (e.g., DMPE-PEG2000); and (3) "derived lipids", such as steroids, etc. In addition, as used herein, the lipids also include lipoid compounds. The term "lipoid compound", also abbreviated as "lipoids", refers to lipid-like compounds such as amphiphilic compounds that have lipid-like physical properties.

The term "lipid nanoparticles" or "LNP" refers to particles having a nanometer scale (nm) (e.g., 1 nm to 1,000 nm) that comprise one or more types of lipid molecules. The LNP provided herein may further comprise at least one non-lipid payload molecule (e.g., one or more nucleic acid molecules). In some embodiments, the LNP comprises non-lipid payload molecules that are partially or fully encapsulated within a lipid shell. In particular, in some embodiments, the payload refers to negatively charged molecules (e.g., mRNA encoding viral protein), and the lipid component of the LNP comprises at least one cationic lipid. It may be expected that the cationic lipid may interact with the negatively charged payload molecules and promote incorporation and/or encapsulation of the payload into the LNP during LNP formation. As provided herein, other lipids that may form part of the LNP include, but are not limited to, neutral lipids and charged lipids, such as steroids, polymer-conjugated lipids, and various zwitterionic lipids. In certain embodiments, the LNP according to the present invention comprises one or more lipids of the Formula (I) (and sub-formulas thereof) described herein.

The term "cationic lipid" refers to a lipid that is positively charged at any pH or hydrogen ion activity of the environment in which it is located, or a lipid that is capable of being positively charged in response to pH or hydrogen ion activity of the environment in which it is located (such as the environment in which it is intended to be used). Thus, the term "cations" encompasses the range of "permanent cations" and "cationizable". In certain embodiments, the positive charge in the cationic lipid is derived from quaternary nitrogen atom. In certain embodiments, the cationic lipid includes a zwitterionic lipid that is positively charged in the environment in which it is intended to be administrated (e.g., at physiological pH). In certain embodiments, the cationic lipid is one or more lipids of the Formula (I) (and sub-formulas thereof) described herein.

The term "polymer-conjugated lipid" or "polymer-conjugate lipid" refers to molecules comprising both a lipid moiety and a polymer moiety. An example of the polymer-conjugated lipid is pegylated lipid (PEG-lipid), wherein the polymer moiety comprises polyethylene glycol.

The term "neutral lipids" encompasses any lipid molecule present in an uncharged form or in a neutral zwitterionic form at a selected pH. In some embodiments, the selected useful pH value or range corresponds to the pH conditions of the environment in which the lipids are intended to be used, such as physiological pH. As a non-limiting example, neutral lipids that can be used in combination with the present disclosure include, but are not limited to, phosphatidylcholine, such as 1,2-distearoyl-sn-glycero-3-phosphocholine (DSPC), 1,2-dipalmitoyl-sn-glycero-3-phosphocholine (DPPC), 1,2-dimyristoyl-sn-glycero-3-phosphocholine (DMPC), 1-palmitoyl-2-oleoyl-sn-glycero-3-phosphocholine (POPC), 11,2-dioleoyl-sn-glycero-3-phosphocholine (DOPC), phosphatidylethanolamine such as 1,2-dioleoyl-sn-glycero-3-phosphoethanolamine (DOPE), 2-(((2,3-bis(oleoyloxy)propyl))dimethylammonium phosphate)ethylhydrogen (DOCP), sphingomyelin (SM), ceramide, steroids such as sterols and derivatives thereof. The neutral lipids may be synthetic or derived (isolated or modified) from natural sources or compounds.

The term "charged lipids" encompasses any lipid molecule present in a positively or negatively charged form at a selected pH or range. In some embodiments, the selected pH or range corresponds to the pH conditions of the environment in which the lipids are intended to be used, such as physiological pH. As a non-limiting example, charged lipids that can be used in combination with the present invention include, but are not limited to, phosphatidylserine, phosphatidic acid, phosphatidylglycerol, phosphatidylinositol, sterol hemisuccinate, dialkyltrimethylammonium-propane (e.g., DOTAP, DOTMA), dialkyldimethylaminopropane, ethylphosphorylcholine, dimethylaminoethane carbamoyl sterol (e.g., DC-Chol), 1,2-dioleoyl-sn-glycero-3-phospho-L-serine sodium salt (DOPS-Na), 1,2-dioleoyl-sn-glycero-3-phospho-(1'-rac-glycerol) sodium salt (DOPG-Na), and 1,2-dioleoyl-sn-glycero-3-phosphate sodium salt (DOPA-Na). The charged lipids provided herein may be synthetic or derived (isolated or modified) from natural sources or compounds.

As described herein, unless otherwise stated, the term "alkyl" refers to a linear or branched hydrocarbon chain group consisting of only saturated carbon and hydrogen atoms. In one embodiment, the alkyl group has, for example, 1 to 24 carbon atoms (C₁-C₂₄ alkyl), 4 to 20 carbon atoms (C₄-C₂₀ alkyl), 10 to 20 carbon atoms (C₁₀-C₂₀ alkyl), 6 to 16 carbon atoms (C₆-C₁₆ alkyl), 6 to 9 carbon atoms (C₆-C₉ alkyl), 1 to 15 carbon atoms (C₁-C₁₅ alkyl), 1 to 12 carbon atoms (C₁-C₁₂ alkyl), 1 to 8 carbon atoms (C₁-C₈ alkyl), or 1 to 6 carbon atoms (C₁-C₆ alkyl), and is linked to the remainder of the molecules by a single bond. Examples of the alkyl groups include, but are not limited to, methyl, ethyl, propyl, 1-methylethyl (isopropyl), n-butyl, n-amyl, 1,1-dimethylethyl (tert-butyl), 3-methylhexyl, 2-methylhexyl, and the like. Unless otherwise stated, the alkyl is optionally substituted.

As described herein, unless otherwise stated, the term "alkenyl" refers to a linear or branched hydrocarbon chain group consisting of only carbon and hydrogen atoms, which contains one or more carbon-carbon double bonds. As understood by those skilled in the art, the term "alkenyl" also includes groups having "cis" and "trans" configurations, or "E" and "Z" configurations. In one embodiment, the alkenyl group has, for example, 2 to 24 carbon atoms (C₂-C₂₄ alkenyl), 4 to 20 carbon atoms (C₄-C₂₀ alkenyl), 6 to 16 carbon atoms (C₆-C₁₆ alkenyl), 6 to 9 carbon atoms (C₆-C₉ alkenyl), 2 to 15 carbon atoms (C₂-C₁₅ alkenyl), 2 to 12 carbon atoms (C₂-C₁₂ alkenyl), 2 to 8 carbon atoms (C₂-C₈ alkenyl), or 2 to 6 carbon atoms (C₂-C₆ alkenyl), and is linked to the remainder of the molecules by a single bond. Examples of alkenyl groups include, but are not limited to, vinyl, prop-1-enyl, but-1-enyl, pent-1-enyl, pent-1,4-dienyl, and the like. Unless otherwise stated, the alkenyl is optionally substituted.

As described herein, unless otherwise stated, the term "alkynyl" refers to a linear or branched hydrocarbon chain group consisting of only carbon and hydrogen atoms, which comprises one or more carbon-carbon triple bonds. In one embodiment, the alkynyl group has, for example, 2 to 24 carbon atoms (C₂-C₂₄ alkynyl), 4 to 20 carbon atoms (C₄-C₂₀ alkynyl), 6 to 16 carbon atoms (C₆-C₁₆ alkynyl), 6 to 9 carbon atoms (C₆-C₉ alkynyl), 2 to 15 carbon atoms (C₂-C₁₅ alkynyl), 2 to 12 carbon atoms (C₂-C₁₂ alkynyl), 2 to 8 carbon atoms (C₂-C₈ alkynyl), or 2 to 6 carbon atoms (C₂-C₆ alkynyl), and is linked to the remainder of the molecules by a single bond. Examples of alkynyl groups include, but are not limited to, ethynyl, propynyl, butynyl, pentynyl, and the like. Unless otherwise stated, the alkynyl is optionally substituted.

As described herein, unless otherwise stated, the term "alkylene" or "alkylene chain" refers to a linear or branched divalent hydrocarbon chain linking the remainder of the molecules to the group consisting of only saturated carbon and hydrogen. In one embodiment, the alkylene has, for example, 1 to 24 carbon atoms (C₁-C₂₄ alkylene), 1 to 15 carbon atoms (C₁-C₁₅ alkylene), 1 to 12 carbon atoms (C₁-C₁₂ alkylene), 1 to 8 carbon atoms (C₁-C₈ alkylene), 1 to 6 carbon atoms (C₁-C₆ alkylene), 2 to 4 carbon atoms (C₂-C₄ alkylene), or 1 to 2 carbon atoms (C₁-C₂ alkylene). Examples of alkylene include, but are not limited to, methylene, ethylene, propylene, n-butene, and the like. The alkylene chain is linked to the remainder of the molecules by a single bond, and to the free radical group by a single bond. The linkage of the alkylene chain to the remainder of the molecule and to the free radical group may be through one carbon or any two carbons in the chain. Unless otherwise stated, the alkylene chain is optionally substituted.

As described herein, unless otherwise stated, the term "alkenylene" refers to a linear or branched divalent hydrocarbon chain linking the remainder of the molecules to a free radical group consisting of only carbon and hydrogen, the free radical group including one or more carbon-carbon double bonds. In one embodiment, the alkenylene has, for example, 2 to 24 carbon atoms (C₂-C₂₄ alkenylene), 2 to 15 carbon atoms (C₂-C₁₅ alkenylene), 2 to 12 carbon atoms (C₂-C₁₂ alkenylene), 2 to 8 carbon atoms (C₂-C₈ alkenylene), 2 to 6 carbon atoms (C₂-C₆ alkenylene), or 2 to 4 carbon atoms (C₂-C₄ alkenylene). Examples of alkenylene include, but are not limited to, vinylidene, propenylidene, n-butenyl, and the like. The alkenylene is linked to the remainder of the molecules by a single bond or a double bond, and to the free radical group by a single bond or a double bond. The linkage of the alkenylene to the remainder of the molecule and to the free radical group may be by one carbon or any two carbons in the chain. Unless otherwise stated, the alkenylene is optionally substituted.

As described herein, unless otherwise stated, the term "cycloalkyl" refers to a saturated non-aromatic monocyclic or polycyclic hydrocarbon group consisting of only carbon and hydrogen atoms. The cycloalkyl may include a fused or bridged ring system. In one embodiment, the cycloalkyl has, for example, 3 to 15 ring carbon atoms (C₃-C₁₅ cycloalkyl), 3 to 10 ring carbon atoms (C₃-C₁₀ cycloalkyl), or 3 to 8 ring carbon atoms (C₃-C₈ cycloalkyl). The cycloalkyl is linked to the remainder of the molecules by a single bond. Examples of monocyclic cycloalkyl include, but are not limited to, cyclopropyl, cyclobutyl, cyclopentyl, cyclohexyl, cycloheptyl, and cyclooctyl. Examples of polycyclic cycloalkyl groups include, but are not limited to, adamantyl, norbornyl, decahydroalkyl, 7,7-dimethyl-bicyclo[2.2.1]heptyl, and the like. unless otherwise stated, the cycloalkyl is optionally substituted.

As described herein, unless otherwise stated, the term "cycloalkylene" refers to a divalent cycloalkyl group. unless otherwise stated, the cycloalkylene is optionally substituted.

As described herein, unless otherwise stated, the term "cycloalkenyl" refers to a non-aromatic monocyclic or polycyclic hydrocarbon group consisting of only carbon and hydrogen atoms and including one or more carbon-carbon double bonds. The cycloalkenyl group may include a fused or bridged ring system. In one embodiment, the cycloalkenyl group has, for example, 3 to 15 ring carbon atoms (C₃-C₁₅ cycloalkenyl), 3 to 10 ring carbon atoms (C₃-C₁₀ cycloalkenyl), or 3 to 8 ring carbon atoms (C₃-C₈ cycloalkenyl). The cycloalkenyl group is linked to the remainder of the molecules by a single bond. Examples of monocyclic cycloalkenyl groups include, but are not limited to, cyclopropenyl, cyclobutenyl, cyclopentenyl, cyclohexenyl, cycloheptenyl, cyclooctylene, and the like. Unless otherwise stated, the cycloalkenyl is optionally substituted.

As described herein, unless otherwise stated, the term "cycloalkenylene" refers to a divalent cycloalkenyl. Unless otherwise stated, the cycloalkenylene is optionally substituted.

As described herein, unless otherwise stated, the term "heterocyclyl" refers to a non-aromatic monocyclic or polycyclic moiety comprising one or more (e.g., one, one or two, one to three, or one to four) heteroatoms independently selected from nitrogen, oxygen, phosphorus, and sulfur. The heterocyclyl group may be attached to the main structure at any heteroatom or carbon atom. The heterocyclyl group may be a monocyclic, bicyclic, tricyclic, tetracyclic, or other polycyclic system, wherein the polycyclic system may be a fused- , bridged-, or spiro- ring system. The heterocyclic polycyclic system may include one or more heteroatoms in one or more rings. The heterocyclyl group may be saturated or partially unsaturated. The saturated heterocycloalkyl group may be referred to as "heterocycloalkyl". If the heterocyclyl group includes at least one double bond, the partially unsaturated heterocycloalkyl group may be referred to as "heterocycloalkenyl"; and if the heterocyclyl group includes at least one triple bond, it may be referred to as "heterocycloalkynyl". In one embodiment, the heterocyclyl group has, for example, 3 to 18 ring atoms (3 to 18 membered heterocyclyl group), 4 to 18 ring atoms (4 to 18 membered heterocyclyl group), 5 to 18 ring atoms (5 to 18 membered heterocyclyl group), 4 to 8 ring atoms (4 to 8 membered heterocyclyl group), or 5 to 8 ring atoms (5 to 8 membered heterocyclyl group). According to the present invention, whenever it occurs, the numerical range such as "3 to 18" refers to each integer within a given range. For example, "3 to 18 membered heterocyclyl group" refers to that the heterocyclyl group may be composed of 3 ring atoms, 4 ring atoms, 5 ring atoms, 6 ring atoms, 7 ring atoms, 8 ring atoms, 9 ring atoms, 10 ring atoms, and up to 18 ring atoms, etc. The heterocyclyl group includes, but is not limited to, imidazolyl, imidazolidinyl, oxazolyl, oxazolidinyl, thiazolyl, thiazolidinyl, pyrazolidinyl, pyrazolyl, isoxazolidinyl, isoxazolyl, isothiazolidinylpyrrolyl, isothiazolyl, furanyl, furyl, piperidinyl, quinolinyl, and isoquinolinyl. Unless otherwise stated, the heterocyclyl group is optionally substituted.

As described herein, unless otherwise stated, the term "heterocyclylene" is a divalent heterocyclyl group. Unless otherwise stated, the heterocyclylene is optionally substituted.

As described herein, unless otherwise stated, the term "aryl" refers to a monocyclic aromatic group and/or a polycyclic monovalent aromatic group comprising at least one aromatic hydrocarbon ring. In certain embodiments, the aryl has 6 to 18 ring carbon atoms (C6-C18 aryl), 6 to 14 ring carbon atoms (C6-C14 aryl), or 6 to 10 ring carbon atoms (C6-C10 aryl). Examples of aryl include, but are not limited to, phenyl, naphthyl, fluorenyl, azulenyl, anthryl, phenanthryl, pyrenyl, biphenyl, and terphenyl. The term "aryl" also refers to a bicyclic, tricyclic, or other polycyclic hydrocarbon ring, where at least one ring is aromatic and the other rings may be saturated, partially unsaturated, or aromatic, such as dihydronaphthyl, indenyl, indanyl, or tetrahydronaphthyl (tetralyl). Unless otherwise stated, the aryl is optionally substituted.

As described herein, unless otherwise stated, the term "arylene" is a divalent aryl. Unless otherwise stated, the arylene is optionally substituted.

As described herein, unless otherwise stated, the term "heteroaryl" refers to a monocyclic aromatic group and/or a polycyclic aromatic group containing at least one aromatic ring, where the at least one aromatic ring contains one or more heteroatoms independently selected from one to three or one to four of O, S, and N. The heteroatoms in the heteroaryl may be linked to the main structure at any carbon atom. In certain embodiments, the heteroaryl has 5 to 20, 5 to 15, or 5 to 10 ring atoms. The term "heteroaryl" also refers to a bicyclic, tricyclic, or other polycyclic ring, of which at least one ring is aromatic and the other rings may be saturated, partially unsaturated, or aromatic, the examples where at least one aromatic ring includes one or more monocyclic heteroaryls includes, but are not limited to, pyrrolyl, pyrazolyl, pyrazolinyl, imidazolyl, oxazolyl, isoxazolyl, thiazolyl, thiadiazolyl, isothiazolyl, furyl, thienyl, oxadiazolyl, pyrazinyl, pyrimidinyl, pyridazinyl, and triazinyl. Examples of bicyclic heteroaryl include, but are not limited to, indolyl, benzothiazolyl, benzoxazolyl, benzothienyl, quinolinyl, tetrahydroisoquinolinyl, isoquinolinyl, benzimidazolyl, benzopyranyl, indolizinyl, benzofuryl, isobenzofuryl, oxynaphthyl, furopyridinyl, thienopyridyl, dihydroisoindolyl, and tetrahydroquinolinyl. Examples of tricyclic heteroaryl include, but are not limited to, carbazolyl, benzoindolyl, phenanthroline, acridinium, phenanthryl, and xanthine. Unless otherwise stated, the heteroaryl is optionally substituted.

As described herein, unless otherwise stated, the term "heteroarylene" is a divalent heteroaryl. Unless otherwise stated, heteroarylene is optionally substituted.

When the groups described herein are "substituted", they may be substituted with any suitable one or more substituents. Illustrative examples of substituents include, but are not limited to, those shown in the exemplary compounds and embodiments provided herein, as well as halogen atoms such as F, Cl, Br, or I; cyano; oxo (=O); hydroxy (-OH); alkyl; alkenyl, alkynyl, cycloalkyl, aryl - (C = O)OR'; -O(C=O)R'; -C(=O)R'; -S(O)ₓR'; -S-SR'; -C(= O)SR'; -SC(= O)R'; -NR'R'; - NR'C(=O)R'; -C(=O)NR'R'; -NR'C(= O)NR'R'; -OC(= O)NR'R'; -NR'C(= O)OR'; - NR'S(O)ₓNR'R'; -NR'S(O)ₓR'; and -S(O)ₓNR'R', wherein R' is independently at each occurrence H, C₁-C₁₅ alkyl or cycloalkyl, and x is 0, 1, or 2. In some embodiments, the substituent is C₁-C₁₂ alkyl. In other embodiments, the substituent is cycloalkyl. In other embodiments, the substituent is a halogen group, such as fluoro. In other embodiments, the substituent is an oxo group. In other embodiments, the substituent is hydroxy. In other embodiments, the substituent is alkoxy (-OR'). In other embodiments, the substituent is carboxy. In other embodiments, the substituent is amino (-NR'R').

As described herein, unless otherwise stated, the term "optional" or "optionally" (e.g., optionally substituted) refers to that an event/situation subsequently described may or may not occur, and the description includes instances of occurence of the event or situation and the instances of not occurrence of the event or situation. For example, the "optionally substituted alkyl" refers to that alkyl may or may not be substituted, and the description includes substituted alkyl and unsubstituted alkyl.

The "prodrug" refers to a compound that may be converted to a biologically active compound under physiological conditions or by solvolysis. Thus, the term "prodrug" refers to a metabolic precursor of a pharmaceutically acceptable biologically active compound. When administered to a subject in need thereof, the prodrug may be inactive, but converted to the biologically active compound of the present invention in vivo. The prodrug is typically converted rapidly in vivo to the parent biologically active compound of the present invention, e.g., by hydrolysis in blood. The prodrug compound typically provides the advantages of solubility, histocompatibility, or delayed release in mammalian organisms (see Bundgard, H., Design of Prodrugs (1985), pp. 7-9, 21-24 (Elsevier, Amsterdam)). Discussion of prodrugs is provided in Higuchi, T., et al., A.C.S. Symposium Series, Vol. 14, and Bioreversible Carriers in Drug Design, Ed. Edward B. Roche, American Pharmaceutical Association and Pergamon Press, 1987.

In some embodiments, the term "prodrugs" is also meant to include any carrier covalently bonded; when administered to a mammalian subject, the prodrugs release the active compounds of the present invention in vivo. The prodrugs of the compounds according to the present invention may be prepared by modifying the functional groups present in the compounds in such a way that the modification is by conventional procedures or by cleavage in vivo into the parent compounds of the present invention. The prodrugs include the following compounds of the present invention: hydroxyl, amino, or sulfydryl is bonded to any following group; when the prodrugs of the compounds according to the present invention is administered to a mammalian subject, the group cleaves to form a free hydroxyl, free amino, or free sulfydryl, respectively.

Examples of "prodrugs" include, but are not limited to, acetate, formate, and benzoate derivatives and the like of amide derivatives of alcohol or amine functional groups in the compounds provided herein.

As described herein, unless otherwise stated, the term "pharmaceutically acceptable salts" includes acid addition salts and base addition salts.

Examples of "pharmaceutically acceptable acid addition salts" include, but are not limited to, hydrochloric acid, hydrobromic acid, sulfuric acid, nitric acid, phosphoric acid, etc., and organic acids, such as, but are not limited to, acetic acid, 2,2-dichloroacetic acid, adipic acid, alginic acid, ascorbic acid, aspartic acid, benzenesulfonic acid, benzoic acid, 4-acetamidobenzoic acid, camphoric acid, camphor-10-sulfonic acid, capric acid, hexanoic acid, caprylic acid, carbonic acid, cinnamic acid, citric acid, cyclic amic acid, lauryl sulfuric acid, ethane-1,2-disulfonic acid, ethanesulfonic acid, 2-hydroxyethanesulfonic acid, formic acid, fumaric acid, galactanic acid, gentisic acid, glucoheptonic acid, gluconic acid, glucuronic acid, glutamic acid, glutaric acid, 2-oxoglutaric acid, glycerophosphoric acid, glycolic acid, hippuric acid, isobutyric acid, lactic acid, lactobionic acid, lauric acid, maleic acid, malic acid, malonic acid, mandelic acid, methanesulfonic acid, muconic acid, naphthalene-1,5-dicarboxylic acid, naphthalene-2-sulfonic acid, 1-hydroxy-2-naphthoic acid, nicotinic acid, oleic acid, orotic acid, oxalic acid, palmitic acid, cetylic acid, propionic acid, pyroglutamic acid, pyruvic acid, salicylic acid, 4-aminosalicylic acid, sebacic acid, stearic acid, succinic acid, tartaric acid, thiocyanic acid, p-toluenesulfonic acid, trifluoroacetic acid, undecylenic acid, etc.

Examples of "pharmaceutically acceptable base addition salts" include, but are not limited to, salts prepared by addition of an inorganic base or an organic base to a free acid compound. Salts derived from inorganic bases include, but are not limited to, sodium salts, potassium salts, lithium salts, ammonium salts, calcium salts, magnesium salts, iron salts, zinc salts, copper salts, manganese salts, aluminum salts, and the like. Preferably, the inorganic salts are ammonium salts, sodium salts, potassium salts, calcium salts, and magnesium salts. Salts derived from organic bases include, but are not limited to, primary, secondary, and tertiary amines, substituted amines (including naturally occurring substituted amines), cyclic amines, and salts of basic ion exchange resins: such as ammonia, isopropylamine, trimethylamine, diethylamine, triethylamine, tripropylamine, diethanolamine, ethanolamine, dealcoholization, 2-dimethylaminoethanol, 2-diethylaminoethanol, lysine, arginine, histidine, caffeine, procaine, hydrazinobenzidine, choline, betaine, benethamine, benzathine, ethylenediamine, glucosamine, methylglucosamine, theobromine, triethanolamine, purine, piperazine, piperidine, N-ethylpiperidine, polyamide resins, and the like. Preferably, the organic bases are isopropylamine, diethylamine, ethanolamine, trimethylamine, dicyclohexylamine, choline, and caffeine.

The compounds provided herein may comprise one or more asymmetric centers and thus may produce enantiomers, diastereomers and other stereoisomeric forms; amino acids may be defined as (R)- or (S)-, or as (D)- or (L)-, in an absolute stereochemical manner. Unless otherwise stated, the compounds provided herein are intended to include all these possible isomers, as well as their racemic and optically pure forms. Optically active (+) and (-), (R)- and (S)- or (D)- and (L)- isomers may be prepared using chiral synthons or chiral reagents, or resolved using conventional techniques, such as chromatography and fractional crystallization. Conventional techniques for preparing/isolating individual enantiomers include chiral synthesis from suitable optically pure precursors or resolution using racemates (or racemates of salts or derivatives) such as chiral High Pressure Liquid Chromatography (HPLC). When the compounds described herein comprise an olefinic double bond or other geometric asymmetric center, unless otherwise stated, the compounds are intended to include E and Z- geometric isomers. Also, all tautomeric forms are intended to be included.

As described herein, unless otherwise stated, the term "isomer" refers to different compounds having the same molecular formula. The "stereoisomer" is an isomer in which only atoms are arranged in a different manner in space. The "atropisomer" is a stereoisomer in which the rotation of atoms about a single bond is hindered. The "enantiomer" is a pair of mirrored stereoisomers that do not overlap each other. A mixture of any ratio of a pair of enantiomers may be referred to as a "racemic" mixture. The "diastereomer" is a stereoisomer having at least two asymmetric atoms, but not mirrored to each other.

The "stereoisomer" may also include E- and Z- isomers or mixtures thereof, and cis and trans isomers or mixtures thereof. In certain embodiments, the compounds described herein are isolated as E or Z-type isomers. In other embodiments, the compounds described herein are mixtures of E and Z-type isomers.

The "tautomer" refers to isomeric forms of compounds that are in equilibrium with each other. The concentration of the isomeric forms varies depending on the environment in which the compound is located, and may depend on whether the compound is solid or in a state of presence in an organic or aqueous solution.

The compounds described herein may comprise an atomic isotope of an unnatural moiety on one or more atoms. For example, the compounds may be radiolabeled with a radioisotope, such as tritium 3(³H), iodine-125(¹²⁵I), sulfur 35(³⁵S), or carbon 14(¹⁴C), or may be deuterium (²H), carbon 13(¹³C), or nitrogen 15(¹⁵N) isotope-enriched. As used herein, "isotopes" are isotope-enriched compounds. The term "isotope-enrichment" refers to atoms having an isotope composition different from the natural isotope composition of the atom. The term "isotope-enriched" may also refer to compounds containing at least one atom having an isotope composition different from the natural isotope composition of the atom. The term "isotopic composition" refers to the amount of each isotope present for a given atom. The radiolabeled and isotope-enriched compounds are useful as therapeutic agents, such as cancer therapeutics, research agents (e.g., binding assay agents), and diagnostic agents (e.g., in vivo imaging agents). All isotope variants of the compounds described herein, whether radioactive or not, are intended to be included within the scope of the embodiments provided herein. In some embodiments, isotopes of the compounds described herein are provided, e.g., isotopes are deuterium, carbon-13, and/or nitrogen 15 enriched. As used herein, "deuteration" refers to compounds in which at least one hydrogen (H) is substituted with deuterium (expressed as D or ²H), i.e., the compounds are deuterium-enriched at least one position.

It is to be noted that if there is a difference between the structure described herein and the name of the structure, the structure described has more weight.

As described herein, unless otherwise stated, the term "pharmaceutically acceptable carrier, diluent, or excipient" includes, but is not limited to, adjuvant, carrier, excipient, glidant, sweetener, diluent, preservative, dye/colorant, flavoring, surfactant, wetting agent, dispersant, suspending agent, stabilizer, isotonic agent, solvent, or emulsifier that has been approved by U.S. Food and Drug Administration for use in humans or livestock.

The term "composition" is intended to encompass products containing optionally specified components (e.g., mRNA molecules) in specified amounts.

The term "polynucleotide" or "nucleic acid" is interchangeable in the present invention, which refers to a polymer of nucleotides of any length, including, for example, DNA and RNA. The nucleotides may be deoxyribonucleotides, ribonucleotides, modified nucleotides or bases and/or analogs thereof, or may be any substrate incorporated into the polymer by DNA polymerase or RNA polymerase or by a synthetic reaction. The polynucleotide may include a modified nucleotide, such as a methylated nucleotide and analogs thereof. The nucleic acid may be in single-stranded or double-stranded form. As described herein, unless otherwise stated, the "nucleic acid" also includes nucleic acid mimics, such as Locked Nucleic Acids (LNA), Peptide Nucleic Acids (PNA) and morpholine ring oligonucleotides. As used herein, the "oligonucleotide" refers to a short synthetic polynucleotide, which is generally but not necessarily less than about 200 nucleotides in length. The term "oligonucleotide" and "polynucleotide" are not mutually exclusive. The description for the polynucleotide above is equally and completely applicable to oligonucleotides. Unless otherwise stated, the left end of any single-stranded polynucleotide sequence disclosed herein is 5' end; and the left direction of the double-stranded polynucleotide sequence is referred to as 5' direction. The direction of addition of the nascent RNA transcript from 5' to 3' is referred to as the transcription direction; the sequence region on the DNA strand having the same sequence as the RNA transcript at the 5' to 5' end of the RNA transcript is referred to as the "upstream sequence"; and the sequence region on the DNA strand having the same sequence as the RNA transcript at the 3' to 3' end is referred to as the 'downstream sequence'.

The "isolated nucleic acid" refers to a nucleic acid, e.g., which may be RNA, DNA or a mixed nucleic acid, that is substantially naturally isolated from other genomic DNA sequences and proteins or complexes such as ribosomes and polymerases, including natural sequence. The "isolated" nucleic acid molecule is a nucleic acid molecule that is isolated from other nucleic acid molecules of natural origin. Furthermore, the "isolated" nucleic acid molecule (e.g., an mRNA molecule) may be substantially free of other cellular materials or media when produced by recombinant techniques, or may be substantially free of chemical precursors or other chemicals when chemically synthesized. In a particular embodiment, one or more nucleic acid molecules encoding the antigens described herein are isolated or purified. The term includes nucleic acid sequences that have been removed from the environment in which they are naturally present, and includes recombinant or cloned DNA or RNA isolates and chemically synthesized analogs or analogs that are biosynthesized by heterologous systems. Substantially pure molecules may include isolated forms of the molecule.

The term "encoding nucleic acid" or a grammatical equivalent thereof includes: (a) a nucleic acid molecule that may transcribe an mRNA capable of being translated into peptide and/or polypeptide when in a native state or operated by a method known to those skilled in the art, and (b) the mRNA molecule itself. The antisense strand is a complementary sequence of the nucleic acid molecule, and a coding sequence may be deduced therefrom. The term "coding region" refers to a portion of the coding nucleic acid sequence that is translatable into peptide or polypeptide. The term "untranslated region" or "UTR" refers to a portion of the coding nucleic acid that is not translated into peptide or polypeptide. This depends on the orientation of the UTR relative to the coding region of the nucleic acid molecule, the UTR is referred to as 5'-UTR if the UTR is located at the 5' end of the coding region; and the UTR is referred to as 3'-UTR if the UTR is located at the 3' end of the coding region.

As described herein, the term "mRNA" refers to a messenger RNA molecule including one or more Open Reading Frames (ORF), which may be translated by a cell or organism to produce one or more peptide or protein products. A region comprising one or more ORF is referred to as the coding region of the mRNA molecule. In certain embodiments, the mRNA molecule further comprises one or more untranslated regions (UTRs).

In certain embodiments, the mRNA is a monocistronic mRNA including only one ORF. In certain embodiments, the monocistronic mRNA encodes a peptide or protein including at least one epitope of a selected antigen (e.g., a pathogenic antigen or a tumor-associated antigen). In other embodiments, the mRNA is a polycistronic mRNA comprising two or more ORF. In certain embodiments, the polycistronic mRNA encodes two or more peptides or proteins that are the same or different from each other. In certain embodiments, each peptide or protein encoded by the polycistronic mRNA comprises at least one epitope of a selected antigen. In certain embodiments, the different peptides or proteins encoded by the polycistronic mRNA each comprise at least one epitope of different antigen. In any of the embodiments described herein, at least one epitope may be at least 2, at least 3, at least 4, at least 5, at least 6, at least 7, at least 8, at least 9, or at least 10 epitopes of one antigen.

The term "nucleobases" encompasses purines and pyrimidines, including natural compounds, i.e., adenine, thymine, guanine, cytosine, uracil, inosine, and natural or synthetic analogs or derivatives thereof.

As used herein, the term "functional nucleotide analogs" refers to modified forms of canonical nucleotides A, G, C, U, or T, which (a) retain base pairing properties of the corresponding canonical nucleotide, and (b) comprise at least one chemical modification of (i) nucleobase, (ii) glycosyl, (iii) phosphate, or (iv) any combination of (i) to (iii) of the corresponding natural nucleotide. As described herein, the base pairs not only encompass standard Watson-Crick A-T, A-U, or C-G base pairs, but also include base pairs formed between canonical nucleotide and functional nucleotide analog or between a pair of functional nucleotide analogs, wherein the arrangement of hydrogen bond donors and hydrogen bond receptors allows for the formation of hydrogen bonds between the modified nucleobase and the standard nucleobase or between two complementary modified nucleobase structures. For example, the functional analog of guanosine (G) retains the ability to base pair with cytosine (C) or functional analog of the cytosine. One example of such non-canonical base pairing is the base pairing between modified nucleotide inosine and adenine, cytosine, or uracil. As described herein, the functional nucleotide analogs may be naturally occurring or non-naturally occurring. Thus, nucleic acid molecules comprising the functional nucleotide analogs may have at least one modified nucleobase, glycosyl, or internucleoside bond. Exemplary chemical modifications to nucleobases, glycosyl, or internucleoside bonds of nucleic acid molecules are provided herein.

As described herein, the terms "translation enhancing element", "TEE" and "translation enhancer" refer to a region in the nucleic acid molecule, the function of which is to promote the translation of the coding sequence of nucleic acid into protein or peptide product, such as via cap-dependent or cap-independent translation. TEE is usually located in the UTR region of the nucleic acid molecules (such as mRNA), and can enhance the translation level of the coding sequence located upstream or downstream. For example, TEE in 5'-UTR of the nucleic acid molecules may be between the promoter and the initiation codon of the nucleic acid molecules. Various TEE sequences are known in the art (Wellensiek et al. Genome-wide profiling of human cap-independent translation-enhancing elements, Nature Methods, 2013 Aug; 10(8): 747-750; Chappell et al. PNAS June 29, 2004 101 (26) 9590-9594). Some TEEs are known to be conserved in a variety of species (Pánek et al. Nucleic Acids Research, Volume 41, Issue 16, 1 September 2013, Pages 7625-7634).

As described herein, the term "stem-loop sequence" refers to a single stranded polynucleotide sequence having at least two regions; when read in opposite directions, the two regions are complementary or substantially complementary to each other to form at least one double helix and non-complementary loop, the resulting loop structure being referred to as a stem-loop structure, a hairpin or a hairpin loop, which is also a secondary structure present in a number of RNA molecules.

As described herein, the term "peptide" refers to a polymer including 2-50 amino acid residues linked by one or more covalent peptide bonds. The term is applicable to natural amino acid polymers and amino acid polymers in which one or more amino acid residues are non-natural amino acids (e.g., amino acid analogs or non-natural amino acids).

The terms "polypeptides" and "proteins" are interchangeable herein, referring to polymers having more than fifty amino acid residues linked by covalent peptide bonds. That is, the description of polypeptides is also applicable to the description of proteins, and vice versa. The terms are applicable to natural amino acid polymers and amino acid polymers in which one or more amino acid residues are non-natural amino acids (e.g., amino acid analogs). As used herein, the terms encompass amino acid chains of any length, including full-length proteins (e.g., antigens).

The term "antigen" refers to a substance that can be recognized by the immune system (including the adaptive immune system) of the subject, and can produce immune response (including antigen-specific immune response) in contact with the antigen in the subject. In certain embodiments, the antigen is protein (e.g., Tumor Associated Antigen (TAA)) associated with diseased cells (e.g., pathogen or neoplastic cell infected cell).

In the context of peptides or polypeptides, the term "fragment" refers to peptide or polypeptide including sequence less than full length amino acid sequence. Such fragments may be derived from N-end truncation, C-terminal truncation, and/or deletion of residues within the amino acid sequence. The fragments may be produced by alternative RNA splicing or in vivo protease. In certain embodiments, the fragment refers to polypeptide including at least 5 consecutive amino acid residues, at least 10 consecutive amino acid residues, at least 15 consecutive amino acid residues, at least 20 consecutive amino acid residues, at least 25 consecutive amino acid sequences, at least 30 consecutive amino acid residues, at least 40 consecutive amino acid residues, at least 50 consecutive amino acid residues, at least 60 consecutive amino acid residues, at least 70 consecutive amino acid residues, at least 80 consecutive amino acid residues, at least 90 consecutive amino acid residues, at least 100 consecutive amino acid residues, at least 125 consecutive amino acid residues, at least 150 consecutive amino acid residues, at least 175 consecutive amino acid residues, at least 200 consecutive amino acid residues, at least 250, at least 300, at least 350, at least 400, at least 450, at least 500, at least 550, at least 600, at least 650, at least 700, at least 750, at least 800, at least 850, at least 900, or at least 950 consecutive amino acid residue sequences. In one particular embodiment, the fragment of the polypeptide retains at least 1, at least 2, at least 3, or more functions of the polypeptide.

The "epitope" is a site of specific antibody molecule binding to the surface of antigen molecules, such as a localized region on the surface of the antigen that is capable of binding to one or more antigen binding regions of the antibody, has antigenic or immunogenic activity in animal, such as mammal (e.g., human), and is capable of eliciting an immune response. The epitope having immunogenic activity is part of polypeptide that elicits an antibody response in animal. The epitope having antigenic activity is a part of antibody-binding polypeptide, as determined by any method known in the art, including, for example, by immunoassay. The antigenic epitope needs not necessarily be immunogenic. The epitopes typically consist of collection of chemically active surface groups of the molecules, such as amino acids or sugar side chains, and typically have specific three-dimensional structural characteristics, as well as specific charge characteristics. The antibody epitope may be linear epitope or conformational epitope. The linear epitope is formed from the continuous sequence of amino acids in protein. The conformational epitope is formed from discontinuous amino acids in the protein sequence, but will bind together when the protein is folded into its three-dimensional structure. When the three-dimensional structure of the protein is in a modified configuration, an induced epitope is formed, such as after activation or binding of another protein or ligand. In certain embodiments, the epitope is a three-dimensional surface feature of polypeptide. In other embodiments, the epitope is a linear feature of polypeptide. In general, the antigen has several or more different epitopes and may react with many different antibodies.

As used herein, the term "gene vaccine" refers to a therapeutic or prophylactic composition including at least one nucleic acid molecule encoding antigen associated with target disease (such as infectious disease or neoplastic disease). The production of peptides or proteins is encoded by administering vaccine (vaccination) to the subject, thereby eliciting an immune response in the subject against the target disease. In certain embodiments, the immune response includes an adaptive immune response, such as producing an antibody against the encoded antigen, and/or immune cells capable of activating and proliferating diseased cells for specific elimination of the expressed antigen. In certain embodiments, the immune response also includes an innate immune response. According to the present invention, the vaccine may be administered to the subject before or after onset of clinical symptoms of the target disease. In some embodiments, vaccination of healthy or asymptomatic subjects makes the vaccinated subject immune or less sensitive to the progression of the target disease. In some embodiments, vaccination of subjects with disease symptoms may improve the disease condition of the vaccinated subject or treat the disease.

The terms "innate immune responses" and "innate immunity" are well known in the art, referring to the non-specific defense mechanism initiated by the human immune system when identifying pathogen-associated molecules, which involve different forms of cellular activity, including cytokine production and cell death in various pathways. As described in the invention, innate immune responses include, but are not limited to, increased production of inflammatory cytokines (e.g., type I interferon or IL-10 production), activation of the NPκB pathway, increased proliferation, maturation, differentiation and/or survival of immune cells, and in some cases, induced apoptosis. Methods known in the art may be used for detecting the activation of innate immunity, for example by measuring the activation of (NF)-xB.

The terms "adaptive immune responses" and "adaptive immunity" are known in the art, referring to antigen-specific defense mechanisms initiated by the immune system of human after recognition of a particular antigen, including humoral responses and cell-mediated responses. As described herein, adaptive immune responses include cell responses triggered and/or enhanced by vaccine compositions (such as genetic compositions described herein). In some embodiments, a vaccine composition comprises an antigen that is a target for an antigen-specific adaptive immune response. In other embodiments, the vaccine composition, upon administration, allows for the production of the antigen in an immunized subject, and this antigen is a target for an antigen-specific adaptive immune response. Methods known in the art may be used for detecting activation of an adaptive immune response, such as by monitoring the production of antigen-specific antibodies or monitoring antigen-specific cell-mediated levels of cytotoxicity.

The term "antibody" is intended to include a polypeptide product secreted by effector B cells, consisting of two pairs of identical polypeptide chains, wherein each pair of polypeptide chains has a heavy chain (about 50-70 kDa) and a light chain (about 25 kDa); the N-end portion of each chain comprises a variable region consisting of about 100 to about 130 or more amino acids; the C-terminal portion of each chain includes a constant region capable of binding to a particular molecular antigen; and the immunoglobulin is not merely an antibody. For example, see Antibody Engineering (Borrebaeck ed., 2d ed. 1995) and Kuby, Immunology (3d ed. 1997). In particular embodiments, a particular molecular antigen includes polypeptide, a fragment or epitope thereof, which may bind to the antibody described herein. The antibodies also include, but are not limited to, synthetic antibodies, antibodies produced by recombination, camelized antibodies, intracellular antibodies, anti-Id antibodies, and functional fragments of these antibodies, which are functional polypeptide fragments isolated from the aforementioned antibody heavy or light chains and capable of retaining part or all of binding activity. Some non-limiting examples of the functional fragments include single chain antibodies (scFv) (including monospecific, bispecific, etc.), Fab fragments, F(ab') fragments, F(ab)2 fragments, F(ab')2 fragments, disulfide bond stable antibodies (dsFv), Fd fragments, Fv fragments, diantibodies, triantibodies, tetraantibodies, and miniantibodies. In particular, the antibodies described herein comprise immunoglobulin molecules and immunologically active portions of immunoglobulin molecules, such as may be antigen binding domains or molecules containing antigen binding sites (e.g., one or more CDRs of the antibody). Such antibody fragments may be described in Harlow and Lane, Antibodies: A Laboratory Manual (1989); Mol. Biology and Biotechnology: A Comprehensive Desk Reference (Myers ed., 1995); Huston et al., 1993, Cell Biophysics 22: 189-224; Plückthun and Skerra, 1989, Meth. Enzymol. 178:497-515; and Day, Advanced Immunochemistry (2d ed. 1990). The antibodies provided herein may be any type of immunoglobulin molecule (e.g., IgG, IgE, IgM, IgD, and IgA type) or any subclass (e.g., IgG1, IgG2, IgG3, IgG4, IgA1, and IgA2 type).

The term "administration" refers to the behavior of delivering an in vitro substance (e.g., the lipid nanoparticle compositions described herein) to a patient, e.g., through mucosa, intramuscular/subcutaneous injection, intravenous injection, or other physical means known in the art. When used for treating disease, disorder, condition, or symptom thereof, the substance is typically administrated after the onset of the disease, disorder, condition, or symptom thereof. When used for preventing disease, disorder, or condition, the administration of the substance is typically performed before the onset of the disease, disorder, or condition.

"Chronic" administration refers to administration in a continuous mode (e.g., for a period of time such as several days, several weeks, several months, or several years) in opposition to an acute administration mode to maintain an initial therapeutic effect (activity) for an extended period of time. "Intermittent" dosing is not continuous but periodic, without interrupting the treatment.

The term "targeted delivery" or "target" in verb form refers to the process of facilitating the delivery of an agent (e.g., the therapeutically payload molecule in the lipid nanoparticle composition described herein) to particular organ, tissue, cell, and/or intracellular compartment (referred to as a target site) such that more target site is delivered than any other organ, tissue, cell, or intracellular compartment (referred to as a non-target site). Targeted delivery may be detected by methods known in the art, e.g., by comparing the concentration of the agent delivered in a target cell population with the concentration of the agent delivered in a non-target cell population following systemic administration. In certain embodiments, targeted delivery results in a concentration at the target site that is at least twice higher than the non-target site.

"Effective amount" is generally an amount sufficient to reduce the severity and/or frequency of symptom, eliminate the symptom and/or root cause, prevent the onset of symptom and/or cause thereof, and/or ameliorate or remedy impairment. Diseases caused by, or associated with, a disease, disorder, or condition include infection and the formation of tumor, etc. In some embodiments, the effective amount is a therapeutically effective amount or a prophylactically effective amount.

As described herein, the term "therapeutically effective amount" refers to the amount of the agent (e.g., vaccine composition) sufficient to reduce and/or ameliorate the severity and/or duration of a given disease, disorder, or condition-related symptom (e.g., infectious disease caused by viral infection, or neoplastic disease of cancer). The "therapeutically effective amount" of the substance/molecule/agent of the present disclosure (e.g., the lipid nanoparticle compositions described herein) may vary depending on factors such as the disease state, age, gender, and weight of an individual, and the ability of the substance/molecule/agent to cause a desired response in an individual, etc. The therapeutically effective amount includes an amount in which any toxic or harmful effect of the substance/molecule/agent is counteracted by the therapeutic beneficial effect. In certain embodiments, the term "therapeutically effective amount" refers to the amount of the lipid nanoparticle composition or therapeutic or prophylactic agent (e.g., therapeutic mRNA) contained therein that is capable of effectively "treating" a disease, disorder, or condition in the subject or mammal.

The "prophylactically effective amount" is an amount of a pharmaceutical composition that will have an expected prophylactic effect when administered to the subject, for example, the amount of the pharmaceutical composition that prevents, delays, or reduces the likelihood of onset (or recurrence) of disease, disorder, and related symptoms (such as infectious disease caused by viral infection or neoplastic disease such as cancer). The condition or related symptoms are typically, but not necessarily, the prophylactically effective amount may be less than the therapeutically effective amount because the prophylactic dose is used in the subject prior to or at an earlier stage of the disease, disorder, or condition. The complete therapeutic or prophylactic effect does not necessarily occur by administering a dose, but may occur only after a series of doses are administered in one or more administrations. Thus, the therapeutically or prophylactically effective amount may be administered by one or more times.

The term "prevention" refers to reducing the likelihood of suffering from disease, disorder, condition, or associated symptom (e.g., infectious disease, e.g., by viral infection or neoplastic disease, e.g., cancer).

The term "management" refers to the beneficial effects on the subject from treatment (e.g., prophylactic or therapeutic agent), which does not lead to healing of the disease. In certain embodiments, one or more therapies (e.g., a prophylactic or therapeutic agent, e.g., the lipid nanoparticle compositions described herein) are administered to the subject to "manage" one or more symptoms of the infectious or neoplastic disease, thereby preventing progression or progression of the disease.

The term "prophylactic agent" refers to any agent that may completely or partially inhibit the progression, recurrence, onset, or spread of the disease and/or symptoms associated therewith in the subject.

The term "therapeutic agent" refers to any drug that may be used for treating, preventing or ameliorating a disease, disorder or condition, including drugs for treating, preventing or ameliorating the disease, disorder or condition and related symptoms.

The term "therapy" refers to any protocol, method and/or agent that can be used for preventing, managing, treating and/or ameliorating disease, disorder or condition. In certain embodiments, the term "therapy" refers to therapies known to those skilled in the art such as biological therapy, supportive therapy and/or other therapies that can be used for preventing, controlling, treating and/or ameliorating known disease, disorder or condition.

The "prophylactically effective serum titer" refers to the serum titer of the antibody in the subject (e.g., human), which completely or partially inhibits the development, recurrence, onset or spread of disease, disorder or condition and symptoms associated therewith.

In certain embodiments, the "therapeutically effective serum titer" refers to the serum titer of the antibody in the subject (e.g., a human), which reduces the severity, duration and/or symptoms associated with disease, disorder or condition.

The term "serum titer" refers to the average serum titer in a population of subjects from multiple samples (e.g., at multiple time points) or at least 10, at least 20, at least 40 subjects, at most about 100, 1000 or more subjects.

The term "side effects" encompasses undesirable and/or adverse effects of a therapy (e.g., prophylactic or therapeutic agent). Harmful effects are not necessarily unfavorable. The adverse effects of the therapy (e.g., prophylactic or therapeutic agent) may be harmful, uncomfortable or risky. Examples of the side effects include diarrhea, cough, gastroenteritis, asthma, nausea, vomiting, anorexia, abdominal colic, fever, pain, weight loss, dehydration, hair loss, dyspnea, insomnia, dizziness, mucositis, neurological and muscular effects, fatigue, dry mouth, loss of appetite, rash or swelling at the site of administration, influenza-like symptoms such as fever, chill, fatigue, digestive tract problems and allergic reactions, etc. Other undesirable effects experienced by patients are known in the art and related introduction is made in Physician's Desk Reference (68th ed. 2014).

The terms "subject" and "patient" may be used interchangeably. As described herein, in certain embodiments, the subject is mammal, such as non-primate (e.g., cattle, pigs, horses, cats, dogs, and rats) or primate (e.g., monkey and human). In particular embodiments, the subject is human. In one embodiment, the subject is mammal (e.g., human) with infectious or neoplastic disease. In another embodiment, the subject is mammal (e.g., a human) that is at risk of developing infectious or neoplastic disease.

The term "detectable probe" refers to a composition that provides a detectable signal. The term includes, but is not limited to, any fluorophore, chromophore, radiolabel, enzyme, antibody or antibody fragment, etc. that provides the detectable signal by its activity.

The term "detectable agent" refers to a substance that can be used for determining the presence of desired molecules in a sample or subject, such as the antigen encoded by the mRNA molecules described herein. The detectable agent may be a substance that may be visualized, or a substance that can be determined and/or measured (e.g., by quantification).

"Substantially all" refers to at least about 60%, at least about 65%, at least about 70%, at least about 75%, at least about 80%, at least about 85%, at least about 90%, at least about 95%, at least about 98%, at least about 99% or about 100%.

As described herein, unless otherwise stated, the term "about" or "approximate" refers to an acceptable error for a particular value determined by a general technician in the art, which depends in part on the way in which the value is measured or determined. In certain embodiments, the term "about" or "approximate" refers to within 1, 2, 3, or 4 standard deviations. In certain embodiments, the term "about" or "approximate" refers to within 20%, 15%, 10%, 9%, 8%, 7%, 6%, 5%, 4%, 3%, 2%, 1%, 0.5%, 0.05% or less than of a given value or range.

Unless otherwise stated in the context, the singular terms used herein "a", "an" and "the" include the plural referents thereof.

All publications, patent applications, reference numbers and other references cited in this specification are incorporated by reference in their entirety, and each individual publication or patent application is expressly and separately incorporated by reference. The publications discussed herein are those published prior to the filing date of this application. Nothing herein shall be construed as an admission that the present invention is not entitled to predate such publication by virtue of a prior present invention. In addition, the release date provided herein may differ from the actual release date, which may need to be independently confirmed.

A number of embodiments have been described for the present invention. However, it will be understood that various modifications can be made without departing from the general conception and scope of the present invention. Therefore, the descriptions in the experimental part and in the Exmaples are intended to illustrate, not limit, the scope of the present invention described in the claims.

### Lipid compound

In one embodiment, the present invention provides a compound represented by a Formula (I): 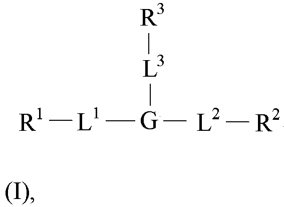 or pharmaceutically acceptable salt or stereoisomer thereof, wherein:
Gis N or CH;
L¹, L² and L³ each are independently selected from bonds or C₁-C₈ alkylene;
R¹ is selected from -C(O)OR⁴ or -OC(O)R⁹;
R² is selected from -C(O)OR⁵, C₃-C₈ cycloalkyl, C₁-C₈ alkyl, OC(O)R¹⁰, -C(O)NHC₁₀-C₂₀ alkyl or -NHC(O)C₁₀-C₂₀ alkyl;
R³ is selected from 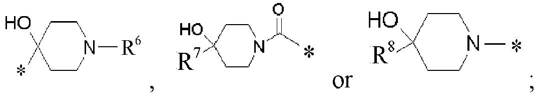
R⁴ is C₁₀-C₂₀ alkyl;
R⁵ is C₁₀-C₂₀ alkyl;
R⁶ is selected from C(O)(CH₂)₁₋₈N(C₁-C₈ alkyl)C₁-C₈ alkyl, C₁-C₈ alkyl or -(CH₂)₁₋₈C(O)OR¹¹;
R⁷ is (CH₂)₁₋₈N(C₁-C₈ alkyl)C₁-C₈ alkyl;
R⁸ is (CH₂)₁₋₈N(C₁-C₈ alkyl)C₁-C₈ alkyl;
R⁹ is C₁₀-C₂₀ alkyl;
R¹⁰ is C₁₀-C₂₀ alkyl;
R¹¹ is C₁₀-C₂₀ alkyl; and
* denotes a ligation site.

In one embodiment, in Formula (I),
GisN;
L¹, L² and L³ each are independently C₁-C₈ alkylene;
R¹ is -C(O)OR⁴;
R² is -C(O)OR⁵;
R³ is 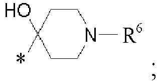
R⁴ is C₁₀-C₂₀ alkyl;
R⁵ is C₁₀-C₂₀ alkyl;
R⁶ is selected from C(O)(CH₂)₁₋₈N(C₁-C₈ alkyl), C₁-C₈ alkyl or C₁-C₈ alkyl; and
* denotes a ligation site.

In one embodiment, in Formula (I),
GisN;
L¹, L² and L³ each are independently C₁-C₈ alkylene;
R¹ is -C(O)OR⁴;
R² is -C(O)OR⁵;
R³ is selected from 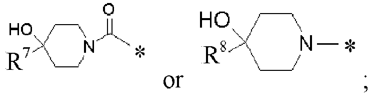
R⁴ is C₁₀-C₂₀ alkyl;
R⁵ is C₁₀-C₂₀ alkyl;
R⁷ is(CH₂)₁₋₈N(C₁-C₈ alkyl)C₁-C₈ alkyl;
R⁸ is-(CH₂)₁₋₈N(C₁-C₈ alkyl)C₁-C₈ alkyl; and
* denotes a ligation site.

In one embodiment, in Formula (I),
GisN;
L¹ and L³ each are independently C₁-C₈ alkylene;
L² is a linkage;
R¹ is -C(O)OR⁴;
R² is selected from C₃-C₈ cycloalkyl or C₁-C₈ alkyl;
R³ is 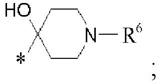
R⁴ is C₁₀-C₂₀ alkyl;
R⁶ is -(CH₂)₁₋₈C(O)OR¹¹;
R¹¹ is C₁₀-C₂₀ alkyl; and
* denotes a ligation site.

In one embodiment, in Formula (I),
G is CH;
L¹, L² and L³ each are independently C₁-C₈ alkylene;
R¹ is -OC(O)R⁹;
R² is -OC(O)R¹⁰;
R³ is 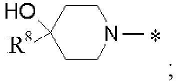
R⁸ is (CH₂)₁₋₈N(C₁-C₈ alkyl)C₁-C₈ alkyl;
R⁹ is C₁₀-C₂₀ alkyl;
R¹⁰ is C₁₀-C₂₀ alkyl; and
* denotes a ligation site.

In one embodiment, in Formula (I), R⁴, R⁵, R⁹, R¹⁰ and R¹¹ are each independently selected from: -(CH₂)₁₋₃-CH(C₅-C₁₀ alkyl) (C₅-C₁₀ alkyl).

In one embodiment, the compound is the compound described in Table 1:

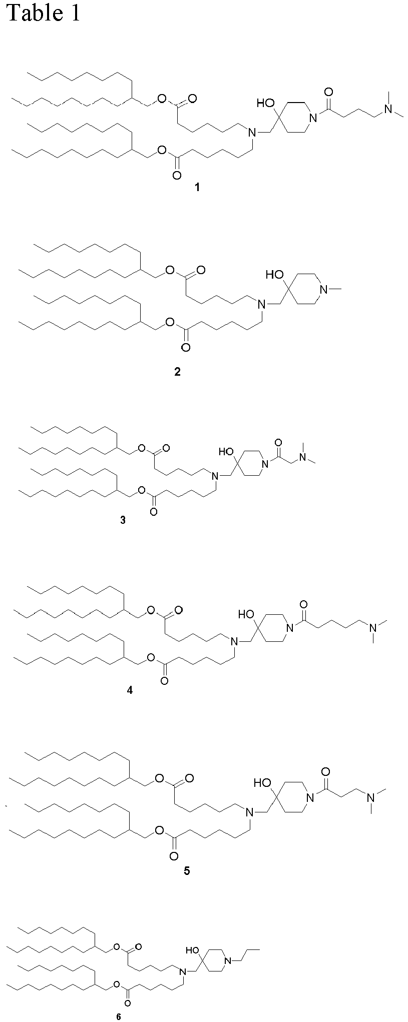

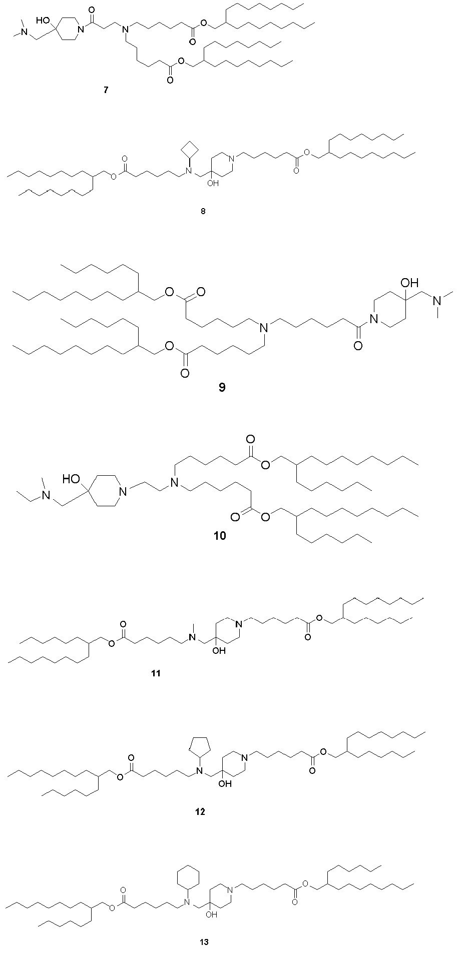

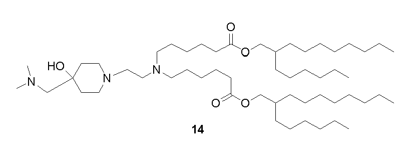

or

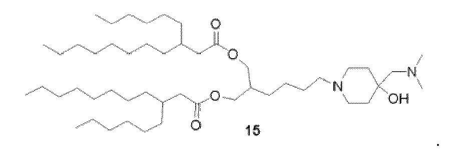

It is to be understood that any embodiment of the compound according to the present invention as described above, and any specific substituents and/or variables of the compound according to the present invention as described above, may be independently combined with other embodiments and/or various variables of substituents and/or compounds to form an unspecified embodiment. In addition, in a case of a list of substituents and/or variables listed for any particular group or variable, it is to be understood that each individual substituent and/or variable may be removed from the particular embodiment and/or claim, and that the remaining list of substituents and/or variables will be deemed to be within the scope of the embodiment provided herein.

It should be understood that in this specification, it is only permissible if the combination of substituents and/or variables of the chemical formula described is such that the compound is stable.

### Nanoparticle composition

In one aspect, the nanoparticle composition comprising the lipid compound described herein is described herein. In particular embodiments, the nanoparticle composition comprises the compound according to Formula (I) (and sub-formulas thereof) described herein.

In some embodiments, the largest dimension of the nanoparticle composition provided herein is 1 µm or less (e.g., ≤ 1 µm, ≤ 900 nm, ≤ 800 nm, ≤ 700 nm, ≤ 600 nm, ≤ 500 nm, ≤ 400 nm, ≤ 300 nm, ≤ 200 nm, ≤ 175 nm, ≤ 150 nm, ≤ 125 nm, ≤ 100 nm, ≤ 75 nm, ≤ 50 nm or less) when measured by Dynamic Light Scattering (DLS), transmission electron microscopy, scanning electron microscopy, or other methods. In one embodiment, at least one dimension of the lipid nanoparticle provided herein has a range from about 40 to about 200nm. In one embodiment, at least one dimension is in a range from about 40 to about 100nm.

The nanoparticle composition that can be used in combination with the present invention includes the lipid nanoparticles (LNP), nanolipoprotein particles, liposomes, lipid vesicles and lipid complexes, etc. In some embodiments, the nanoparticle composition comprises one or more vesicles of lipid bilayers. In some embodiments, the nanoparticle composition comprises two or more concentric bilayers separated by aqueous compartments. Lipid bilayers may be functionalized and/or cross-linked with each other. Lipid bilayers may include one or more ligands, proteins, or channels.

The characteristics of the nanoparticle composition may depend on its components. For example, a nanoparticle composition comprising cholesterol as a structural lipid may have different characteristics from a nanoparticle composition comprising different structural lipid. Similarly, the characteristics of the nanoparticle composition may depend on the absolute or relative amounts of its components. For example, a nanoparticle composition comprising a higher molar fraction of phospholipid may have different properties from a nanoparticle composition comprising a lower molar fraction of phospholipid. The properties may also vary depending on the method and conditions for preparing the nanoparticle composition.

The nanoparticle composition may be characterized by a variety of methods. For example, microscopes (transmission electron microscope or scanning electron microscope, etc.) may be used for detecting the morphology and size distribution of nanoparticle compositions. Dynamic light scattering or potentiometric methods (such as potentiometric titration) can be used for measuring ζ potential. Dynamic light scattering may also be used for determining particle size. Instruments such as Zetasizer Nano ZS (Malvern Instruments Ltd, Malvem, and Worcestershire, UK) may also be used for measuring multiple characteristics of nanoparticle compositions, such as particle size, polydispersity index and ζ potential.

Dh (size): the average size of the nanoparticle compositions may be between 10s nm and 100s nm. For example, the average size may be between about 40 nm and about 150 nm, such as about 40 nm, 45 nm, 50 nm, 55 nm, 60 nm, 65 nm, 70 nm, 75 nm, 80 nm, 85 nm, 90 nm, 95 nm, 100 nm, 105 nm, 110 nm, 115 nm, 120 nm, 125 nm, 130 nm, 135 nm, 140 nm, 145 nm, or 150 nm. In some embodiments, the average size of the nanoparticle compositions may be between about 50 nm and about 100 nm, between about 50 nm and about 90 nm, between about 50 nm and about 80 nm, between about 50 nm and about 70 nm, between about 50 nm and about 60 nm, between about 60 nm and about 100 nm, between about 60 nm and about 90 nm, between about 60 nm and about 80 nm, between about 60 nm and about 70 nm, between about 70 nm to about 100 nm, between about 70 nm and about 90 nm, between about 70 nm and about 80 nm, between about 80 nm and about 100 nm, between about 80 nm and about 90 nm, or between about 90 nm and about 100 nm. In certain embodiments, the average size of the nanoparticle compositions may be between about 70nm and about 100nm. In some embodiments, the average size may be about 80nm. In other embodiments, the average size may be about 100nm.

PDI: the composition of nanoparticles may be relatively uniform. A polydispersity index may be used for indicating the uniformity of the nanoparticle composition, e.g., the particle size distribution of the nanoparticle composition. A small (e.g., less than 0.3) polydispersity index generally indicates a narrow particle size distribution. The nanoparticle composition may have a polydispersity index of about 0 to about 0.25, e.g., 0.01, 0.02, 0.03, 0.04, 0.05, 0.06, 0.07, 0.08, 0.09, 0.10, 0.11, 0.12, 0.13, 0.14, 0.15, 0.16, 0.17, 0.18, 0.19, 0.20, 0.21, 0.22, 0.23, 0.24, or 0.25. In some embodiments, the polydispersity index of the nanoparticle composition may be about 0.10 to about 0.20.

Encapsulation efficiency: encapsulation efficiency of therapeutic and/or prophylactic agent represents a ratio of an amount of the therapeutic and/or prophylactic agent encapsulated or combined with the nanoparticle composition after preparation, relative to the initially provided amount. High encapsulation efficiency (e.g., approximate to 100%) is desired. Encapsulation efficiency may be measured by comparing amounts of the therapeutic and/or prophylactic agent comprising the nanoparticle composition prior to decomposition of the nanoparticle composition with one or more organic solvents or detergents, and after decomposition in solution. Fluorescence may be used for measuring the amount of free therapeutic and/or prophylactic agents (e.g., RNA) in solution. For the nanoparticle compositions described herein, encapsulation efficiency of the therapeutic and/or prophylactic agent may be at least 50%, such as 50%, 55%, 60%, 65%, 70%, 75%, 80%, 85%, 90%, 91%, 92%, 93%, 94%, 95%, 96%, 97%, 98%, 99% or 100%. In some embodiments, encapsulation efficiency may be at least 80%. In certain embodiments, encapsulation efficiency may be at least 90%.

Apparent pKa: the ζ potential of the nanoparticle composition can be used for indicating the electrodynamic potential of the composition. For example, ζ potential may describe the surface charge of the nanoparticle composition. For the nanoparticle composition, it is often expected to have a relatively low positive or negative charge, as higher-charge substances may interact adversely with cells, tissues, and other elements of the human body. In some embodiments, the ζ potential of the nanoparticle composition may be about -10 mV to about +20 mV, about -10 mV to about +15 mV, about -10 mV to about +10 mV, or about -10 mV to about +5 mV, about -10 mV to about 0 mV, about -10 mV to about -5 mV, about -5 mV to about +20 mV, about -5 mV to about +15 mV, about -5 mV to about +10 mV, about -5 mV to about +5 mV, about -5 mV to about 0 mV, about 0 mV to about +20 mV, about 0 mV to about +15 mV, about 0 mV to about +10 mV, about 0 mV to about +5 mV, about +5 mV to about +20 mV, about +5 mV to about +15 mV or about +5 mV to about +10 mV.

In another embodiment, self-replicating RNA may be formulated in liposomes. As a non-limiting example, self-replicating RNA may be formulated in liposomes, as described in International Publication No. WO20120067378, which is incorporated herein by reference in its entirety. In one aspect, liposomes may comprise lipids that facilitate delivery of pKa values for mRNA. In another aspect, liposomes may have a substantially neutral surface charge at physiological pH and thus may be useful for immunization (see, for example, liposomes described in International Publication No. WO20120067378, which is incorporated herein by reference in its entirety).

In some embodiments, the nanoparticle composition comprises the lipid component comprising at least one lipid, such as a compound according to the Formula (I) (and its sub-formula) described herein. For example, in some embodiments, the nanoparticle composition may include the lipid component that includes one of the compounds presented herein. Nanoparticle compositions may also comprise one or more other lipid or non-lipid components as described below.

### Cationic/ionizable lipid

As described herein, in some embodiments, the nanoparticle composition provided herein comprises one or more charged or ionizable lipids in addition to the lipid according to the Formula (I) (and sub-formulas thereof). It may be contemplated that some of the charged or zwitterionic lipid components of the nanoparticle composition resemble the lipid components in the cell membrane, which may thus improve the cellular uptake of the nanoparticles. Exemplary charged or ionizable lipids that may form part of the nanoparticle composition of the present invention include, but are not limited to, 3-(docylamino)-N1,N1,4-tridodecyl-1-piperazinethylamine (KL10), N1-[2-(docanekylamino)ethyl]-N1,N4,N4-tridodecyl-1,4-piperazinedienamide (KL22), 14,25-docodecyl-15,18,21,24-tetraazaoctadecane (KL25), 1,2-dilinoleoyloxy-N,N-dimethylaminopropane (DLinDMA), 2,2-dilinoleoyl-4-dimethylaminomethyl-[1,3]-dioxolane (DLin-K-DMA), heptatriaconta-6,9,28,31-tetraen-19-yl 4-(dimethylamino)butyrate (DLin-MC3-DMA), 2,2-dilinoleoyl-4-(2-dimethylaminoethyl)-[1,3]-dioxolane (DLin-KC2-DMA), 1,2-dioloxy-N,N-dimethylaminopropane (DODMA), 2-({8-[(3β)-cholest-5-en-3-yloxy]octyl}oxy)-N,N-dimethyl-3 [(9Z,12Z)-octadeca-9,12-dien-1-yloxy]propyl-1-amine (octyl-CLinDMA), (2R)-2-({8-[(3β)-cholest-5-en-3-yloxy]octyl}oxy)-N,N-dimethyl-3-[[(9Z,12Z)-octadeca-9,12-dien-1-yloxy]propyl-1-amine (octyl-CLinDMA(2R)), (2S)-2-({8-[(3β)-cholest-5-en-3-yloxy]octyl}oxy)-N,N-dimethylethyl-3-[((9Z-,12Z)-octadec-9,12-dien-1-yloxy]propyl-1-amine (Octyl-CLinDMA(2S)), (12Z,15Z)-N,N-dimethyl-2-nonyldocyl 12,15-den-1-amine, and N,N-dimethyl-1-{((1S,2R)-2-octylcyclopropyl}heptadecan-8-amine. Additional exemplary charged or ionizable lipids (e.g., lipid 5) that may form part of the nanoparticle composition of the present invention, including those described in Sabnis et al. "A Novel Amino Lipid Series for mRNA Delivery: Improved Endosomal Escape and Sustained Pharmacology and Safety in Non-human Primates", Molecular Therapy Vol. 26 No 6, 2018, all of which are incorporated herein by reference in their entirety.

In some embodiments, suitable cationic lipids include N-[1-(2,3-dioleyloxy)propyl]-N,N,N-trimethylammonium chloride (DOTMA); N-[1-(2,3-dioleoyloxy)propyl]-N,N,N-trimethylammonium chloride (DOTAP); 1,2-dioleoyl-sn-glycero-3-ethylcholine phosphate (DOEPC); 1,2-dilauroyl-sn-glycero-3-ethylcholine phosphate (DLEPC); 1,2-dimyristoyl-sn-glycero-3-ethylcholine phosphate (DMEPC); 1,2-dimyristoleoyl-sn-glycero-3-ethylcholine phosphate (14:1); N1-[2-((1S)-1-[(3-aminopropyl)amino]-4-[bis(3-aminopropyl)amino]butylformamido)ethyl]-3,4-di[oleoyloxy]-benzoamide (MVL5); bis-octadecylamino-glycylspermine (DOGS); 3b-[N-(N',N'-dimethylaminoethyl)carbamoyl]cholesterol (DC-Chol); Dimethyldioctadecylammonium bromide (DDAB);SAINT-2, N-methyl-4-(dioleyl)methylpyridine; 1,2-dimyristyloxypropyl-3-dimethylhydroxyethylammonium bromide (DMRIE); 1,2-dioleoyl-3-dimethyl-hydroxyethylammonium bromide (DORIE); 1,2-dioleoyloxypropyl-3-dimethyl hydroxyethylammonium chloride (DORI), di-alkylated amino acids (DILA²) (e.g., C18:1-norArg-C16); dioleyldimethylammonium chloride (DODAC), 1-palmitoyl-2-oleoyl-sn-glycero-3-ethylcholine phosphate (POEPC); 1,2-dimyristoleoyl-sn-glycero-3-ethylcholine phosphate (MOEPC); (R)-5-((dimethylamino)pentane-1,2-diyldioleate hydrochloride (DODAPen-Cl); (R)-5-guanidinopentane-1,2-diyldioleate hydrochloride (DOPen-G); and (R)-N,N,N-trimethyl-4,5-bis(oleoyloxy)penta-1-ammonium chloride (DOTAPen). Cationic lipids with head groups charged at physiological pH, such as primary amines (e.g., DODAG N',N'-bisoctadecyl-N-4,8-diaza-10-aminodecanoylglycinamide) and guanidine head groups (e.g., bis-guanidine-spermidine-cholesterol (BGSC), bis-guanidine triaminoethylamine-cholesterol (BGTC), PONA, and (R)-5-guanidinopentane-1,2-diyldioleate hydrochloride (DOPen-G)) are also suitable. Another suitable cationic lipid is (R)-5-(dimethylamino)pentane-1,2-diyldioleate hydrochloride (DODAPen-Cl). In certain embodiments, the cationic lipid is a specific enantiomer or racemic form, and includes multiple salt forms (e.g., chloride or sulfate) of the cationic lipid as described above. For example, in some embodiments, the cationic lipids are N-[1-(2,3-dioleoyloxy)propyl]-N,N,N-trimethylammonium chloride (DOTAP-Cl) or N-[1-(2,3-dioleoyloxy)propyl]-N,N,N-trimethylammonium sulfate (DOTAP-sulfate). In some embodiments, the cationic lipids are ionizable cationic lipids , such as (for example) bisoctadecyldimethylammonium bromide (DDAB); 1,2-dilinoleyloxy-3-dimethylaminopropane (DLinDMA); 2,2-dilinoleyl-4-(2-dimethylaminoethyl)-[1,3]-dioxane (DLin-KC2-DMA); hepta-triacontan-6,9,28,31-tetraen-19-yl4-(dimethylamino)butyrate (DLin-MC3-DMA); 1,2-dioleoyloxy-3-dimethylaminopropane (DODAP); 1,2-dioleyloxy-3-dimethylaminopropane (DODMA); and morpholinocholesterol (Mo-CHOL). In some embodiments, the lipid nanoparticles include a combination of two or more cationic lipids (e.g., two or more cationic lipids as above).

In addition, in some embodiments, the charged or ionizable lipids that may form part of the present composition of nanoparticles are lipids that include cyclic amine groups. Additional cationic lipids applicable to the formulations and methods disclosed herein include those described in WO2015199952, WO2016176330, and WO2015011633, all of which are incorporated herein by reference in their entirety.

### Polymer-conjugated lipid

In some embodiments, the lipid components of the nanoparticle composition may include one or more polymer-conjugated lipids (polymer-conjugate lipids), such as PEGylated lipids (PEG lipids). It may be expected that the polymer-conjugate lipid components of the nanoparticle composition may improve colloidal stability and/or reduce protein uptake by the nanoparticles. Exemplary cationic lipids that may be used in combination with the present disclosure include, but are not limited to, PEG-modified phosphatidylethanolamine, PEG-modified phosphatidic acid, PEG-modified ceramide, PEG-modified dialkylamine, PEG-modified diacylglycerol, PEG-modified dialkylglycerol, and mixtures thereof. For example, the PEG lipid may be PEG-c-DOMG, PEG-DMG, PEG-DLPE, PEG-DMPE, PEG-DPPC, PEG-DSPE, ceramide-PEG2000, or Chol-PEG2000.

In one embodiment, the polymer-conjugated lipids are pegylated lipids. Some embodiments include pegylated diacylglycerols (PEG-DAG), such as 1-(monomethoxy-polyethylene glycol)-2,3-dimyristoyl glycerol (PEG-DMG), pegylated phosphatidyl ethanolamine (PEG-PE), PEG-succinylated diacylglycerol (PEG-S-DAG), such as 4-O-(2',3'-bis (tetradecanoyloxy)propyl-1-O-(co-methoxy(polyethoxy)ethyl)succinate (PEG-S-DMG), pegylated ceramide (PEG-cer), or PEG dialkoxypropyl carbamate, such as co-methoxy(polyethoxy)ethyl-N-(2,3-bis(tetradecyloxy)propyl)carbamate or 2,3-bis(tetradecyloxy)propyl-N-(ω-methoxy)(polyethoxy)ethyl)carbamate.

In one embodiment, the polymer-conjugated lipid is present at a molar concentration of from 1.0 to 2.5%. In one embodiment, the polymer-conjugated lipid is present at a molar concentration of about 1.7%. In one embodiment, the polymer-conjugated lipid is present at a molar concentration of about 1.5%.

In one embodiment, the molar ratio of cationic lipid to polymer-conjugated lipid is from about 35:1 to about 25:1. In one embodiment, the molar ratio of cationic lipid to polymer-conjugated lipid is from about 100:1 to about 20:1.

In one embodiment, the pegylated lipid has the following formula: 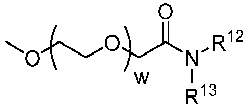 or a pharmaceutically acceptable salt, tautomer or stereoisomer thereof, wherein:
R¹² and R¹³ are each independently a linear or branched saturated or unsaturated alkyl chain containing 10 to 30 carbon atoms, where the alkyl chain is optionally interrupted by one or more ester linkages; and
the average value of w is 30-60.

In one embodiment, R¹² and R¹³ are each independently a straight chain saturated alkyl chain containing 12 to 16 carbon atoms. In other embodiments, w is on average in the range of 42 to 55, e.g., w is on average 42, 43, 44, 45, 46, 47, 48, 49, 50, 51, 52, 53, 54, or 55. In particular embodiments, the w is on average about 49.

In one embodiment, the pegylated lipid has the following formula: 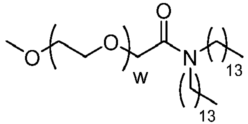 wherein the average value of w is about 49.

### Structural lipid

In some embodiments, the lipid component of the nanoparticle composition may include one or more structural lipids. It may be expected that the structural lipid may stabilize the amphiphilic structure of nanoparticles, such as, but not limited to, the lipid bilayer structure of the nanoparticle. Exemplary structural lipids that may be used in combination with the present disclosure include, but are not limited to, cholesterol, nonsterols, sitosterol, ergosterol, campesterol, stigmasterol, brassinosterol, tomatine, tomatidine, ursolic acid, α-tocopherol, and mixtures thereof. In certain embodiments, the structural lipid is cholesterol. In some embodiments, the structural lipids include cholesterol and corticosteroids (e.g., prednisolone, dexamethasone, prednisone, and hydrocortisone), or combinations thereof.

In one embodiment, the lipid nanoparticles provided herein comprise steroids or steroid analogues. In one embodiment, steroids or steroid analogues are cholesterol. In one embodiment, steroids are present at molar concentration ranges of 39-49%, 40-46%, 40-44%, 40-42%, 42-44%, or 44-46%. In one embodiment, steroids are present at a molar concentration of 40, 41, 42, 43, 44, 45, or 46%.

In one embodiment, the molar ratio of cationic lipids to steroids is 1.0:0.9 to 1.0:1.2, or 1.0:1.0 to 1.0:1.2. In one embodiment, the molar ratio of cationic lipid to cholesterol is about 5:1 to 1:1. In one embodiment, steroids are present at a molar concentration of 32-40% of steroids.

### Phospholipid

In some embodiments, the lipid component of the nanoparticle composition may include one or more phospholipids, for example one or more (poly) unsaturated lipids. It may be expected that the phospholipids may be assembled into one or more lipid bilayer structures. Exemplary phospholipids that may form part of the present nanoparticle composition include, but are not limited to, 1,2-distearoyl-sn-glycero-3-phosphocholine (DSPC), 1,2-dioleoyl-sn-glycero-3-phosphoethanolamine (DOPE), 1,2-dioleoyl-sn-glycero-3-phosphocholine (DLPC), 1,2-dimyristoyl-sn-glycerophosphocholine (DMPC), 1,2-dioleoyl-sn-glycero-3-phosphocholine (DOPC), 1,2-dipalmitoyl-sn-glycero-3-phosphocholine (DPPC), 1,2-heneicosanoyl-sn-glycerophosphocholine (DUPC), 1-palmitoyl-2-oleoyl-sn-glycero-3-phosphocholine (POPC), 1,2-di-O-octadecene-sn-glycero-3-phosphocholine (18:0 Diether PC), 1-oleyl-2-cholesteryl-hemisuccinyl-sn-glycero-3-phosphocholine (OChemsPC), 1-hexadecyl-sn-glycero-3-phosphocholine (C16 Lyso PC), 1,2-dilinoleyl-sn-glycero-3-phosphocholine, 1,2-diarachidonoyl-sn-glycero-3-phosphocholine, 1,2-docosahexaenoyl-sn-glycero-3-phosphocholine, 1,2-diphytanoyl-sn-glycero-3-phosphoethanolamine (ME 16.0 PE), 1,2-distearoyl-sn-glycero-3-phosphoethanolamine, 1,2-dilinoleyl1-sn-glycero-3-phosphoethanolamine, 1,2-dilinoleyl-sn-glycero-3-phosphoethanolamine, 1,2-diarachidonoyl-sn-glycero-3-phosphoethanolamine, 1,2-docosahexaenoyl-sn-glycero-3 -phosphoethanolamine, 1,2-dioleoyl-sn-glycero-3-phospho-rac-(1-glycero) sodium salt (DOPG) and sphingomyelin. In certain embodiments, the nanoparticle compositions include DSPC. In certain embodiments, the nanoparticle composition comprises DOPE. In some embodiments, the nanoparticle compositions include DSPC and DOPE.

Additional exemplary neutral lipid includes dipalmitoyl phosphatidylglycerol (DPPG), palmitoyl oleoyl-phosphatidylethanolamine (POPE) and dioleoyl-phosphatidylethanolamine 4-(N-maleimidomethyl)-cyclohexane-1-carboxylate (DOPE-mal), dipalmitoyl phosphatidylethanolamine (DPPE), distearoyl-phosphatidylethanolamine (DSPE), 16-0-monomethyl PE, 16-O-dimethyl PE, 18-1-trans PE, 1-stearoyl-2-oleoyl phosphatidylethanolamine (SOPE) and 1,2-dielaidoyl-sn-glycero-3-phosphoethanolamine (transDOPE). In one embodiment, the neutral lipid is 1,2-distearoyl-sn-glycero-3 phosphorylcholine (DSPC). In one embodiment, the neutral lipid is selected from DSPC, DPPC, DMPC, DOPC, POPC, DOPE and SM.

In one embodiment, the neutral lipid is Phosphatidylcholine (PC), Phosphatidylethanolamine (PE), Phosphatidylserine (PS), Phosphatidic Acid (PA) or Phosphatidylglycerol (PG).

Additional phospholipids that may form part of the nanoparticle composition of the present invention are also included in those described in WO2017/112865, which is incorporated herein by reference in its entirety.

### Therapeutic payload

The nanoparticle composition described herein may further comprise one or more therapeutic and/or prophylactic agents. These therapeutic and/or prophylactic agents are sometimes referred to in the present disclosure as "therapeutic payloads" or "payloads". In some embodiments, the nanoparticles may be used as a delivery vehicle to administer the therapeutic payload in vivo or in vitro.

In some embodiments, the nanoparticle composition comprises small molecule compounds (e.g., small molecule drugs) as the therapeutic payload, such as anti-neoplastic agents (e.g., vincristine, doxorubicin, mitoxantrone, camptothecin, cisplatin, bleomycin, cyclophosphamide, methotrexate, and streptozotocin), antineoplastic agents (e.g., Dactinomycin, vincristine, vinblastine, cytarabine, anthracyclines, alkylating agents, platinum compounds, antimetabolites, and nucleoside analogues such as methotrexate, purine, and pyrimidine analogues), anti-infective agent, local anesthetics (e.g., dibucaine and chlorpromazine), β-adrenergic blockers (e.g., propranolol, timolol, and labetalol), anti-hypertensives (e.g., clonidine and hydralazine), antidepressants (e.g., imipramine, amitriptyline, and doxepin), anti-convulsants (e.g., sodium phenytoin), anti-histamines (e.g., diphenhydramine, chlorpheniramine, and promethazine), antibiotics/antimicrobials (e.g., gentamicin, ciprofloxacin, and cefoxitin), antifungals (e.g., miconazole, terconazole, econazole, isoconazole, buconazole, clotrimazole, itraconazole, nystatin, naftifine, and amphotericin B), anti-parasitic drugs, hormones, hormone antagonists, immunomodulators, neurotransmitter antagonists, anti-glaucoma agents, anesthetics and imaging agents.

In some embodiments, the therapeutically payloads include cytotoxins, radioactive ions, chemotherapeutic agents, vaccines, compounds that elicit an immune response, and/or another therapeutic and/or prophylactic agent. Cytotoxin or cytotoxic agents include any substance that may be harmful to cells. Examples include, but are not limited to, paclitaxel, cytochalasin B, gramicidin D, ethidium bromide, emetine, mitomycin, etoposide, teniposide, vincristine, vinblastine, colchicine, adriamycin, daunorubicin, dihydroxyanthraquinone, keto-methasone, 1-nortestosterone, oryzaein, glucocorticoid, procaine, tetracaine, lidocaine, propranolol, puromycin, maytansinoid, maytanol, rachelmycin (CC-1065) and analogs or homologs thereof. Radioactive ions include, but are not limited to, iodine (e.g., iodine 125 or iodine 131), strontium-89, phosphorus, palladium, cesium, iridium, phosphate, cobalt, yttrium-90, samarium-153, and praseodymium.

In other embodiments, the therapeutic payload of the present nanoparticle composition may include, but is not limited to, therapeutic and/or prophylactic agents, such as anti-metabolites (e.g., methotrexate, 6-mercaptopurine, 6-thioguanine, cytarabine, 5-fluorouracil, dacarbazine), alkylating agents (e.g., mechloroethylamine, metsulfovax chlorambucil, rachelmycin (CC-1065), melphalan, carmustine (BSNU), lomustine (CCNU), cyclophosphamide, busulfan, dibromomannitol, streptozotocin, Mitomycin C and cis-dichlorodiamine platinum(II) (DDP) cisplatin), anthracyclines (e.g., daunorubicin (formerly daunomycin) and adriamycin), antibiotics (e.g., Dactinomycin (formerly actinomycin), bleomycin, mithramycin, and anthramycin (AMC)), and antimitotic agents (e.g., vinblastine, vincristine, paclitaxel, and maydenistin alkaloids).

In some embodiments, the nanoparticle composition comprises biomolecules, such as peptides and polypeptide, which serve as therapeutic payloads. The biomolecules that form part of the composition of the nanoparticles may be of natural or synthetic origin. For example, in some embodiments, therapeutic payloads of the nanoparticle composition may include, but be not limited to, gentamicin, amikacin, insulin, erythropoietin (EPO), granulocyte colony-stimulating factor (G-CSF), granulocyte-macrophage colony-stimulating factor (GM-CSF), factor VIR, luteinizing hormone-releasing hormone (LHRH) analogues, interferon, heparin, hepatitis B surface antigen, typhoid vaccine, cholera vaccine, as well as peptides and polypeptides.

### Nucleic acids

In some embodiments, the nanoparticle composition comprises one or more nucleic acid molecules (such as DNA or RNA molecules) as the therapeutic payload. Exemplary forms of nucleic acid molecules that may be used as the therapeutic payload in the nanoparticle composition include but are not limited to deoxyribonucleic acid (DNA), ribonucleic acid (RNA) including messenger mRNA (mRNA), and hybrid forms thereof, RNAi inducer, RNAi agent, siRNA, shRNA, miRNA, antisense RNA, ribozyme, catalytic DNA, RNA that induces triple helix formation, aptamer, carrier and the like. In certain embodiments, the therapeutic payload includes RNA. RNA molecules that may be comprised in the nanoparticle composition of the invention as a therapeutic payload include but are not limited to: short isomers, agomirs, antagomirs, antisense molecules, ribozymes, small interfering RNAs (siRNAs), asymmetric interfering RNAs (aiRNAs), microRNA (miRNAs), Dicer-substrate RNAs (dsRNAs), small hairpin RNAs (shRNAs), transfer RNAs (tRNAs), messenger RNAs (mRNAs) and other forms of RNA molecules known in the art. In particular embodiments, the RNA is mRNA.

In other embodiments, the nanoparticle composition comprises siRNA molecules as the therapeutic payload. In particular, in some embodiments, the siRNA molecules are capable of selectively interfering with and down-regulating expression of a target gene. In some embodiment, when the nanoparticle composition comprising siRNA is administered to a subject to be administered, the siRNA payload will selectively silence genes associated with particular disease, disorder, or condition. In some embodiments, the siRNA molecules comprise a sequence complementary to an mRNA sequence encoding a protein product of interest. In some embodiments, the siRNA molecules are immunomodulatory siRNA.

In some embodiments, the nanoparticle composition comprises shRNA molecules or a vector encoding the shRNA molecule as therapeutic payload. In particular, in some embodiments, the therapeutic payload produces shRNA within target cells upon administration to the target cells. Constructs and mechanisms related to shRNA are known in the art.

In some embodiments, the nanoparticle composition comprises mRNA molecules as therapeutic payloads. In particular, in some embodiments, the mRNA molecules encode the target polypeptide, including any naturally or non-naturally occurring polypeptide or modified polypeptide. Polypeptide encoded by mRNA may be of any size and may have any secondary structure or activity. In some embodiments, polypeptide encoded by mRNA payloads may have therapeutic effects when expressed in cells.

In some embodiments, the nucleic acid molecules of the present disclosure comprise mRNA molecules. In particular embodiments, the nucleic acid molecules comprise at least one coding region (e.g., an Open Reading Frame (ORF)) encoding target peptide or polypeptide. In some embodiments, the nucleic acid molecules further comprise at least one untranslated region (UTR). In particular embodiments, the untranslated region (UTR) is located upstream (5' end) of the coding region, referred to herein as 5'-UTR. In particular embodiments, the untranslated region (UTR) is located downstream (3' end) of the coding region, referred to herein as 3'-UTR. In particular embodiments, the nucleic acid molecules include both 5'-UTR and 3'-UTR. In some embodiments, the 5'-UTR includes a 5'-cap structure. In some embodiments, the nucleic acid molecules comprise a Kozak sequence (e.g., in 5'-UTR). In some embodiments, the nucleic acid molecules comprise a poly-A region (e.g., in 3'-UTR). In some embodiments, the nucleic acid molecules comprise a polyadenylic acid signal (e.g., in 3'-UTR). In some embodiments, the nucleic acid molecules comprise a conserved region (e.g., in 3'-UTR). In some embodiments, the nucleic acid molecules comprise a secondary structure. In some embodiments, the secondary structure is a stem-loop. In some embodiments, the nucleic acid molecules comprise a stem-loop sequence (e.g., in 5'-UTR and/or 3'-UTR). In some embodiments, the nucleic acid molecules comprise one or more intron regions capable of being excised during a splicing process. In a specific embodiment, the nucleic acid molecules comprise one or more regions selected from the group consisting of 5'-UTR and coding region. In a specific embodiment, the nucleic acid molecules comprise one or more regions selected from the group consisting of coding region and 3'-UTR. In a specific embodiment, the nucleic acid molecules comprise one or more regions selected from the group consisting of 5'-UTR, coding region and 3'-UTR.

### Coding region

In some embodiments, the nucleic acid molecules of the present disclosure comprise at least one coding region. In some embodiments, the coding region is an Open Reading Frame (ORF) encoding single peptide or protein. In some embodiments, the coding region includes at least two ORF, each ORF encoding the peptide or protein. In embodiments that the coding region includes more than one ORF, the peptides and/or proteins encoded by ORF may be the same or different from each other. In some embodiments, multiple ORFs in the coding region are separated by a non-coding sequence. In particular embodiments, the non-coding sequence separating the two ORFs comprises an Internal Ribosome Entry Site (IRES).

It may be expected that the Internal Ribosome Entry Site (IRES) may serve as the sole ribosome binding site, or may serve as one of multiple ribosome binding sites for the mRNA. The mRNA molecules containing more than one functional ribosome binding site may encode several peptides or polypeptides that are independently translated by the ribosome (e.g., polycistronic mRNA). Therefore, in some embodiments, the nucleic acid molecules of the present disclosure (e.g., mRNA) comprise one or more Internal Ribosome Entry Sites (IRES). Examples of IRES sequences that may be used in combination with the present disclosure include, but are not limited to, sequences from Polyomavirus (e.g., FMDV), Pest virus (CFFV), Poliovirus (PV), Encephalomyocarditis Virus (ECMV), Foot-and-Mouth Disease Virus (FMDV), Hepatitis C Virus (HCV), Classical Swine Fever Virus (CSFV), Murine Leukemia Virus (MLV), Simian Immunodeficiency Virus (SIV), or Paralysis Virus (CrPV).

In various embodiments, the nucleic acid molecules according to the present invention encode at least 1, 2, 3, 4, 5, 6, 7, 8, 9, 10 or more than 10 peptides or proteins. The peptides and proteins encoded by the nucleic acid molecule may be the same or different. In some embodiments, the nucleic acid molecules of the present disclosure encode dipeptide (such as carnosine and goose carnosine). In some embodiments, the nucleic acid molecules encode tripeptide. In some embodiments, the nucleic acid molecules encode tetrapeptide. In some embodiments, the nucleic acid molecules encode pentapeptide. In some embodiments, the nucleic acid molecules encode hexapeptide. In some embodiments, the nucleic acid molecules encode heptapeptide. In some embodiments, the nucleic acid molecules encode octapeptide. In some embodiments, the nucleic acid molecules encode nonapeptide. In some embodiments, the nucleic acid molecules encode decapeptide. In some embodiments, the nucleic acid molecules encode peptide or polypeptide having at least about 15 amino acids. In some embodiments, the nucleic acid molecules encode peptide or polypeptide having at least about 50 amino acids. In some embodiments, the nucleic acid molecules encode peptide or polypeptide having at least about 100 amino acids. In some embodiments, the nucleic acid molecules encode peptide or polypeptide having at least about 150 amino acids. In some embodiments, the nucleic acid molecules encode peptide or polypeptide having at least about 300 amino acids. In some embodiments, the nucleic acid molecules encode peptide or polypeptide having at least about 500 amino acids. In some embodiments, the nucleic acid molecules encode peptide or polypeptide having at least about 1000 amino acids.

In some embodiments, the length of the nucleic acid molecules of the present disclosure is at least about 30 nucleotides (nt). In some embodiments, the length of the nucleic acid molecules is at least about 35 nt. In some embodiments, the length of the nucleic acid molecules is at least about 40 nt. In some embodiments, the length of the nucleic acid molecules is at least about 45 nt. In some embodiments, the length of the nucleic acid molecules is at least about 50 nt. In some embodiments, the length of the nucleic acid molecules is at least about 55 nt. In some embodiments, the length of the nucleic acid molecules is at least about 60 nt. In some embodiments, the length of the nucleic acid molecules is at least about 65 nt. In some embodiments, the length of the nucleic acid molecules is at least about 70 nt. In some embodiments, the length of the nucleic acid molecules is at least about 75 nt. In some embodiments, the length of the nucleic acid molecules is at least about 80 nt. In some embodiments, the length of the nucleic acid molecules is at least about 85 nt. In some embodiments, the length of the nucleic acid molecules is at least about 90 nt. In some embodiments, the length of the nucleic acid molecules is at least about 95 nt. In some embodiments, the length of the nucleic acid molecules is at least about 100 nt. In some embodiments, the length of the nucleic acid molecules is at least about 120 nt. In some embodiments, the length of the nucleic acid molecules is at least about 140 nt. In some embodiments, the length of the nucleic acid molecules is at least about 160 nt. In some embodiments, the length of the nucleic acid molecules is at least about 180 nt. In some embodiments, the length of the nucleic acid molecules is at least about 200 nt. In some embodiments, the length of the nucleic acid molecules is at least about 250 nt. In some embodiments, the length of the nucleic acid molecules is at least about 300 nt. In some embodiments, the length of the nucleic acid molecules is at least about 400 nt. In some embodiments, the length of the nucleic acid molecules is at least about 500 nt. In some embodiments, the length of the nucleic acid molecules is at least about 600 nt. In some embodiments, the length of the nucleic acid molecules is at least about 700 nt. In some embodiments, the length of the nucleic acid molecules is at least about 800 nt. In some embodiments, the length of the nucleic acid molecules is at least about 900 nt. In some embodiments, the length of the nucleic acid molecules is at least about 1000 nt. In some embodiments, the length of the nucleic acid molecules is at least about 1100 nt. In some embodiments, the length of the nucleic acid molecules is at least about 1200 nt. In some embodiments, the length of the nucleic acid molecules is at least about 1300 nt. In some embodiments, the length of the nucleic acid molecules is at least about 1400 nt. In some embodiments, the length of the nucleic acid molecules is at least about 1500 nt. In some embodiments, the length of the nucleic acid molecules is at least about 1600 nt. In some embodiments, the length of the nucleic acid molecules is at least about 1700 nt. In some embodiments, the length of the nucleic acid molecules is at least about 1800 nt. In some embodiments, the length of the nucleic acid molecules is at least about 1900 nt. In some embodiments, the length of the nucleic acid molecules is at least about 2000 nt. In some embodiments, the length of the nucleic acid molecules is at least about 2500 nt. In some embodiments, the length of the nucleic acid molecules is at least about 3000 nt. In some embodiments, the length of the nucleic acid molecules is at least about 3500 nt. In some embodiments, the length of the nucleic acid molecules is at least about 4000 nt. In some embodiments, the length of the nucleic acid molecules is at least about 4500 nt. In some embodiments, the length of the nucleic acid molecules is at least about 5000 nt.

In particular embodiments, therapeutic payloads include the vaccine composition (e.g., gene vaccines) described herein. In some embodiments, the therapeutic payloads comprise compounds capable of eliciting immunity against one or more target disorders or diseases. In some embodiments, the target symptoms are associated with pathogens, such as coronaviruses (e.g., 2019-nCoV), influenza, measles, Human Papillomavirus (HPV), rabies, meningitis, pertussis, tetanus, plague, hepatitis, and tuberculosis, or infections caused thereby. In some embodiments, the therapeutic payloads include nucleic acid sequences (e.g., mRNA) encoding pathogen-specific pathogenic proteins or antigenic fragments or epitopes thereof. The vaccines express the encoded pathogenic proteins (or antigenic fragments or epitopes thereof) upon inoculation to the subject, thereby eliciting immunity against the pathogen in the subject.

In some embodiments, the target disorder is associated with or caused by neoplastic growth of cells, such as cancer. In some embodiments, the therapeutic payload comprises a nucleic acid sequence (e.g., mRNA) encoding cancer-characteristic Tumor Associated Antigen (TAA) or an antigenic fragment or epitope thereof. The vaccine expresses the encoded TAA (or antigenic fragment or epitope thereof) upon administration to a vaccinated subject, thereby eliciting immunity against TAA-expressing tumor cells in the subject.

### 5'-cap structure

It may be expected that the 5'-cap structure of polynucleotide participates in nuclear export and improves the stability of the polynucleotide, and binds to mRNA Cap Binding Protein (CBP) responsible for the stability of the polynucleotide in the cells. Mature cyclic mRNA is formed by binding of CBP and poly-A binding protein, thereby obtaining translation ability. The 5'-cap structure further assists in removal of intron at 5' end during mRNA splicing. Therefore, in some embodiments, the nucleic acid molecules of the present disclosure comprise 5'-cap.

The nucleic acid molecules may be 5' end capped by the endogenous transcription mechanism of cells, thereby producing a 5'-ppp-5'-triphosphate bond between the guanine cap terminal residue and the transcribed sense nucleotide at the 5' end of the polynucleotide. This 5'-guanylate cap is then methylated to produce an N7-methyl-guanylate residue. The ribose of the terminal and/or pre-terminal transcribed nucleotide at 5' end of the polynucleotide may also optionally be 2'-O-methylated. 5'-decapping by hydrolysis and cleavage of the guanylate cap structure may target the nucleic acid molecules, such as mRNA molecules, for degradation.

In some embodiments, the nucleic acid molecules of the present disclosure comprise one or more changes in the native 5'-cap structure produced by the endogenous process. Modification of the 5'-cap may increase the stability of the polynucleotide, increase the half-life of the polynucleotide, and may increase the translation efficiency of the polynucleotide.

Exemplary changes to the native 5'-Cap structure comprise the production of a non-hydrolysable cap structure, thereby preventing uncapping and increasing the half-life of the polynucleotide. In some embodiments, as hydrolysis of the cap structure requires the cleavage of the 5'-ppp-5' phosphodiester bond, the modified nucleotides may be used during the end capping reaction in some embodiments. For example, in some embodiments, a vaccinia capping enzyme from New England Biolabs may be used with α-thioguanosine nucleotide in accordance with the manufacturer specification to produce a phosphorothioate linkage in 5'-ppp-5'. Other modified guanosine nucleotides, such as α-methylphosphonates and selenophosphate nucleotides, may also be used.

Other exemplary changes to the native 5'-Cap structure also include the modification at the 2'- and/or 3'-position of the capped Guanosine Triphosphate (GTP), replacement of the sugar epoxy (oxygen involved in the carbocyclic ring) with the methylene moiety (CH2), the modification of the triphosphate bridge moiety of the cap structure, or the modification of the nucleobase (G) moiety.

Other exemplary changes to the native 5'-Cap structure include, but are not limited to, 2'-O-methylation of the 5'-terminal and/or 5'-terminal nucleic acids of the polynucleotide on the 2'-hydroxy group of the ribose, which can produce multiple different 5'-Cap structures of the polynucleotide (e.g., mRNA molecules). Additional exemplary 5'-Cap structures that may be used in combination with the present disclosure also include those described in International Patent Publication Numbers WO2008127688, WO 2008016473 and WO 2011015347, all of which are incorporated herein by reference in their entirety.

In various embodiments, 5'-caps may include cap analogs. The cap analogs, also referred to herein as synthetic cap analogs, chemical caps, chemical cap analogs or structural or functional cap analogs, are chemically different from the native (i.e., endogenous, wild-type or physiological) 5'-cap structure while retaining the function of the cap. The cap analogs may be chemically (i.e., non-enzymatically) or enzymatically synthesized and/or linked to polynucleotides.

For example, the Anti-Reverse Cap Analog (ARCA) cap comprises two guanosine linked by a 5'-5'-triphosphate group, one of which comprises N7-methyl and 3'-O-methyl (i.e., N7,3'-O-dimethyl-guanosine-5'-triphosphate-5'-guanosine, m7G-3'mppp-G, which may be equivalently referred to as 3'O-Me-m7G (5') ppp (5') G). The 3'-O atom of the other unchanged guanosine is linked to the 5'-terminal nucleotides of capped polynucleotide (e.g., mRNA). The N7-and 3'-O-methylated guanosines provide a terminal portion of the capped polynucleotide (e.g., mRNA). Another exemplary cap structure is mCAP, which is similar to ARCA, but has 2'-O-methyl on the guanosine (i.e., (N7,2'-O-dimethyl-guanosine-5'-triphosphate-5'-guanosine, m₇Gm-ppp-G).

In some embodiments, the cap analogs may be dinucleotide cap analogs. As a non-limiting example, the dinucleotide cap analogs may be modified at different phosphoric acid positions by a boronic acid phosphate group or a phosphoselenate group, e.g., the analogs of the dinucleotide cap described in U.S. Patent No. 8,519,110, which is incorporated herein by reference in its entirety.

In some embodiments, the cap analogs may be N7-(4-chlorophenoxyethyl) substituted dinucleotide cap analogs known in the art and/or described herein. Non-limiting examples of N7-(4-chlorophenoxyethyl) substituted dinucleotide cap analogs include N7-(4-chlorophenoxyethyl)-G (5')ppp(5')G and N7-(4-chlorophenoxyethyl)-m3'-OG (5')ppp(5')G cap analogs (e.g., see various cap analogs and methods of synthesizing the cap analogs described in Kore et al. Bioorganic & Medicinal Chemistry 2013 21:4570-4574, which is incorporated herein by reference). In other embodiments, the cap analogs of nucleic acid molecules that can be used in the present disclosure are 4-chloro/bromophenoxyethyl analogs.

In various embodiments, the cap analogs can include guanosine analogs. Available guanosine analogs include, but are not limited to, inosine, N1-methyl-guanosine, 2'-fluoro-guanosine, 7-deaza-guanosine, 8-oxo-guanosine, 2-amino-guanosine, LNA-guanosine, and 2-azido.

It can be expected that up to 20% of the transcripts remain uncapped although the cap analogs allow simultaneous capping of the polynucleotides in the in vitro transcription reactions. This differs from the structure of cap analogs of the native 5'-cap structure of polynucleotides produced from the cellular endogenous transcription mechanism, which may lead to reduced translation ability and reduced cell stability.

Therefore, in some embodiments, the nucleic acid molecules of the present disclosure may also be capped after transcription using enzymes to produce a more authentic 5'-cap structure. As used herein, the phrase "more authentic" refers to characteristics that closely reflect or simulate endogenous or wild-type features structurally or functionally. That is, a "more authentic" characteristic represents a better endogenous, wild-type, native, or physiological cell function and/or structure than the synthetic or analogues thereof of prior art, or a native type, native, or physiological characteristic that performs better than one or more aspects of the corresponding endogenous, wild-type. Non-limiting examples of more authentic 5'-cap structures used in combination with the nucleic acid molecules of the present disclosure are those with enhanced binding of cap-binding protein, increased half-life, and decreased sensitivity to 5'. 5'-uncapping of β-endonuclease is reduced compared to synthetic 5'-cap structures known in the art (or wild-type, native, or physiological 5'-cap structures). For example, in some embodiments, the recombinant vaccinia virus capping enzyme and the recombinant 2'-O-methyltransferase generate canonical 5'-5'-triphosphate linkages between the 5'-terminal nucleotide of the polynucleotide and the guanosine cap nucleotide. Cap guanine comprises N7-methylation, while the 5'-terminal nucleotide of polynucleotides contains 2'-O-methyl. This structure is called Cap1 structure. This cap results in higher translational capacity, cell stability, and reduced activation of cellular pro-inflammatory cytokines compared to, for example, other 5' cap analogue structures known in the art. Other exemplary capping structures include 7mG(5')ppp(5')N,pN2p (Cap 0), 7mG(5')ppp(5')NlmpNp (Cap 1), 7mG(5')-ppp(5')NlmpN2mp (Cap 2), and m(7)Gpppm(3)(6,6,2')Apm(2')Apm(2')Cpm(2)(3,2')Up (Cap 4).

It may be expected that the nucleic acid molecules of the present disclosure may be capped after transcription, and nearly 100% of the nucleic acid molecules may be capped as the process is more efficient.

### Untranslated regions (UTRs)

In some embodiments, the nucleic acid molecules of the present disclosure comprise one or more untranslated regions (UTRs). In some embodiments, the UTRs are located upstream of the coding region of the nucleic acid molecule, referred to as 5'-UTRs. In some embodiments, the UTRs are located downstream of the coding region of the nucleic acid molecules, referred to as 3'-UTRs. The sequences of the UTRs may be homologous or heterologous to the sequences of the coding region in the nucleic acid molecules. The nucleic acid molecules may include multiple UTRs, which may have the same or different sequences and/or genetic origins. According to the present invention, any portion of the UTRs in the nucleic acid molecule (including the absence thereof) may be codon-optimized and may independently comprise one or more different structural or chemical modifications, prior to and/or after codon-optimization.

In some embodiments, the nucleic acid molecules of the present disclosure (e.g., mRNA) comprise UTRs and an coding regions that are homologous to each other. In other embodiments, the nucleic acid molecules of the present disclosure (e.g., mRNA) comprise UTRs and an coding region that are heterologous with respect to each other. In some embodiments, to detect the activity of the UTR sequence, nucleic acid molecules comprising UTR and a coding sequence for detectable probe may be administered in vitro (e.g., cell or tissue culture) or in vivo (e.g., to the subject). The effects of the UTR sequence (e.g., regulation of expression levels, cellular localization of the encoded product, or half-life of the encoded product) may be detected using methods known in the art.

In some embodiments, the UTRs of nucleic acid molecules of the present disclosure (e.g., mRNA) comprises at least one Translation Enhancer Element (TEE) that functions to increase the production of polypeptides or proteins produced from the nucleic acid molecule. In some embodiments, the TEE is located in the 5'-UTR of the nucleic acid molecule. In other embodiments, the TEE is located at the 3'-UTR of the nucleic acid molecules. In other embodiments, at least two TEEs are located at the 5'-UTR and the 3'-UTR of the nucleic acid molecules, respectively. In some embodiments, the nucleic acid molecules of the present disclosure (e.g., mRNA) may comprise one or more copies of the TEE sequence or comprise more than one different TEE sequence. In some embodiments, the different TEE sequences in the nucleic acid molecule may be homologous or heterologous to each other.

Various TEE sequences which may be used in combination with the present disclosure are known in the art. For example, in some embodiments, the TEE may be an Internal Ribosome Entry Site (IRES), an HCV-IRES or IRES element. Chappell et al. Chappell et al. Proc. Natl. Acad. Sci. USA 101:9590-9594, 2004; Zhou et al. Proc. Natl. Acad. Sci. 102:6273-6278, 2005. Additional internal ribosomal entry sites (IRES) that may be used in combination with the present disclosure include, but are not limited to, those disclosed in U.S. Patent No. 7,468,275, U.S. Patent Publication No. 2007/0048776 and U.S. Patent Publication No. 2011/0124100, and International Patent Publication No. WO2007/025008 and International Patent Publication No. WO2001/055369, all of which are incorporated herein by reference in their entirety. In some embodiments, the TEE may be those described in supplementary Tables 1 and 2 of Wellensiek et al Genome-wide profiling of human cap-independent translation-enhancing elements, Nature Methods, 2013 Aug; 10(8):747-750, the contents of which are incorporated herein by reference in their entirety.

Additional exemplary TEEs that may be used in combination with the present disclosure include, but are not limited to, TEE sequences disclosed in the U.S. Patent No. 6,310,197, U.S. Patent No. 6,849,405, U.S. Patent No. 7,456,273, U.S. Patent No. 7,183,395, U.S. Patent No. 2009/0226470, U.S. Patent Publication No. 2013/0177581, U.S. Patent Publication No. 2007/0048776, U.S. Patent Publication No. 2011/ 0124100, U.S. Patent No. 2009/0093049, International Patent Publication No. WO2009/075886, International Patent Publication No. WO2012/009644 and International Patent Publication No. WO1999/024595, International Patent Publication No. WO2007/025008, International Patent Publication No. WO2001/ 055371, European Patent No. 2610341 and the European Patent No. 2610340, all of which are incorporated herein by reference in their entirety.

In various embodiments, the nucleic acid molecules of the present disclosure (e.g., mRNA) comprise at least one UTR, including at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 30, at least 35, at least 40, at least 45, at least 50, at least 55 or more than 60 TEE sequences. In some embodiments, the TEE sequences in the UTR of the nucleic acid molecules are copies of the same TEE sequences. In other embodiments, at least two TEE sequences in the UTR of the nucleic acid molecule have different sequences. In some embodiments, multiple different TEE sequences are arranged in one or more repetitive patterns in the UTR region of the nucleic acid molecules. For illustrative purposes only, the repetitive patterns may be for example, ABABAB, ABABBAABBAABB, ABCABCABC, etc., and in these exemplary patterns, each capital letter (A, B or C) represents a different TEE sequence. In some embodiments, at least two TEE sequences are consecutive to each other in the UTR of the nucleic acid molecules (i.e., there is no spacer sequence therebetween). In other embodiments, at least two TEE sequences are separated by a spacer sequence. In some embodiments, the UTR may comprise a TEE sequence-spacer sequence module that repeats at least once, at least twice, at least three times, at least four times, at least five times, at least six times, at least seven times, at least eight times, at least nine times or more than nine times. In any embodiment described in this paragraph, the UTR may be 5'-UTR, 3'-UTR, or both 5'-UTR and 3'-UTR of the nucleic acid molecules.

In some embodiments, the UTR of nucleic acid molecules of the present disclosure (e.g., mRNA) comprises at least one translation suppressor element that functions to reduce the amount of polypeptide or protein produced from the nucleic acid molecule. In some embodiments, the UTR of the nucleic acid molecules comprise one or more miR sequences recognized by one or more microRNAs or fragments thereof (e.g., miR seed sequences). In some embodiments, the UTR of the nucleic acid molecules comprise one or more stem-loop structures that down-regulate the translation activity of the nucleic acid molecule. Other mechanisms for inhibiting the translational activity associated with the nucleic acid molecule are known in the art. In any embodiment described in this paragraph, the UTR may be 5'-UTR, 3'-UTR, or both 5'-UTR and 3'-UTR of the nucleic acid molecules.

### Polyadenylated (Poly-A) region

During natural RNA processing, a long-chain adenosine nucleotide (poly-A) region is usually added to a messenger RNA (mRNA) molecule to increase the stability of the molecule. After transcription, the 3'-end of transcript is immediately cleaved to release 3'-hydroxy. Then, the poly-A polymerase adds an adenosine nucleotide chain to the RNA. The process is referred to as polyadenylation, with a poly-A region having a length of 100 to 250 residues being added. It may be expected that the poly-A region can impart various advantages to the nucleic acid molecule of the present invention.

Therefore, in some embodiments, the nucleic acid molecules (e.g., mRNA) of the disclosure comprise a polyadenylation signal. In some embodiments, the nucleic acid molecules (e.g., mRNA) of the disclosure comprise one or more polyadenylation (poly-A) regions. In some embodiments, the poly-A region is composed entirely of adenine nucleotide or a functional analogue thereof. In some embodiments, the nucleic acid molecules comprise at least one poly-A region at 3' end thereof. In some embodiments, the nucleic acid molecules comprise at least one poly-A region at 5' end thereof. In some embodiments, the nucleic acid molecules comprise at least one poly-A region at 5' end thereof and at least one poly-A region at 3' end thereof.

According to the present invention, in different embodiments, the poly-A region may have varying lengths. In particular, in some embodiments, the length of the poly-A region of the nucleic acid molecules of the present disclosure is at least 30 nucleotides. In some embodiments, the length of the poly-A region of the nucleic acid molecules of the present disclosure is at least 35 nucleotides. In some embodiments, the length of the poly-A region of the nucleic acid molecules of the present disclosure is at least 40 nucleotides. In some embodiments, the length of the poly-A region of the nucleic acid molecules of the present disclosure is at least 45 nucleotides. In some embodiments, the length of the poly-A region of the nucleic acid molecules of the present disclosure is at least 50 nucleotides. In some embodiments, the length of the poly-A region of the nucleic acid molecules of the present disclosure is at least 55 nucleotides. In some embodiments, the length of the poly-A region of the nucleic acid molecules of the present disclosure is at least 60 nucleotides. In some embodiments, the length of the poly-A region of the nucleic acid molecules of the present disclosure is at least 65 nucleotides. In some embodiments, the length of the poly-A region of the nucleic acid molecules of the present disclosure is at least 70 nucleotides. In some embodiments, the length of the poly-A region of the nucleic acid molecules of the present disclosure is at least 75 nucleotides. In some embodiments, the length of the poly-A region of the nucleic acid molecules of the present disclosure is at least 80 nucleotides. In some embodiments, the length of the poly-A region of the nucleic acid molecules of the present disclosure is at least 85 nucleotides. In some embodiments, the length of the poly-A region of the nucleic acid molecules of the present disclosure is at least 90 nucleotides. In some embodiments, the length of the poly-A region of the nucleic acid molecules of the present disclosure is at least 95 nucleotides. In some embodiments, the length of the poly-A region of the nucleic acid molecules of the present disclosure is at least 100 nucleotides. In some embodiments, the length of the poly-A region of the nucleic acid molecules of the present disclosure is at least 110 nucleotides. In some embodiments, the length of the poly-A region of the nucleic acid molecules of the present disclosure is at least 120 nucleotides. In some embodiments, the length of the poly-A region of the nucleic acid molecules of the present disclosure is at least 130 nucleotides. In some embodiments, the length of the poly-A region of the nucleic acid molecules of the present disclosure is at least 140 nucleotides. In some embodiments, the length of the poly-A region of the nucleic acid molecules of the present disclosure is at least 150 nucleotides. In some embodiments, the length of the poly-A region of the nucleic acid molecules of the present disclosure is at least 160 nucleotides. In some embodiments, the length of the poly-A region of the nucleic acid molecules of the present disclosure is at least 170 nucleotides. In some embodiments, the length of the poly-A region of the nucleic acid molecules of the present disclosure is at least 180 nucleotides. In some embodiments, the length of the poly-A region of the nucleic acid molecules of the present disclosure is at least 190 nucleotides. In some embodiments, the length of the poly-A region of the nucleic acid molecules of the present disclosure is at least 200 nucleotides. In some embodiments, the length of the poly-A region of the nucleic acid molecules of the present disclosure is at least 225 nucleotides. In some embodiments, the length of the poly-A region of the nucleic acid molecules of the present disclosure is at least 250 nucleotides. In some embodiments, the length of the poly-A region of the nucleic acid molecules of the present disclosure is at least 275 nucleotides. In some embodiments, the length of the poly-A region of the nucleic acid molecules of the present disclosure is at least 300 nucleotides. In some embodiments, the length of the poly-A region of the nucleic acid molecules of the present disclosure is at least 350 nucleotides. In some embodiments, the length of the poly-A region of the nucleic acid molecules of the present disclosure is at least 400 nucleotides. In some embodiments, the length of the poly-A region of the nucleic acid molecules of the present disclosure is at least 450 nucleotides. In some embodiments, the length of the poly-A region of the nucleic acid molecules of the present disclosure is at least 500 nucleotides. In some embodiments, the length of the poly-A region of the nucleic acid molecules of the present disclosure is at least 600 nucleotides. In some embodiments, the length of the poly-A region of the nucleic acid molecules of the present disclosure is at least 700 nucleotides. In some embodiments, the length of the poly-A region of the nucleic acid molecules of the present disclosure is at least 800 nucleotides. In some embodiments, the length of the poly-A region of the nucleic acid molecules of the present disclosure is at least 900 nucleotides. In some embodiments, the length of the poly-A region of the nucleic acid molecules of the present disclosure is at least 1000 nucleotides. In some embodiments, the length of the poly-A region of the nucleic acid molecules of the present disclosure is at least 1100 nucleotides. In some embodiments, the length of the poly-A region of the nucleic acid molecules of the present disclosure is at least 1200 nucleotides. In some embodiments, the length of the poly-A region of the nucleic acid molecules of the present disclosure is at least 1300 nucleotides. In some embodiments, the length of the poly-A region of the nucleic acid molecules of the present disclosure is at least 1400 nucleotides. In some embodiments, the length of the poly-A region of the nucleic acid molecules of the present disclosure is at least 1500 nucleotides. In some embodiments, the length of the poly-A region of the nucleic acid molecules of the present disclosure is at least 1600 nucleotides. In some embodiments, the length of the poly-A region of the nucleic acid molecules of the present disclosure is at least 1700 nucleotides. In some embodiments, the length of the poly-A region of the nucleic acid molecules of the present disclosure is at least 1800 nucleotides. In some embodiments, the length of the poly-A region of the nucleic acid molecules of the present disclosure is at least 1900 nucleotides. In some embodiments, the length of the poly-A region of the nucleic acid molecules of the present disclosure is at least 2000 nucleotides. In some embodiments, the length of the poly-A region of the nucleic acid molecules of the present disclosure is at least 2250 nucleotides. In some embodiments, the length of the poly-A region of the nucleic acid molecules of the present disclosure is at least 2500 nucleotides. In some embodiments, the length of the poly-A region of the nucleic acid molecules of the present disclosure is at least 2750 nucleotides. In some embodiments, the length of the poly-A region of the nucleic acid molecules of the present disclosure is at least 3000 nucleotides.

In some embodiments, the length of the poly-A region in the nucleic acid molecules may be selected based on the total length of the nucleic acid molecule or the portion thereof (e.g., the length of the coding region or the length of the open reading frame). For example, in some embodiments, the poly-A region accounts for about 5%, 10%, 15%, 20%, 25%, 30%, 35%, 40%, 45%, 50%, 55%, 60%, 65%, 70%, 75%, 80%, 85%, 90%, 95% or more of the total length of the nucleic acid molecule comprising the poly-A region.

It may be expected that certain RNA binding proteins may be bound to the poly-A region located at the 3 'end of the mRNA molecules. These poly-A binding proteins (PABP) may modulate mRNA expression, e.g., interact with translation initiation mechanisms in cells and/or protect the 3'-poly-A tail from degradation. In some embodiments, the nucleic acid molecules of the present disclosure (e.g., mRNA) comprise at least one binding site for the poly-A binding protein (PABP). In other embodiments, the nucleic acid molecules are conjugated or complexed with PABP prior to loading into a delivery vector (e.g., lipid nanoparticles).

In some embodiments, the nucleic acid molecules of the present disclosure (e.g., mRNA) comprise a poly-A-G tetramer. The G tetramer is a cyclic hydrogen bond array of four guanosine nucleotides, which may be formed from G-rich sequences in DNA and RNA. In this embodiment, the G tetramer is bound to the end of the poly-A region. The stability, protein production, and other parameters of the resulting polynucleotide (e.g., mRNA), including half-lives at different time points, may be determined. The studies have shown that the yield of the protein produced by the polyA-G tetramer structure at least equal to 75% of that of the protein produced by using 120 nucleotides of the poly-A region alone.

In some embodiments, the nucleic acid molecules of the present disclosure (e.g., mRNA) may include the poly-A region and may be stabilized by the addition of 3' stabilizing region. In some embodiments, the 3' stabilizing region that can be used for stabilizing the nucleic acid molecules (e.g., mRNA), including poly-A or poly-A-G tetrameric structures, is documented in the International Patent Publication No. WO2013/103659, which is incorporated herein by reference in its entirety.

In other embodiments, the 3' stabilizing regions that can be used in combination with the nucleic acid molecules of the present invention include chain-terminating nucleosides, such as, but not limited to, 3'-deoxyadenosine (cordycepin), 3'-deoxyuridine, 3'-deoxycytosine, 3'-deoxyguanosine, 3'-deoxythymine, 2',3'-dideoxynucleoside, 2',3'-dideoxyadenosine, 2',3'-dideoxyuridine, 2',3'-dideoxycytosine, 2',3'-dideoxyguanosine, 2',3'-dideoxythymine, 2'-deoxynucleoside or O-methylnucleoside, 3'-deoxynucleoside, 2',3'-dideoxynucleoside 3'-O-methylnucleoside, 3'-O-ethylnucleoside, 3'-arabinoside, and other alternative nucleosides described herein or known in the art.

### Secondary structure

The stem-loop structure may guide RNA folding, protect the structural stability of nucleic acid molecules (such as mRNA), provide recognition sites for RNA binding proteins and be used as an enzymatic reaction substrate. For example, the shape of the stem-loop region will be altered by integration of the miR sequence and/or TEE sequence, which may increase and/or decrease translation (Kedde *et al.* A Pumilio-induced RNA structure switch in p27-3'UTR controls miR-221 and miR-222 accessibility. Nat Cell Biol., 2010 Oct; 12(10):1014-20, the content of which is incorporated herein by reference in its entirety).

Therefore, in some embodiments, the nucleic acid molecules described herein (e.g., mRNA), or a portion thereof, can take a stem-loop structure, such as, but not limited to, a histone stem-loop. In some embodiments, the stem-loop structure is formed from a stem-loop sequence of about 25 or about 26 nucleotides in length, which may be, but not limited to, those described in International Patent Publication No. WO2013/103659, which is incorporated herein by reference in its entirety. Other examples of stem-loop sequences include those described in International Patent Publication No. WO2012/019780 and International Patent Publication No. WO201502667, the contents of which are incorporated herein by reference. In some embodiments, the stem-loop sequences include TEE as described herein. In some embodiments, the stem-loop sequences include miR sequences as described herein. In particular embodiments, the stem-loop sequences may include miR-122 seed sequences. In particular embodiments, the nucleic acid molecules include a stem-loop sequence CAAAGGCTCTTTTCAGAGCCACCA(SEQ ID NO: 1). In other embodiments, the nucleic acid molecules include a stem-loop sequence CAAAGGCUCUUUUCAGAGCCACCA (SEQ ID NO:2).

In some embodiments, the nucleic acid molecules of the present disclosure (e.g., mRNA) include a stem-loop sequence located upstream (5 'end) of the coding region of the nucleic acid molecules. In some embodiments, the stem-loop sequence is located within 5'-UTR of the nucleic acid molecule. In some embodiments, the nucleic acid molecules of the present disclosure (e.g., mRNA) comprise a stem-loop sequence located downstream (3' end) of the coding region of the nucleic acid molecule. In some embodiments, the stem-loop sequence is located within 3'-UTR of the nucleic acid molecule. In some cases, the nucleic acid molecule may comprise more than one stem-loop sequences. In some embodiments, the nucleic acid molecules comprise at least one stem-loop sequence in 5'-UTR and at least one stem-loop sequence in 3'-UTR.

In some embodiments, the nucleic acid molecules including the stem-loop structure further comprise a stabilizing region. In some embodiments, the stabilizing region includes at least one chain-terminating nucleoside that acts to slow degradation and thus increase the half-life of the nucleic acid molecule. Exemplary strand-terminating nucleosides that may be used in combination with the present disclosure include, but are not limited to, 3'-deoxyadenosine (cordycepin), 3'-deoxyuridine, 3'-deoxycytosine, 3'-deoxyguanosine, 3'-deoxythymine, 2',3'-dideoxynucleoside, 2',3'-dideoxyadenosine, 2',3'-dideoxyuridine, 2',3'-dideoxycytosine, 2',3'-dideoxyguanosine, 2',3'-dideoxythymine, 2'-deoxynucleoside or O-methylnucleoside, 3'-deoxynucleoside, 2',3'-dideoxynucleoside 3'-O-methylnucleoside, 3'-O-ethylnucleoside, 3'-arabinoside, and other alternative nucleosides described herein or known in the art. In other embodiments, the stem-loop structure may be stabilized by altering the 3' region of the polynucleotide, and the alteration may prevent and/or inhibit the addition of oligio (U) (International Patent Publication No. WO2013/103659, which is incorporated herein by reference in its entirety).

In some embodiments, the nucleic acid molecules of the present disclosure comprise at least one stem-loop sequence and the poly-A region or polyadenylation signal. Non-limiting examples of the polynucleotide sequence comprising at least one stem-loop sequence and the poly-A region or polyadenylation signal are described in International Patent Publication No. WO2013/120497, International Patent Publication No. WO2013/120629, International Patent Publication No. WO2013/120500, No. WO2013/120627 International Patent, No. WO2013/120498 International Patent, International Patent Publication No. WO2013/120626, International Patent Publication No. WO2013/120499 and International Patent Publication No. WO2013/120628, all of which are incorporated herein by reference in their entirety.

In some embodiments, the nucleic acid molecules comprising the stem-loop sequence and the poly-A region or the polyadenylation signal may encode a pathogen antigen or fragment thereof, as described in International Patent Publication No. WO2013/120499 and International Patent Publication No. WO2013/120628, all of which are incorporated herein by reference in their entirety.

In some embodiments, the nucleic acid molecules comprising the stem-loop sequence and the poly-A region or polyadenylation signal may encode a therapeutic protein, as described in International Patent Publication No. WO2013/120497 and International Patent Publication No. WO2013/120629, all of which are incorporated herein by reference in their entirety.

In some embodiments, the nucleic acid molecules comprising the stem-loop sequence and the poly-A region or polyadenylation signal may encode a tumor antigen or fragment thereof, as described in International Patent Publication No. WO2013/120500 and International Patent Publication No. WO2013/120627, all of which are incorporated herein by reference in their entirety.

In some embodiments, the nucleic acid molecules comprising the stem-loop sequence and the poly-A region or polyadenylation signal may encode an allergen antigen or an autoimmune autoantigen, as described in International Patent Publication No. WO2013/120498 and International Patent Publication No. WO2013/120626, all of which are incorporated herein by reference in their entirety.

### Functional nucleotide analogs

In some embodiments, the nucleic acid molecules comprising the stem-loop sequence and the poly-A region or polyadenylation signal may encode an allergen antigen or an autoimmune autoantigen, as described in International Patent Publication No. WO2013/120498 and International Patent Publication No. WO2013/120626, all of which are incorporated herein by reference in their entirety.

Therefore, in some embodiments, the payload nucleic acid molecules comprise at least one functional nucleotide analog described herein. In some embodiments, the functional nucleotide analog comprises at least one chemical modification of a nucleobase, a glycosyl group, and/or a phosphate group. Thus, the payload nucleic acid molecule comprising at least one functional nucleotide analog includes at least one chemical modification of nucleobase, glycosyl, and/or a nucleoside bond. Exemplary chemical modifications of a nucleobase, glycosyl, or nucleoside bond of the nucleic acid molecules are provided herein.

As described herein, all nucleotides in the payload nucleic acid molecule ranging from 0% to 100% may be functional nucleotide analogues as described herein. For example, in various embodiments, from about 1% to about 20%, from about 1% to about 25%, from about 1% to about 50%, from about 1% to about 60%, from about 1% to about 70%, from about 1% to about 80%, about 1% to about 90%, about 1% to about 95%, about 10% to about 20%, about 10% to about 25%, about 10% to about 50%, about 10% to about 60%, about 10% to about 70%, about 10% to about 80%, about 10% to about 90%, about 10% to about 95%, about 10% to about 100%, about 20% to about 25%, about 20% to about 50%, about 20% to about 60%, about 20% to about 70%, about 20% to about 80%, about 20% to about 90%, about 20% to about 95%, about 20% to about 100%, about 50% to about 60%, about 50% to about 70%, about 50% to about 80%, about 50% to about 90%, about 50% to about 95%, about 50% to about 100%, about 70% to about 80%, about 70% to about 90%, about 70% to about 95%, about 70% to about 100%, about 80% to about 90%, about 80% to about 95%, about 80% to about 100%, about 90% to about 95%, about 90% to about 100%, or about 95% to about 100% are functional nucleotide analogues described herein. In any of these embodiments, the functional nucleotide analogues may be present at any location of the nucleic acid molecule, including the 5'-end, 3'-end, and/or one or more internal positions. In some embodiments, the single nucleic acid molecule may comprise different sugar modifications, different nucleobase modifications, and/or different types of nucleosidic bonds (e.g., backbone structures).

As described herein, 0% to 100% of all nucleotides in a type may be functional nucleotide analogues as described herein (e.g., all purine-containing nucleotides of one type, or all pyrimidine-containing nucleotides of one type, or the payload nucleic acid molecules with all A, G, C, T, or U ranging from 0% to 100% "as one type"). For example, in various embodiments, from about 1% to about 20%, from about 1% to about 25%, from about 1% to about 50%, from about 1% to about 60%, from about 1% to about 70%, from about 1% to about 80%, about 1% to about 90%, about 1% to about 95%, about 10% to about 20%, about 10% to about 25%, about 10% to about 50%, about 10% to about 60%, about 10% to about 70%, about 10% to about 80%, about 10% to about 90%, about 10% to about 95%, about 10% to about 100%, about 20% to about 25%, about 20% to about 50%, about 20% to about 60%, about 20% to about 70%, about 20% to about 80%, about 20% to about 90%, about 20% to about 95%, about 20% to about 100%, about 50% to about 60%, about 50% to about 70%, about 50% to about 80%, about 50% to about 90%, about 50% to about 95%, about 50% to about 100%, about 70% To about 80%, about 70% to about 90%, about 70% to about 95%, about 70% to about 100%, about 80% to about 90%, about 80% to about 95%, about 80% to about 100%, about 90% to about 95%, about 90% to about 100%, or about 95% to about 100% are functional nucleotide analogues described herein. In any of these embodiments, the functional nucleotide analogues may be present at any location of the nucleic acid molecule, including the 5'-end, 3'-end, and/or one or more internal positions. In some embodiments, the single nucleic acid molecule may contain different sugar modifications, different nucleobase modifications, and/or different types of nucleosidic bonds (e.g., backbone structures).

### Modification of bases

In some embodiments, the functional nucleotide analogs comprise non-standard nucleobases. In some embodiments, standard nucleobases (e.g., adenine, guanine, uracil, thymine, and cytosine) in nucleotides may be modified or replaced to provide one or more functional analogs of the nucleotides. Exemplary modifications of nucleobases include, but are not limited to, one or more substitutions or modifications including, but are not limited to, alkyl, aryl, halogen, oxo, hydroxy, alkoxy, and/or thio-substitutions; one or more fused or open ring, oxidation, and/or reduction.

In some embodiments, the non-standard nucleobase is modified uracil. Exemplary nucleobases and nucleosides with modified uracil include pseudo-uridine (ψ), pyridin-4-one nucleoside, 5-azauracil, 6-azauracil, 2-thio-5-azauracil, 2-thiouracil (s²U), 4-thio-uracil (s⁴U), 4-thio-pseudouridine, 2-thio-pseudouridine, 5-hydroxy-uracil (ho⁵U), 5-aminoallyl-uracil, 5-halo-uracil (e.g., 5-iodo-uracil or 5-bromouracil), 3-methyluracil (m³U), 5-methoxyuracil (mo⁵U), uracil 5-oxoacetic acid (cmo⁵U), uracil methyl 5-oxoacetate (mcmo⁵U), 5-carboxymethyl-uracil (cm⁵U), 1-carboxymethyl-pseudouridine, 5-carboxyhydroxymethyl-uracil (chm⁵U), 5-carboxymethyl-uracil methyl ester (mchm⁵U), 5-methoxycarbonylmethyluracil (mcm⁵U), 5-methoxycarbonylmethyl-2-thiouracil (mcm⁵s²U), 5-aminomethyl-2-thiouracil (nm⁵s²U), 5-methylaminomethyl-2-uracil (mnm⁵U), 5-methylaminomethyl-2-thiouracil (mnm⁵s²U), 5-methylaminomethyl-2-selenouracil (mnm⁵se²U), 5-carbamoylmethyluracil (ncm5U), 5-carboxymethylaminomethyluracil (cmnm⁵U), 5-carboxymethylaminomethyl-2-thiouracil (cmnm⁵s²U), 5-propynyluracil, 1-propynyl-pseudouracil, methyluracil 5-taurate (τm⁵U), methyl-pseudouridine 1-taurate, methyl-2-thiouracil 5-taurate (τm⁵s²U), 1-taurinylmethyl-4-thio-pseudouridine, 5-methyl-uracil (m⁵U, i.e., with nucleobase deoxythymine), 1-methyl-pseudoneuroside (m¹ψ), 1-ethyl-pseudoneuroside (Et¹ψ), 5-methyl-2-thio-uracil (m⁵s²U), 1-methyl-4-thio-pseudouridine (m¹s⁴ψ), 4-thio-1-methyl-pseudouridine, 3-methyl-pseudouridine (m³ψ), 2-thio-1-methyl-pseudouridine, 1-methyl-1-deaza-pseudouridine, 2-thio-1-methyl-1-deaza-pseudouridine, dihydrouracil (D), dihydropseudouridine, 5,6-dihydrouracil, 5-methyl-dihydrouracil (m⁵D), 2-thio-dihydrouracil, 2-thio-dihydropseudouridine, 2-methoxy-uracil, 2-methoxy-4-thiouracil, 4-methoxy-pseudouridine, 4-methoxy-2-thiopseudouridine, N1-methyl-pseudouridine, 3-(3-amino-3-carboxypropyl)uracil (acp³U), 1-methyl-3-(3-amino-3-carboxypropyl)pseudouridine (acp³ψ), 5-(prenylaminomethyl)uracil (m⁵U), 5-(prenyl)aminomethyl)-2-thiouracil (m⁵s²U), 5,2'-O-dimethyluridine (m⁵Um), 2-thio-2'-O-methyluridine (s²Um), 5-methoxycarbonylmethyl-2'-O-methyluridine (mcm⁵Um), 5-carbamoylmethyl-2'-O-methyluridine (ncm⁵Um), 5-carboxymethylaminomethyl-2'-O-methyluridine (cmnm⁵Um), 3,2'-O-dimethyluridine (m³Um), and 5-(prenylaminomethyl)- 2'-O-methyl-uridine (inm⁵Um), 1-thio-uracil, deoxythymidine, 5-(2-carbonomethoxyvinyl)-uracil, 5-(carbamoylhydroxymethyl)-uracil, 5-carbamoylmethyl-2-thiouracil, 5-carboxymethyl-2-thiouracil, 5-cyanomethyluracil, 5-methoxy-2-thiouracil, and 5-3-(1-E-propenylamino)uracil.

In some embodiments, the non-standard nucleobases are modified cytosine. Exemplary nucleobases and nucleosides with the modified cytosine include 5-azacytosine, 6-azacytosine, pseudoisocytidine, 3-methylcytosine (m3C), N4-acetylcytosine (ac4C), 5-formylcytosine (f5C), N4-methyl-cytosine (m4C), 5-methyl-cytosine (m5C), 5-halo-cytosine (e.g., 5-iodo-cytosine), 5-hydroxymethyl-cytosine (hm5C), 1-methyl-pseudoisocytidine, pyrrolocytosine, pyrrolopseudoisocytidine, 2-thiocytidine (s2C), 2-thio-5-methylcytidine, 4-thiopseudoisocytidine, 4-thio-1-methyl-pseudoisocytidine, 4-thio-1-methyl-1-deaza-pseudoisocytidine, 1-methyl-1-deaza-pseudoisocytidine, zebularine, 5-aza-zebularine, 5-methyl-zebularine, 5-aza-2-thio-zebularine, 2-thio-zebularine, 2-methoxy-cytosine, 2-methoxy-5-methylcytosine, 4-methoxy-pseudoisocytidine, 4-methoxy-1-methyl-pseudoisocytidine, lysine (k2C), 5,2'-O-dimethylcytidine (m5Cm), N4-acetyl-2'-O-methylcytidine (ac4Cm), N4,2'-O-dimethylcytidine (m4Cm), 5-formyl-2'-O-methylcytidine (fSCm), N4,N4,2'-O-trimethylcytidine (m42Cm), 1-thiocytosine, 5-hydroxy-cytosine, 5-(3-azidopropyl)-cytosine, and 5-(2-azidoethyl)-cytosine.

In some embodiments, the non-standard nucleobases are modified adenine. Exemplary nucleobases and nucleosides with alternative adenine include 2-aminopurine, 2,6-diaminopurine, 2-amino-6-halopurine (e.g., 2-amino-6-chloropurine), 6-halopurine (e.g., 6-chloropurine), 2-amino-6-methylpurine, 8-azidoadenine, 7-deazaadenine, 7-deaza-8-azaadenine, 7-deaza-2-aminopurine, 7-deaza-8-aza-2-aminopurine, 7-deaza-2,6-diaminopurine, 7-deaza-8-aza-2,6-diaminopurine, 1-methyladenine (m1A), 2-methyladenine (m2A), N6-methyladenine (m6A), 2-methylthio-N6-methyladenine (ms2m6A), N6-prenyladenine (i6A), 2-methylthio-N6-prenyladenine (ms2i6A), N6-(cis-hydroxyprenyl)adenine (io6A), 2-methylthio-N6-(cis-hydroxyprenyl)adenine (ms2io6A), N6-glycylcarbamoyl-adenine (g6A), N6-threoncarbamoyl-adenine (t6A), N6-methyl-N6-threoncarbamoyl-adenine (m6t6A), 2-methylthio-N6-threoncarbamoyl-adenine (ms2g6A), N6,N6-dimethyl-adenine (m62A), N6-hydroxy-n-pentylcarbamoyl-adenine (hn6A), 2-methylthio-N6-hydroxy-n-pentylcarbamoyl-adenine (ms2hn6A), N6-acetyl adenine (ac6A), 7-methyladenine, 2-methylthioadenine, 2-methoxyadenine, N6,2'-O-dimethyladenosine (m6Am), N6,N6,2'-O-trimethyladenosine (m62Am), 1,2'-O-dimethyladenosine (m1Am), 2-amino-N6-methylpurine, 1-thioadenine, 8-azidoadenine, N6-(19-amino-pentaoxadodecane)-adenine, 2,8-dimethyl-adenine, N6-formyl-adenine, and N6-hydroxymethyl-adenine.

In some embodiments, the non-standard nucleobases are modified guanine. Exemplary nucleobases and nucleosides with modified guanine include inosine (I), 1-methylinosine (m1I), inosine (imG), methylinosine (mimG), 4-demethylinosine (imG-14), isotyrosine (imG2), wybutosine (yW), peroxytyrosine (o2yW), hydroxytyrosine (OHyW), undermodified hydroxytyrosine (OHyW*), 7-deazaguanine, queuosine (Q), epoxy queuosine (oQ), galactosylqueuosine (galQ), mannosylqueuosine, 7-cyano-7-deazaguanine (preQO), 7-aminomethyl-7-deazaguanine (preQ1), archaeosine (G+), 7-deaza-8-azaguanine, 6-thioguanine, 6-thio-7-deaza-guanine, 6-thio-7-deaza-8-aza-guanine, 7-methyl-guanine (m7G), 6-thio-7-methylguanine, 7-methyl-inosine, 6-methoxy-guanine, 1-methylguanine (m1G), N2-methylguanine (m2G), N2,N2-dimethylguanine (m22G), N2,7-dimethylguanine (m2,7G), N2,N2,7-dimethylguanine (m2,2,7G), 8-oxoguanine, 7-methyl-8-oxoguanine, 1-methyl-6-thioguanine, N2-methyl-6-thioguanine, N2-methyl-6-thioguanine, N2, N2-dimethyl-6-thioguanine, N2-methyl-2'-O-methyl-guanine (m2Gm), N2,N2-dimethyl-2'-O-methylguanosine (m22Gm), 1-methyl-2'-O-methylguanosine (m1Gm), N2,7-dimethyl-2'-O-methylguanosine (m2,7Gm), 2'-O-methylinosine (Im), 1,2'-O-dimethylinosine (mIm), 1-thioguanine, and O-6-methylguanine.

In some embodiments, the non-standard nucleobases of functional nucleotide analogues may independently be purines, pyrimidines, or purine or pyrimidine analogues. For example, in some embodiments, the non-canonical nucleobases may be a modified adenine, cytosine, guanine, uracil, or hypoxanthine. In other embodiments, the non-canonical nucleobases may also include, for example, naturally occurring and synthetic derivatives of bases, including pyrazolo[3,4-d]pyrimidine, 5-methylcytosine (5-me-C), 5-hydroxymethylcytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-proynyluracil and cytosine, 6-azouracil, cytosine and thymine, 5-uracil (pseudouracil), 4-thiouracil, 8-halo (e.g., 8-bromo), 8-amino, 8-thiol, 8-thioalkyl, 8-hydroxy and other 8-substituted adenine and guanine, 5-halo, especially 5-bromo, 5-trifluoromethyl and other 5-substituted uracil and cytosine, 7-methylguanine and 7-methyladenine, 8-azaguanine and 8-azaadenine, deazaguanine, 7-deazaguanine, 3-deazaguanine, deazaadenosine, 7-deazaadenosine, 3-deazaadenosine, pyrazolo[3,4-d]pyrimidine, imidazo[1,5-a]1,3,5-triazinone, 9-deazapurine, imidazolo[4,5-d]pyrazine, thiazolo[4,5-d]pyrimidine, pyrazine-2-one, 1,2,4-triazine, pyridazine, or 1,3,5-triazine.

### Modification of sugar

In some embodiments, the functional nucleotide analog comprises non-standard sugar group. In various embodiments, the non-standard sugar group may be a 5-carbon or 6-carbon sugar (e.g., pentose, ribose, arabinose, xylose, glucose, galactose, or a deoxygenated derivative thereof) having one or more substituents, which may be halogen, hydroxyl, thiol, alkyl, alkoxy, alkenyloxy, alkynyloxy, cycloalkyl, aminoalkoxy, alkoxyalkoxy, hydroxyalkoxy, amino, azido group, aryl, aminoalkyl, aminoalkenyl, aminoalkynyl, etc.

Generally, RNA molecules include a ribose group that is a five-membered ring having oxygen. Exemplary non-limiting alternative nucleotides include oxygen replacements in ribose (e.g., substitution with S, Se, or an alkylene group, such as methylene or ethylene); addition of double bonds (e.g., substitution of ribose with cyclopentenyl or cyclohexenyl); ring-closing of ribose (e.g., four-membered ring forming cyclobutane or oxetane); ring extensions of ribose (e.g., formation of 6 or 7-membered ring having additional carbon atoms or heteroatoms, such as anhydrohexanol, arabitol, mannitol, cyclohexyl, cyclohexenyl, and morpholino (also having an phosphoramidate backbone)); polycyclic forms (e.g., tricyclic and "unlocked" forms, such as Ethylene Glycol Nucleic Acid (GNA) (e.g., R-GNA or S-GNA, wherein the ribose is substituted with an ethylene glycol unit attached to a phosphodiester bond), Threose Nucleic Acid (TNA, wherein ribose is substituted with α-L-threofuranosyl- (3'→2') and Peptide Nucleic Ccid (PNA, wherein 2-aminoethyl-glycine bonds substitute ribose and phosphodiester backbone).

In some embodiments, the sugar group comprises one or more carbons having a stereochemical configuration opposite to a corresponding carbon in ribose. Accordingly, the nucleic acid molecules may include a nucleotide containing, for example, arabinose or L-ribose as a sugar. In some embodiments, the nucleic acid molecules include at least one nucleoside, wherein the sugar is L-ribose, 2'-O-methylribose, 2'-fluororibose, arabinose, hexitol, LNA or PNA.

### Modification of nucleoside bonds

In some embodiments, the payload nucleic acid molecules of the present disclosure may comprise one or more modified nucleoside bonds (e.g., a phosphate backbone). The phosphate group of the backbone may be altered by substituting one or more oxygen atoms with different substituents.

In some embodiments, the functional nucleotide analogs may comprise another nucleotide bond substituted for unaltered phosphoric acid moiety. Examples of alternative phosphate groups include, but are not limited to, phosphorothioates, phosphoroselenite, phosphoroborates, phosphoroborates, hydrogen phosphonates, phosphoramidates, phosphorodiamidate, alkyl or aryl phosphonates, and phosphotriesters. Both the non-attached oxygens of the phosphorodithioate are substituted with sulfur. The modified phosphate bonds may also be attached by substituting oxygen with nitrogen (bridged phosphoramidate), sulfur (bridged phosphorothioate), and carbon (bridged methylene phosphonate).

The alternative nucleosides and nucleotides include borane moiety (BH₃), thio, methyl, ethyl, and/or methoxy groups in place of one or more non-bridged oxygens. As a non-limiting example, two non-bridged oxygens at the same position (e.g., α, β, or γ position) may be substituted by thio and methoxy. By substitution of one or more oxygen atoms at the position of the phosphate moiety (e.g., α-thiophosphate), the stability of RNA and DNA (e.g., against exonuclease and endonuclease) is enhanced with non-natural thiophosphate backbone linkages. Thiophosphate DNA and RNA have enhanced nuclease resistance and thus have longer half-lives in the cellular environment.

Other nucleoside bonds used in accordance with the present disclosure include nucleoside bonds that do not contain phosphorus atoms.

Other examples of nucleic acid molecules (e.g., mRNA), compositions, preparations and/or methods related thereto that may be used in combination with the present disclosure are further included in WO2002/098443, WO2003/051401, WO2008/052770, WO2009127230, WO2006122828, WO2008/083949, WO2010088927, WO2010/037539, WO2004/004743, WO2005/016376, WO2006/024518, WO2007/095976, WO2008/014979, WO2008/077592, WO2009/030481, WO2009/095226, WO2011069586, WO2011026641, WO2011/144358, WO2012019780, WO2012013326, WO2012089338, WO2012113513, WO2012116811, WO2012116810, WO2013113502, WO2013113501, WO2013113736, WO2013143698, WO2013143699, WO2013143700, WO2013/120626, WO2013120627, WO2013120628, WO2013120629, WO2013174409/WO2015127917, WO2015 024667, WO2015/024665, WO2015/024666, WO2015/024664, WO2015101415, WO2015101414, WO2015024667, WO2015062738, and WO2015101416, each of which is incorporated herein in their entirety.

### Dosage form

According to the present disclosure, the nanoparticle composition described herein may comprise at least one lipid component and one or more other components, such as therapeutic and/or prophylactic agents. The nanoparticle composition may be designed for one or more particular applications or targets. The elements of the nanoparticle compositions may be selected based on particular applications or targets and/or based on the efficacy, toxicity, cost, ease of use, availability, or other characteristics of one or more elements. Similarly, particular formulations of the nanoparticle compositions may be selected for particular applications or targets based on the efficacy and toxicity of particular combinations of elements.

The lipid components of the nanoparticle composition may include lipids of the Formula (I) (and sub-formulas thereof) as described herein, phospholipids (e.g., unsaturated lipids such as DOPE or DSPC), PEG lipids, and structural lipids. The elements of the lipid components may be provided in particular proportions.

In one embodiment, the nanoparticle composition is provided herein which comprises the cationic or ionizable lipid compound provided herein, the therapeutic agent, and one or more excipients. In one embodiment, the cationic or ionizable lipid compound comprises the compound of the Formula (I) (and formulas thereof) as described herein, and optionally one or more other ionizable lipid compounds. In one embodiment, the one or more excipients are selected from the group consisting of neutral lipids, steroids, and polymer-conjugated lipids. In one embodiment, the therapeutic agent is encapsulated within or associated with the lipid nanoparticles.

In one embodiment, the present invention provides a nanoparticle composition (lipid nanoparticles), comprising:
i) 40 to 50 mole percent of cationic lipids;
ii) neutral lipids;
iii) steroids;
iv) polymer-conjugate lipids; and
v) therapeutic agents.

As described herein, the "mole percent" refers to the mole percent of the component relative to the total mole number of all lipid components in LNP (i.e., the total mole number of cationic lipids, neutral lipids, steroids, and polymer conjugate lipids).

In one embodiment, the lipid nanoparticles account for 41 to 49 mole percent, 41 to 48 mole percent, 42 to 48 mole percent, 43 to 48 mole percent, 44 to 48 mole percent, and 45 to 48 mole percent, and the content of cationic lipids is 46 to 48 mole percent, or 47.2 to 47.8 mole percent. In one embodiment, the lipid nanoparticles account for about 47.0, 47.1, 47.2, 47.3, 47.4, 47.5, 47.6, 47.7, 47.8, 47.9, or 48.0 mole percent of cationic lipids.

In one embodiment, the neutral lipids are present at a concentration of 5 to 15 mole percent, 7 to 13 mole percent, or 9 to 11 mole percent. In one embodiment, the neutral lipids are present at a concentration of about 9.5, 10, or 10.5 mole percent. In one embodiment, the molar ratio of cationic lipids to neutral lipids is about 4.1:1.0 to about 4.9:1.0, about 4.5:1.0 to about 4.8:1.0, or about 4.7:1.0 to 4.8:1.0.

In one embodiment, the steroids are present at a concentration range of 39 to 49 mole percent, 40 to 46 mole percent, 40 to 44 mole percent, 40 to 42 mole percent, 42 to 44 mole percent, or 44 to 46 mole percent. In one embodiment, the steroids are present at a concentration of 40, 41, 42, 43, 44, 45, or 46 mole percent. In one embodiment, the molar ratio of cationic lipids to steroids is 1.0:0.9 to 1.0:1.2, or 1.0:1.0 to 1.0:1.2. In one embodiment, the steroids are cholesterol.

In one embodiment, the ratio of therapeutic agent to lipid in LNP (i.e., N/P, N representing the mole of cationic lipids and P representing the mole of phosphate present as part of the nucleic acid backbone) is 2:1 to 2. 30:1, e.g., 3:1 to 22:1. In one embodiment, N/P is 6:1 to 20:1 or 2:1 to 12:1. Exemplary N/P ranges include about 3:1, about 6:1, about 12:1, and about 22:1.

In one embodiment, the present invention provides lipid nanoparticles, comprising:
i) cationic lipids with an effective pKa greater than 6.0;
ii) neutral lipids of 5 to 15 mole percent;
iv) 30 to 45 mole percent of steroids;
v) polymer-conjugate lipids; and
vi) therapeutic agents or pharmaceutically acceptable salts or prodrugs thereof,
wherein, the mole percent is determined based on the total moles of lipids present in the lipid nanoparticles.

In one embodiment, the cationic lipids may be any of multiple lipids that carry a net positive charge at a selected pH (e.g., physiological pH). Exemplary cationic lipids are described below. In one embodiment, the cationic lipid has a pKa greater than 6.25. In one embodiment, the cationic lipid has a pKa greater than 6.5. In one embodiment, the cationic lipid has a pKa greater than 6.1, greater than 6.2, greater than 6.3, greater than 6.35, greater than 6.4, greater than 6.45, greater than 6.55, greater than 6.6, greater than 6.65, or greater than 6.7.

In one embodiment, the lipid nanoparticles account for 40 to 45 mole percent of cationic lipids. In one embodiment, the lipid nanoparticles account for 45 to 50 mole percent of cationic lipids.

In one embodiment, the molar ratio of cationic lipids to neutral lipids is from about 2:1 to about 8:1. In one embodiment, the neutral lipids account for 5 to 10 mole percent of lipids in the lipid nanoparticles.

Exemplary anionic lipids include, but are not limited to, phosphatidylglycerol, dioleoyl phosphatidylglycerol (DOPG), dipalmitoyl phosphatidylglycerol (DPPG), or 1,2-distearoyl-sn-glycero-3-phosphate-(1'-rac-glycerol) (DSPG).

In one embodiment, the lipid nanoparticles comprise 1 to 10 mol% of the anionic lipids. In one embodiment, the lipid nanoparticles comprise 1 to 5 mol% of the anionic lipids. In one embodiment, the anionic lipids are contained in the lipid nanoparticles in amounts of 1 to 9 mol%, 1 to 8 mol%, 1 to 7 mol%, or 1 to 6 mol%. In one embodiment, the molar ratio of the anionic lipids to neutral lipids is 1:1 to 1:10.

In one embodiment, steroid cholesterol. In one embodiment, the molar ratio of cationic lipid to cholesterol is about 5:1 to 1:1. In one embodiment, the lipid nanoparticle comprises 32-40 mol% of steroid.

In one embodiment, the sum of the mole percent of the neutral lipid and the mole percent of anionic lipid is 5-15 mole percent. In one embodiment, the sum of the mole percent of the neutral lipid and the mole percent of anionic lipid is 7-12 mole percent.

In one embodiment, the molar ratio of the anionic lipids to neutral lipids is 1:1 to 1:10. In one embodiment, the sum of the mole percent of the neutral lipid and the mole percent of the steroid is 35-45 mole percent.

In one embodiment, the lipid nanoparticles include:
i) 45-55 mole percent of cationic lipids;
ii) 5-10 mole percent of neutral lipids;
iii) 1-5 mole percent of anionic lipids; and
iv) 32-40 mole percent of steroids.

In one embodiment, the lipid nanoparticles include 1.0-2.5 mole percent of polymer-conjugated lipid. In one embodiment, the polymer-conjugated lipid is present at a concentration of about 1.5 mole percent.

In one embodiment, the neutral lipids are present at a concentration of 5 to 15 mole percent, 7 to 13 mole percent, or 9 to 11 mole percent. In one embodiment, the neutral lipids are present at a concentration of about 9.5, 10, or 10.5 mole percent. In one embodiment, the molar ratio of cationic lipids to neutral lipids is about 4.1:1.0 to about 4.9:1.0, about 4.5:1.0 to about 4.8:1.0, or about 4.7:1.0 to 4.8:1.0.

In one embodiment, the steroids are cholesterol. In one embodiment, the steroid is present at a concentration ranging from 39-49 mole percent, 40-46 mole percent, 40-44 mole percent, 40-42 mole percent, 42-44 mole percent, or 44-46 mole percent. In one embodiment, the steroids are present at a concentration of 40, 41, 42, 43, 44, 45, or 46 mole percent. In some embodiments, the molar ratio of cationic lipid to steroid is 1.0:0.9 to 1.0:1.2, or 1.0:1.0 to 1.0:1.2.

In one embodiment, the molar ratio of the cationic lipid to steroid is 5:1 to 1:1.

In one embodiment, the lipid nanoparticles comprise 1.0 to 2.5 mole percent of polymer-conjugated lipid. In one embodiment, the polymer-conjugated lipid is present at a concentration of about 1.5 mole percent.

In one embodiment, the molar ratio of cationic lipid to polymer-conjugated lipid is from about 100:1 to about 20:1. In one embodiment, the molar ratio of cationic lipid to polymer-conjugated lipid is from about 35:1 to about 25:1.

In one embodiment, the lipid nanoparticles have an average diameter of 50nm to 100nm, or 60nm to 85nm.

In one embodiment, the composition comprises the cationic lipid, DSPC, cholesterol and PEG-lipids, and mRNA provided herein. In one embodiment, the cationic lipid, DSPC, cholesterol and PEG-lipids are provided herein in a molar ratio of about 50:10:38.5:1.5.

The nanoparticle composition may be designed for one or more particular applications or targets. For example, nanoparticle compositions may be designed to deliver therapeutic and/or prophylactic agents (e.g., RNA) to specific cells, tissues, organs, or systems thereof, etc. within a mammal. The physicochemical properties of the nanoparticle compositions may be altered to increase selectivity to specific body targets. For example, particle diameter may be adjusted based on the fenestration size of different organs. Therapeutic and/or prophylactic agents comprised in the nanoparticle composition may also be selected based on one or more delivery targets as desired. For example, therapeutic and/or prophylactic agents may be selected for specific indications, conditions, diseases, or disorders and/or delivery to specific cells, tissues, organs, or systems, etc. (e.g., local or specific delivery). In certain embodiments, the nanoparticle composition may comprise mRNA encoding polypeptide of interest capable of being produced intracellularly by translation. Such composition may be specifically designed for delivery to specific organs. In certain embodiments, the composition may be designed for specific delivery to the liver of mammal.

The amount of therapeutic and/or prophylactic agents in the nanoparticle composition may depend on the size, composition, desired target and/or other properties of the nanoparticle composition and the nature of the therapeutic and/or prophylactic agents. For example, the amount of RNA available in the nanoparticle composition can depend on the size, sequence, and other characteristics of the RNA. The relative amounts of therapeutic and/or prophylactic agents and other elements (e.g., lipids) in the nanoparticle composition may also be adjusted. In some embodiments, the wt/wt ratio of the lipid component to therapeutic and/or prophylactic agents in the nanoparticle composition may be about 5:1 to about 60:1, e.g., 5:1, 6:1, 7:1, 8:1, 9:1, 10:1, 11:1, 12:1, 13:1, 14:1, 15:1, 16:1, 17:1, 18:1, 19:1, 20:1, 25:1, 30:1, 35:1, 40:1, 45:1, 50:1, and 60:1. The wt/wt ratio of lipid fractions to therapeutic and/or prophylactic agents may range from about 10:1 to about 40:1. In certain embodiments, the weight/weight ratio is about 20:1. The amount of therapeutic and/or prophylactic agents in the nanoparticle composition may be measured by absorption spectrometry (e.g., ultraviolet-visible spectroscopy).

In some embodiments, the nanoparticle composition comprises one or more RNAs, and the one or more RNAs, lipids, and amounts thereof may be selected to provide a particular N:P ratio. The N:P ratio of the composition refers to the molar ratio of nitrogen atoms in the one or more lipids to the number of phosphate groups in the RNA. In some embodiments, a lower N:P ratio is selected. The one or more RNAs, lipids, and amounts thereof may be selected such that the N:P ratio is from about 2:1 to about 30:1, e.g., 2:1, 3:1, 4:1, 5:1, 6:1, 7:1, 8:1, 9:1, 10:1, 12:1, 14:1, 16:1, 18:1,20:1, 22:1, 24:1, 26:1, 28:1, or 30:1. In certain embodiments, the N:P ratio may be from about 2:1 to about 8:1. In other embodiments, the N:P ratio may be from about 5:1 to about 8:1. For example, the N:P ratio may be from about 5.0:1, about 5.5:1, about 5.67:1, about 6.0:1, about 6.5:1, or about 7.0:1. For example, the N:P ratio may be from about 5.67:1.

The physical properties of the nanoparticle composition may depend on its components. For example, the nanoparticle composition comprising cholesterol as structural lipid may have different properties as compared to the nanoparticle composition including a different structural lipids. Similarly, the properties of the nanoparticle composition may depend on the absolute or relative amounts of its components. For example, the nanoparticle composition comprising higher mole fraction of phospholipid may have different properties as compared to the nanoparticle composition comprising lower mole fraction of phospholipid. The properties may also vary depending on the method and conditions of preparing the nanoparticle composition.

The nanoparticle composition may be characterized by a variety of methods. For example, microscopes (such as transmission electron microscope or scanning electron microscope) may be used for examining the morphology and size distribution of nanoparticle compositions. Dynamic light scattering or potentiometric methods (such as potentiometric titration) may be used for measuring ζ potential. Dynamic light scattering may also be used for determining particle size. Zetasizer Nano ZS (Malvern Instruments Ltd, Malvern, Worcestershire, UK) may also be used for measuring multiple characteristics of the nanoparticle composition, such as particle size, polydispersity index, and zeta potential.

In various embodiments, the average size of the nanoparticle composition may be between 10snm and 100snm. For example, the average size may be about 40 nm to about 150 nm, for example, about 40 nm, 45 nm, 50 nm, 55 nm, 60 nm, 65 nm, 70 nm, 75 nm, 80 nm, 85 nm, 90 nm, 95 nm, 100 nm, 105 nm, 110 nm, 115 nm, 120 nm, 125 nm, 130 nm, 135 nm, 140 nm, 145 nm, or 150 nm. In some embodiments, the average size of the nanoparticle compositions may be between about 50 nm and about 100 nm, between about 50 nm and about 90 nm, between about 50 nm and about 80 nm, between about 50 nm and about 70 nm, between about 50 nm and about 60 nm, between about 60 nm and about 100 nm, between about 60 nm and about 90 nm, between about 60 nm and about 80 nm, between about 60 nm and about 70 nm, between about 70 nm to about 100 nm, between about 70 nm and about 90 nm, between about 70 nm and about 80 nm, between about 80 nm and about 100 nm, between about 80 nm and about 90 nm, or between about 90 nm and about 100 nm. In certain embodiments, the average size of the nanoparticle compositions may be between about 70nm and about 100nm. In some embodiments, the average size may be about 80nm. In other embodiments, the average size may be about 100nm.

The nanoparticle composition may be relatively uniform. A polydispersity index may be used for indicating the uniformity of the nanoparticle composition, e.g., the particle size distribution of the nanoparticle composition. A small (e.g., less than 0.3) polydispersity index generally indicates a narrow particle size distribution. The nanoparticle composition may have a polydispersity index of from about 0 to about 0.25, e.g., 0.01, 0.02, 0.03, 0.04, 0.05, 0.06, 0.07, 0.08, 0.09, 0.10, 0.11, 0.12, 0.13, 0.14, 0.15, 0.16, 0.17, 0.18, 0.19, 0.20, 0.21, 0.22, 0.23, 0.24, or 0.25. In some embodiments, the polydispersity index of the nanoparticle composition may be about 0.10 to about 0.20.

The ζ potential of the nanoparticle composition can be used for indicating the electrodynamic potential of the composition. For example, ζ potential may characterize the surface charge of nanoparticle compositions. Compositions of nanoparticles with a relatively low positive or negative charge are generally expected, since substances with a higher charge can interact adversely with cells, tissues and other elements of the human body. In some embodiments, the ζ potential of the nanoparticle composition may be about -10 mV to about +20 mV, about -10 mV to about +15 mV, about -10 mV to about +10 mV, about -10 mV to about +5 mV, about -10 mV to about 0 mV, about -10 mV to about -5 mV, about -5 mV to about +20 mV, about -5 mV to about +15 mV, about -5 mV to about +10 mV, about -5 mV to about +5 mV, about -5 mV to about 0 mV, about 0 mV to about +20 mV, about 0 mV to about +15 mV, about 0 mV to about +10 mV, about 0 mV to about +5 mV, about +5 mV to about +20 mV, about +5 mV to about +15 mV, or about +5 mV to about +10 mV.

The encapsulation efficiency of therapeutic and/or prophylactic agents describes the amount of therapeutic and/or prophylactic agents that are encapsulated or associated with nanoparticle compositions after preparation relative to the initial amount supplied. High encapsulation efficiency is expected (e.g., close to 100%). Encapsulation efficiency may be measured, for example, by comparing the amount of therapeutic and/or prophylactic agents before decomposition of the nanoparticle composition with one or more organic solvents or detergents and after treatment in a solution comprising the nanoparticle composition. Fluorescence may be used for measuring the amount of free therapeutic and/or prophylactic agents (e.g., RNA) in solution. For the nanoparticle compositions described herein, the encapsulation efficiency of therapeutics and/or prophylactic agents may be at least 50%, e.g., 50%, 55%, 60%, 65%, 70%, 75%, 80%, 85%, 90%, 91%, 92%, 93%, 94%, 95%, 96%, 97%, 98%, 99% or 100%. In some embodiments, encapsulation efficiency may be at least 80%. In some embodiments, encapsulation efficiency may be at least 90%.

The nanoparticle composition may optionally include one or more coatings. For example, the nanoparticle composition may be formulated into capsules, films, or tablets with coatings. The composition described herein in the form of capsules, films or tablets may be of any useful size, tensile strength, hardness or density.

### Pharmaceutical composition

According to the present invention, the nanoparticle composition may be formulated into a pharmaceutical composition in part or in whole. The pharmaceutical composition may include one or more nanoparticle compositions. For example, the pharmaceutical composition may include one or more nanoparticle compositions, and one or more different therapeutic and/or prophylactic agents. The pharmaceutical composition may further include one or more pharmacologically acceptable excipients or auxiliary ingredients, such as those described herein. General guidelines for the formulation and manufacture of pharmaceutical compositions and preparations are relatively described, for example, in Remington's The Science and Practice of Pharmacy, 21st Edition, A. R. Gennaro; Lippincott, Williams & Wilkins, Baltimore, Md., 2006. Conventional excipients and auxiliary ingredients may be used in any pharmaceutical composition, unless they are incompatible with one or more components of the nanoparticle composition. If the excipients or auxiliary ingredients are incompatible with the components of the nanoparticle composition, the combination may lead to undesirable biological effects or harmful effects.

In some embodiments, one or more excipients or auxiliary ingredients may account for greater than 50% of the total mass or volume of pharmaceutical composition including the nanoparticle composition. For example, usually one or more excipients or auxiliary ingredients can account for 50%, 60%, 70%, 80%, 90% or more of medicine. In some embodiments, the pharmaceutically acceptable excipients are at least 95%, at least 96%, at least 97%, at least 98%, at least 99% or 100% pure. In some embodiments, the excipients are approved for human and veterinary use. In some embodiments, the excipients are approved by U.S. Food and Drug Administration. In some embodiments, the excipients are pharmaceutical grade. In some embodiments, the excipients meet the standards of United States Pharmacopoeia (USP), European Pharmacopoeia (EP), British Pharmacopoeia and/or International Pharmacopoeia.

Variations in the relative amounts of one or more nanoparticle compositions, one or more pharmaceutically acceptable excipients and/or any other ingredients in the pharmaceutical composition according to the present disclosure is adjusted, depending on the characteristics, size and other related conditions thereof, and further depending on the subject of administration route of administration of the composition. For example, the pharmaceutical composition may comprise 0.1% to 100% (wt/wt) of one or more nanoparticle compositions.

In certain embodiments, the nanoparticle composition and/or pharmaceutical composition of the present disclosure are refrigerated or frozen for storage and transportation. For example, they are stored between about -150°C and 0°C at a temperature of 4°C or lower or at a temperature of about -80°C to about -20°C, such as about -5°C, -10°C, -15°C, -20°C, -25°C, -30°C, -40°C, -50°C, -60°C, -70°C, -80°C, -90°C, -130°C or -150°C). The pharmaceutical composition comprising the compound of Formula (I) and the sub-formula in solution form are refrigerated for storage or transport at about -20°C, -30°C, -40°C, -50°C, -60°C, -70°C or -80°C. In some embodiments, the present disclosure also relates to a method for improving stability of a nanoparticle composition and/or a pharmaceutical composition of a compound comprising Formula (I) (and sub-formulas thereof). By storing the nanoparticle composition and/or pharmaceutical composition at a temperature of 4°C or lower, such as between about -150°C and about 0°C or between about -80°C and about -20°C, such as about -5°C, -10°C, -15°C, -20°C, -25°C, -30°C, -40°C, -50°C, -60°C, - 70°C, -80°C, -90°C, -130°C or -150°C. The nanoparticle composition and/or pharmaceutical composition disclosed herein is stable at temperatures of 4°C or lower (such as between about 4°C and -20°C) for at least 1 week, at least 2 weeks, at least 3 weeks, at least 4 weeks, at least 5 weeks, at least 6 weeks, at least 1 month, at least 2 months, at least 4 months, at least 6 months, at least 8 months, at least 10 months, at least 12 months, at least 14 months, at least 16 months, at least 18 months, at least 20 months, at least 22 months, or at least 24 months. In one embodiment, the formulation is stable at approximately 4°C for at least 4 weeks. In certain embodiments, the disclosed pharmaceutical composition consists of the nanoparticle composition disclosed herein and one or more of the pharmaceutically acceptable carriers selected from Tris, acetate (e.g., acetic acid), citrate (e.g., sodium citrate), saline, PBS, and sucrose. In certain embodiments, the pH of the pharmaceutical composition of the disclosure is between about 7 and 8 (e.g., 6.8, 6.9, 7.0, 7.1, 7.2, 7.3, 7.4, 7.5, 7.6, 7.7, 7.8, 7.9 or 8.0, or between 7.5 and 8 or 7 and 7.8). The pharmaceutical composition of the disclosure comprises the nanoparticle composition, Tris, saline, and sucrose disclosed herein, and has a pH of about 7.5-8, which is suitable for storage or transportation at approximately -20°C. For example, the pharmaceutical composition of the disclosure comprises the nanoparticle composition and PBS disclosed herein and has a pH of about 7-7.8, which is suitable for storage or transportation at temperatures such as about 4°C or lower. In the context of the present disclosure, "stable" and "stability" refer to the resistance of the nanoparticle composition or pharmaceutical composition herein to chemical or physical changes (e.g., degradation, particle size change, and aggregation change) under given conditions of manufacture, preparation, transportation, storage and/or use (e.g., applied stress (shear, freeze/thaw stress)).

The nanoparticle composition and/or pharmaceutical composition comprising one or more nanoparticle compositions may be administered to any patient or subject, including providing beneficial therapeutic effects by delivering therapeutic and/or prophylactic agents to a patient or subject specific cells, tissues, organs, or systems thereof, such as the renal system. While the description of nanoparticle composition and pharmaceutical composition including nanoparticle composition herein is primarily directed towards composition suitable for administration to human, those skilled in the art should understand that such compositions are generally suitable for administration to any other mammal. Modifications to compositions suitable for administration to humans to make the compositions suitable for administration to various animals are well known, and the veterinarians and pharmacologists of ordinary skill may design and/or make such modifications through only general experiments. Subjects intended for administration of the compositions include, but are not limited to, humans, other primates and other mammals, including commercially relevant mammals, such as cattle, pigs, horses, sheep, cats, dogs, mice and rats.

The pharmaceutical composition including one or more nanoparticle compositions may be prepared by any method known in the pharmacological art or developed later. Such methods of preparation generally include combining an active ingredient with an excipient and/or one or more other auxiliary ingredients, and if necessary, separately shaping and/or packaging the product into a single or mixed form of desired multiple dose units.

The pharmaceutical composition according to the present disclosure may be prepared, packaged and/or sold as a single unit dose and/or as multiple single unit doses in bulk. The "unit dose" refers to a discrete amount of the pharmaceutical composition comprising a predetermined amount of active ingredient (e.g., the nanoparticle composition). The amount of the active ingredient is generally equal to a dose of the active ingredient to be administered to the subject and/or a convenient fraction of the dose, e.g., half or one third of the dose.

The pharmaceutical composition may be prepared into various forms suitable for various routes and administration methods. For example, the pharmaceutical composition may be prepared into liquid dosage forms (such as emulsion, microemulsion, nano-emulsion, solution, suspension, syrup and elixir), injectable dosage forms, solid dosage forms (such as capsules, tablets, pills, powder and granules), dosage forms for local and/or transdermal administration (such as ointment, paste, cream, lotion, gel, powder, solution, spray, inhalant and patch), suspension, powder and other forms.

The liquid dosage forms for oral and parenteral administration include, but are not limited to, pharmaceutically acceptable emulsions, microemulsions, nanoemulsions, solutions, suspensions, syrups, and/or elixirs. In addition to the active ingredient, liquid dosage forms may also comprise inert diluents commonly used in the art, such as water or other solvents, solubilizers and emulsifiers, like ethanol, isopropanol, ethyl carbonate, ethyl acetate, benzyl alcohol, benzyl benzoate, propylene glycol, 1,3-butanediol, dimethylformamide, oils (especially cottonseed, peanuts, corn, germ, olive oil, castor and sesame oils), glycerol, tetrahydrofurfuryl alcohol, polyethylene glycol and fatty acid esters of sorbitol and their mixtures. In addition to inert diluents, oral compositions may comprise other therapeutic and/or prophylactic agents, such as other preparations such as wetting agents, emulsifying and suspending agents, sweeteners, flavoring agents and/or flavoring agents. In certain embodiments for parenteral administration, the compositions are mixed with solubilizers such as CremophorTM, alcohols, oils, modified oils, glycols, polysorbates, cyclodextrin polymers and/or combinations thereof.

The injectable formulations, such as aqueous or oily suspensions that can be aseptically injected, may be formulated using suitable dispersants, wetting agents, and/or suspensions depending on the known technology. The sterile injectable preparations may be sterile solutions for injection, suspensions, and/or emulsions in non-toxic, parenterally acceptable diluents and/or solvents, such as those in 1,3-butanediol. Acceptable vehicles and solvents that can be used include water, American Ringer's solution, and isotonic sodium chloride solution. Sterile, non-volatile oils are often used as solvents or suspension media. Therefore, any mild, non-volatile oil may be used, including synthetic mono- or diglycerides. Fatty acids such as oleic acid can be used in the preparation of injections.

The injectable formulation can be filtered by a bacterial retention filter and/or sterilized by incorporation of a sterilizing agent in the form of a sterile solid composition, that is dissolved or dispersed in sterile water or other sterile injectable medium prior to use.

The present invention discloses methods for delivering therapeutic agents and/or prophylactic agents to mammalian cells or organs, producing target polypeptides in mammalian cells, and treating diseases or disorders of mammals by administration to and/or by contacting the mammalian cells with the nanoparticle compositions comprising therapeutic and/or prophylactic agents.

### Examples

The Examples in this section are only provided as examples and are not limiting.

### General methods

Conventional preparative HPLC method: HPLC purification is performed on a Waters 2767 equipped with a diode array detector (DAD) on an Inertsil Pre-C8 OBD column, typically using water containing 0.1% TFA as a solvent A and acetonitrile as a solvent B.

Universal LCMS method: LCMS analysis is performed on the Shimadzu (LC-MS2020) system. Chromatography is performed on SunFire C18 and typically uses water containing 0.1% formic acid as the solvent A and acetonitrile containing 0.1% formic acid as the solvent B.

### Example 1: Synthesis of compound 1.

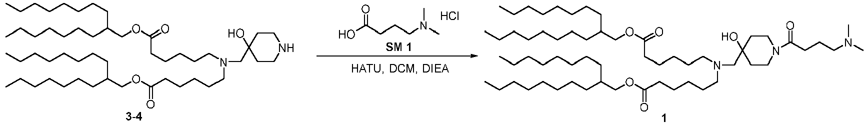

### Synthesis of compound 1

A compound 3-4 (80 mg, 0.09 mmol, 1.0 eq.), a compound SM1 (16 mg, 0.11 mmol, 1.2 eq.), HATU (42 mg, 0.11 mmol, 1.2 eq.) and DIEA (35 mg, 0.27 mmol, 3.0 eq.) were dissolved in methylene chloride (3 mL) and stirred for 1 hour at room temperature. The mixture was concentrated and purified by high-performance liquid chromatography to obtain the colorless oily compound 1 (32 mg, yield: 34.2%). (MC20-1207-108)

**¹H NMR (400 MHz, CDCl3) δ:**0.81-0.94 (m, 12H), 1.16-1.35 (m, 54H), 1.36-1.49 (m, 5H), 1.50-1.69 (m, 20H), 1.76-1.86 (m, 2H), 2.17-2.42 (m, 14H), 2.45-2.53 (m, 3H), 2.92-3.03 (m, 1H), 3.34-3.46 (m, 1H), 3.62-3.74 (m, 1H), 3.97 (d, *J* = 5.6 Hz, 3H), 4.36-4.48 (m, 1H).

**LCMS:** Rt: 1.270 min; MS m/z (ESI): 976.9 [M+H]⁺.

### Example 2: Synthesis of compound 2.

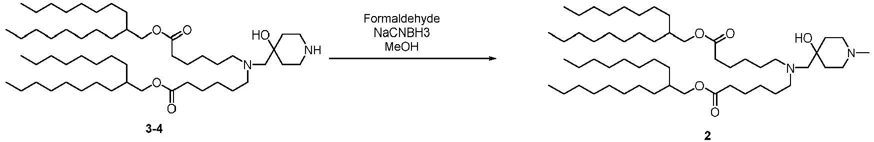

### Synthesis of compound 2

A compound 3-4 (150 mg, 0.17 mmol, 1.0 eq.) was dissolved in methanol (4 mL), 37% formaldehyde in water (0.1 mL) and acetic acid (1 drop) were added and stirred for 2 hours at room temperature. Then, NaCNBH₃ (21 mg, 0.34 mmol, 2.0 eq.) was added; and the resulting mixture was stirred at room temperature for 16 hours. LCMS showed that the reaction was completed. The reaction mixture was concentrated and purified by high-performance liquid chromatography to obtain the colorless oily compound 2 (41 mg, yield: 27%).

**¹H NMR (400 MHz, CDCl3) δ:**0.86-0.90 (m, 12H), 1.26-1.32 (m, 61H), 1.41-1.50 (m, 4H), 1.52-1.70 (m, 10H), 2.30 (t, *J* = 7.4Hz, 4H), 2.37-2.47 (m, 4H), 2.49-2.52(m, 6H), 2.69-2.77 (m, 2H), 3.43-3.55 (m, 1H), 3.96-3.97 (m, 4H).

**LCMS:** Rt: 2.360 min; MS m/z (ESI): 877.8 [M+H] ⁺.

### Example 3: Synthesis of compound 3.

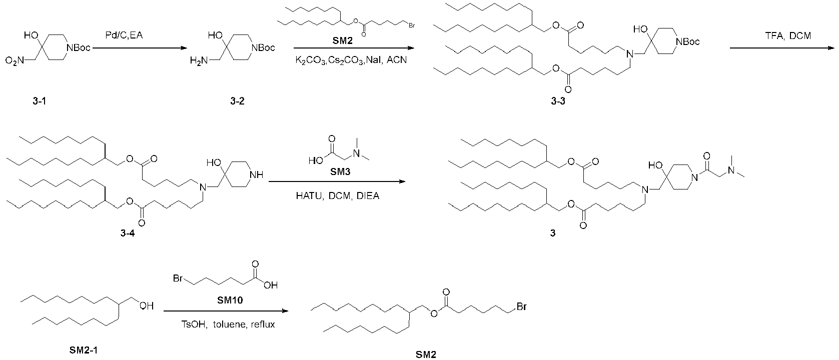

### Step 1: Synthesis of compound 3-2

A compound 3-2 (1 g, 3.85 mmol, 1.0 eq.) and Pd/C were added to ethyl acetate (40 mL) under hydrogen protection, and stirred overnight at 40°C. The reaction solution was filtered with diatomaceous earth, and the filtrate was concentrated to obtain 3-2 as the colorless oily compound (789 mg, yield: 89.2%). (MC20-1207-092)

**LCMS:** Rt: 0.380 min; MS m/z (ESI): 231.2 [M+H] ⁺.

### Step 2: Synthesis of compound 3-3

A compound 3-2 (82 mg, 0.36 mmol, 1.0 eq.), a compound SM2 (400 mg, 0.89 mmol, 2.5 eq.), K₂CO₃ (149 mg, 1.08 mmol, 3.0 eq.), and Cs₂CO₃ (3 mg, 0.01 mmol, 0.03 eq.) were added to acetonitrile (10 mL), and stirred overnight at 100°C. After being concentrated, the reaction solution was purified by silica gel column chromatography (MeOH: DCM = 0%-3%) to obtain the yellow oily compound 3-3 (162 mg, yield: 47.2%). (MC20-1207-094)

**LCMS:** Rt: 2.200 min; MS m/z (ESI): 963.9 [M+H] ⁺.

### Step 3: Synthesis of compound 3-4

A compound 3-4 (162 mg, 0.17 mmol, 1.0 eq.) was dissolved in dichloromethane (3 mL), and TFA (1 mL, 13.5 mmol, 79.0 eq.) was added, and stirred for 3 hours at room temperature. The reaction solution was concentrated to obtain 177 mg of yellow oily compound 3-4 as crude product. (MC20-1207-095)

**LCMS:** Rt: 1.950 min; MS m/z (ESI): 863.8 [M+H] ⁺.

### Step 4: Synthesis of compound 3

A compound 3-4 (84 mg, 0.10 mmol, 1.0 eq.), a compound SM3 (15 mg, 0.15 mmol, 1.5 eq.), HATU (46 mg, 0.12 mmol, 1.2 eq.), and DIEA (65 mg, 0.5 mmol, 5.0 eq.) were added to methylene chloride (3 mL), and stirred for 1 hour at room temperature. The reaction solution was concentrated and purified by high-performance liquid chromatography to obtain the colorless oily compound 3 (35 mg, yield: 38.0%). (MC20-1207-099).

**¹H NMR (400 MHz, CDCl3) δ:**0.82-0.94 (m, 12H), 1.03-1.35 (m, 59H), 1.35-1.49 (m, 5H), 1.49-1.71 (m, 15H), 2.19-2.29 (m, 6H), 2.29-2.38 (m, 4H), 2.43-2.54 (m, 3H), 2.94-3.09 (m, 2H), 3.12-3.20 (m, 1H), 3.33-3.45 (m, 1H), 3.84-3.93 (m, 1H), 3.97 (d, *J=* 6.0 Hz, 3H), 4.32-4.47 (m, 1H).

**LCMS:** Rt: 1.900 min; MS m/z (ESI): 948.8 [M+H]⁺.

### Step 5: Synthesis of compound SM2

A compound SM2-1 (2.0 g, 7.394 mmol, 1.0 eq.), a compound SM10 (2.2 g, 11.09 mmol, 1.5 eq.), and TsOH (500 mg) were added to toluene (20 mL), and stirred to reflux for 2 hours. TLC showed that the reaction was complete. After being concentrated, the mixed solution was purified by column chromatography to obtain the yellow oily compound SM2 (3 g, yield: 90.90%). (MC20-1195-029)

### Example 4: Synthesis of compound 4.

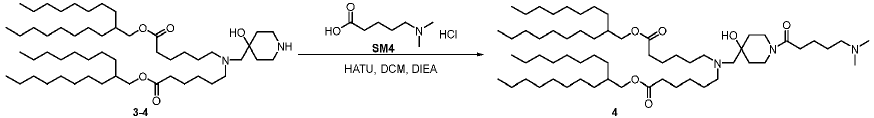

### Synthesis of compound 4

A compound 3-4 (80 mg, 0.09 mmol, 1.0 eq.), a compound SM4 (18 mg, 0.10 mmol, 1.1 eq.), HATU (42 mg, 0.11 mmol, 1.2 eq.), and DIEA (36 mg, 0.27 mmol, 3.0 eq.) were added to methylene chloride (3 mL), and stirred for 1 hour at room temperature. The mixture was concentrated and purified by high-performance liquid chromatography to obtain the colorless oily compound 4 (26 mg, yield: 28.3%). (MC20-1207-101)

**¹H NMR (400 MHz, CDCl3) δ:**0.81-0.93 (m, 12H), 1.08-1.35 (m, 60H), 1.35-1.74 (m, 23H), 2.18-2.40 (m, 14H), 2.44-2.57 (m, 3H), 2.91-3.03 (m, 1H), 3.36-3.48 (m, 1H), 3.60-3.71 (m, 1H), 3.97 (d, *J* = 6.0 Hz, 3H), 4.36-4.48 (m, 1H).

**LCMS:** Rt: 1.900 min; MS m/z (ESI): 990.9 [M+H]⁺.

### Exmaple 5: Synthesis of compound 5.

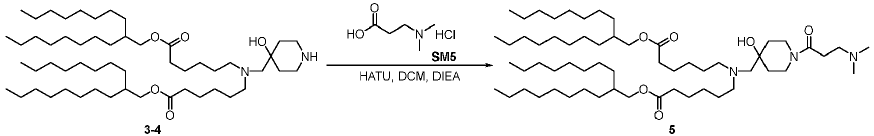

### Synthesis of compound 5

A compound 3-4 (80 mg, 0.09 mmol, 1.0 eq.), a compound SM5 (16 mg, 0.11 mmol, 1.2 eq.), HATU (42 mg, 0.11 mmol, 1.2 eq.), and DIEA (35 mg, 0.27 mmol, 3.0 eq.) were added to methylene chloride (3 mL), and stirred for 1 hour at room temperature. The mixture was concentrated and then purified by high-performance liquid chromatography to obtain the yellowish oily compound 5 (37 mg, yield: 40.0%). (MC20-1207-110).

**¹H NMR (400 MHz, CDCl3) δ:**0.80-0.94 (m, 12H), 1.12-1.35 (m, 57H), 1.36-1.49 (m, 5H), 1.50-1.69 (m, 17H), 1.91-2.06 (m, 1H), 2.16-2.38 (m, 10H), 2.43-2.57 (m, 5H), 2.61-2.71 (m, 1H), 2.92-3.03 (m, 1H), 3.36-3.50 (m, 1H), 3.60-3.73 (m, 1H), 3.97 (d, *J=* 6.0 Hz, 3H), 4.36-4.48 (m, 1H).

**LCMS:** Rt: 1.890 min; MS m/z (ESI): 962.9 [M+H]⁺.

### Example 6: Synthesis of compound 6.

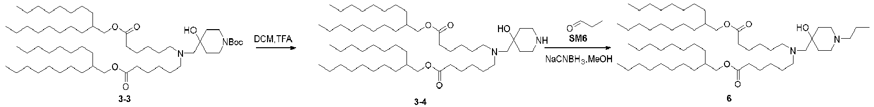

### Step 1: Synthesis of compound 3-4

A compound 3-3 (500 mg, 0.57 mmol, 1.0 eq.) was dissolved in dichloromethane (6 mL), TFA (1 mL) was added and stirred for 1 hour at room temperature, and TLC showed that the reaction was complete. The reaction solution was quenched with water, extracted with ethyl acetate, and then concentrated to obtain the yellow oily compound 3-4 (412 mg, yield: 84%). (MC21-151-055)

### Step 2: Synthesis of compound 6

A compound 3-4 (200 mg, 0.23 mmol, 1.0 eq.), and a compound SM6 (14 mg, 0.23 mmol, 1.0 eq.) were dissolved in methanol (10 mL); 1 drop of acetic acid was added, and stirred for 5 hours at room temperature; and reagent NaCNBH₃ (20 mg) was added to the mixture and stirred for 1 hour. LCMS showed that the reaction was completed. After concentration, it was purified by high-performance liquid chromatography to obtain the yellow oily compound 6 (58 mg, yield: 28%).

**¹H NMR (400 MHz, CDCl₃):**0.81-0.91 (m, 12H), 0.92-1.00 (m, 3H), 1.15-1.35 (m, 62H), 1.36-1.51 (m, 5H), 1.52-1.71 (m, 11H), 1.73-1.91 (m, 2H), 2.23-2.36 (m, 4H), 2.37-2.43 (m, 2H), 2.44-2.55 (m, 7H), 3.91-4.02 (m, 4H).

**LCMS:** Rt: 2.080 min; MS m/z (ESI): 905.9 [M+H] ⁺.

### Example 7: Synthesis of compound 7

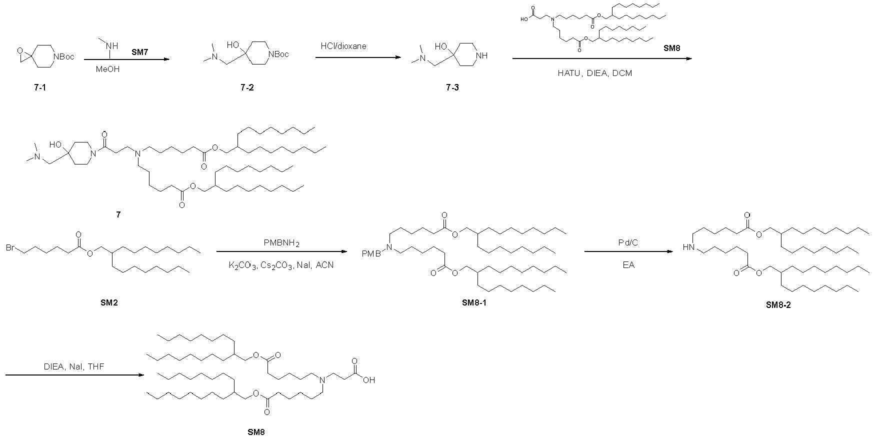

### Step 1: Synthesis of compound 7-2

A compound 7-1 (1.0 g, 4.7 mmol, 1.0 eq.) and a compound SM7 (11.7 mL, 23.5 mmol, 5.0 eq. 2M in THF) were dissolved in ACN (10.0 mL), and stirred for 16 hours at room temperature. LCMS showed that the reaction was completed, and the mixture was concentrated to obtain the yellow oily compound 7-2 (1.2 g, crude). (MC20-1234-065)

**LCMS:** Rt: 0.733 min; MS m/z (ESI):259.2 [M+H] ⁺.

### Step 2: Synthesis of compound 7-3

A compound 7-2 (1.2 g, 40.65 mmol, 1.0 eq.) was dissolved in methylene chloride (10.0 mL), dioxane hydrochloride (10.0 mL) was added at 0°C, and stirred for 16 hours. LCMS showed that the reaction was completed, and the mixture was concentrated to obtain the brown solid compound 7-3 (1.0 g, crude). (MC20-1234-067)

**LCMS:** Rt: 0.278 min; MS m/z (ESI):159.2 [M+H] ⁺.

### Step 3: Synthesis of compound 7

A compound 7-3 (200.0 mg, 1.26 mmol, 1.0 eq.) and a compound SM8 (1.0 g, 1.26 mmol, 1.0 eq.) were dissolved in methylene chloride (10.0 mL), DIEA (812.0 mg, 6.3 mmol, 5.0 eq.) and HATU (625.0 mg, 1.64 mmol, 1.3 eq.) were added at 0°C, and stirred for 16 hours at room temperature. LCMS showed that the reaction was completed, and the mixture was concentrated and purified by high-performance liquid chromatography to obtain the yellow oily compound 7 (20.0 mg, yield: 2%). (MC20-1234-079).

**¹H NMR (400 MHz, CDCl3) δ:**0.86-0.90 (m, 12H), 1.14-1.36 (m, 63H), 1.56-1.68 (m, 13H), 2.28-2.38 (m, 6H), 2.47 (s, 6H), 2.75 (s, 5H), 3.00-3.06 (m, 3H), 3.38-3.50 (m, 1H), 3.65-3.71 (m, 1H), 3.95-3.97 (m, 4H), 4.34-4.44 (m, 1H).

**LCMS:** Rt: 0.530 min; MS m/z (ESI): 962.8 [M+H]⁺.

### Step 4: Synthesis of compound SM8-1

A compound SM2 (2 g, 4.469 mmol, 3.0 eq.) was dissolved in acetonitrile (30 mL); PMBNH₂ (200 mg, 1.490 mmol, 1.0 eq.), K₂CO₃ (0.62 g, 4.469 mmol, 3.0 eq.), Cs₂CO₃ (150 mg, 0.4469 mmol, 0.3 eq.), and NaI (22 mg, 0.1490 mmol, 0.1 eq.) were added; and the reaction mixture was stirred at 85°C for 16 hours. Thin layer chromatography showed that the reaction was completed. After concentration, it was purified to obtain the yellow oily compound SM8-1 (1.1 g, yield: 84.84%). (MC20-1144-082)

### Step 5: Synthesis of compound SM8-2

A compound SM8-1 (1.1 g, 1.264 mmol) was dissolved in ethyl acetate (40 mL) under hydrogen protection, and Pd/C (200 mg) was added. The reaction mixture was stirred at 40°C for 16 hours. Thin layer chromatography showed that the reaction was completed. After filtration with Celite pads, it was rinsed with ethyl acetate, and concentrated and purified by silica gel column chromatography to obtain the yellow oily compound SM8-2 (0.9 g, yield: 94.91%). (MC20-1195-083)

### Step 6: Synthesis of compound SM8

SM8-2 (434 mg, 0.58 mmol, 1.0 eq.), 3-bromopropionic acid (266 mg, 1.74 mmol, 3.0 eq.), DIEA (374 mg, 2.90 mmol, 5.0 eq.), and NaI (44 mg, 0.29 mmol, 0.5 eq.) were added to tetrahydrofuran (20 ml); and the mixture was stirred overnight at 70°C. The mixture was concentrated to obtain the white solid compound SM8 (821 mg, crude). (MC20-1207-115)

**LCMS:** Rt: 1.547 min; MS m/z (ESI): 822.7 [M+H]⁺.

### Example 8: Synthesis of compound 8.

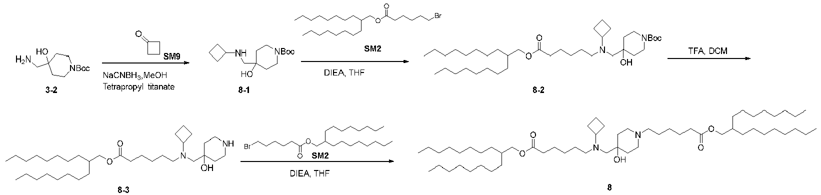

### Step 1: Synthesis of compound 8-1

A compound 8-1 (460.0 mg, 2.0 mmol, 1.0 eq.) and a compound SM9 (168.0 mg, 2.4 mmol, 1.2 eq.) were dissolved in methanol (10.0 mL); Tetrapropyl titanate (804.0 mg, 3.0 mmol, 1.5 eq.) was added at room temperature; the mixture was stirred at 50°C for 2 hours; then NaCNBH₃ (136.0 mg, 4.0 mmol, 2.0 eq.) was added; and the mixture was stirred at 50°C for 16 hours. LCMS showed that the reaction was completed, water (20.0 mL) was added; and after concentration, column purification was carried out (DCM/MeOH = 1/0-10/1) to obtain a yellow oily compound 8-1 (0.4 g, crude). (MC20-1234-074)

**LCMS:** Rt: 0.703 min; MS m/z (ESI): 285.2 [M+H] ⁺.

### Step 2: Synthesis of compound 8-2

A compound 8-1 (0.4 g, 1.4 mmol, 1.0 eq.) and a compound SM2 (628.0 mg, 1.4 mmol, 1.0 eq.) were dissolved in tetrahydrofuran (10.0 mL); and DIEA (903.0 mg, 7.0 mmol, 5.0 eq.) and NaI (20.0 mg, 0.04 mmol, 0.1 eq.) were added at 0°C. The mixture was stirred at 70°C for 16 hours. LCMS showed that the reaction was completed; and after concentration, column purification was carried out (DCM/MeOH = 1/0-20/1) to obtain the yellow oily compound 8-2 (0.2 g, yield: 22 %) (MC20 - 1234 - 077)

**LCMS:** Rt: 0.942 min; MS m/z (ESI): 651.6 [M+H] ⁺.

### Step 3: Synthesis of compound 8-3

A compound 8-2 (0.2 g, 0.31 mmol, 1.0 eq.) was dissolved in methylene chloride (10.0 mL), and TFA (1.0 mL) was added at 0°C. The mixture was stirred at room temperature for 16 hours, and the LCMS showed that the reaction was complete; and the mixture was concentrated to obtain the brown oily compound 8-3 (0.19 g, crude). (MC20-1234-078)

**LCMS:** Rt: 0.808 min; MS m/z (ESI): 551.5 [M+H] ⁺.

### Step 4: Synthesis of compound 8

A compound 8-3 (190.0 mg, 0.345 mmol, 1.0 eq.) and a compound SM2 (154.0 mg, 0.345 mmol, 1.0 eq.) were dissolved in tetrahydrofuran (10.0 mL); and DIEA (222.0 mg, 1.73 mmol, 5.0 eq.) and NaI (5.0 mg, 0.034 mmol, 0.1 eq.) were added at 0°C. The mixture was stirred at 70°C for 16 hours. LCMS showed that the reaction was completed; and after concentration, it was purified by high-performance liquid chromatography to obtain the yellow oily compound 8 (20.0 mg, yield: 6%). (MC20-1234-080).

**¹H NMR (400 MHz, CDCl3) δ:**0.86-0.90 (m, 12H), 1.14-1.36 (m, 62H), 1.54-1.69 (m, 17H),1.79-1.98 (m, 6H),2.27-2.36 (m, 7H), 2.50-2.54 (m, 3H), 3.33-3.35 (m, 1H), 3.95-3.97 (m, 4H).

**LCMS:** Rt: 0.524 min; MS m/z (ESI): 917.8 [M+H]⁺.

### Exmaple 9: Synthesis of compound 9.

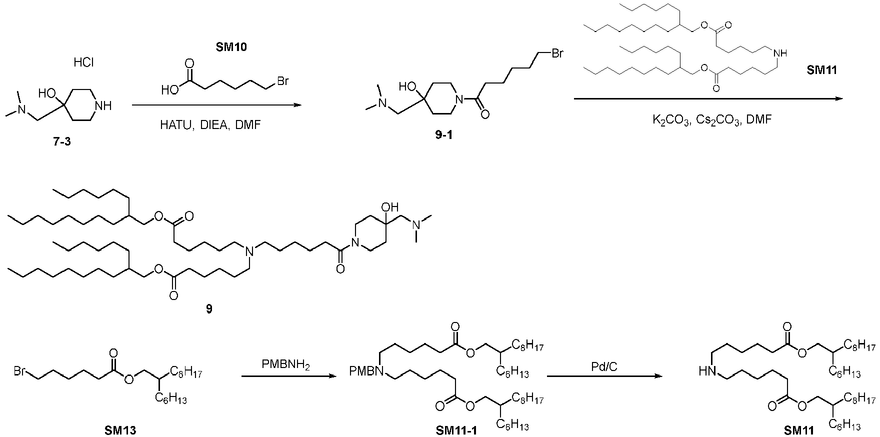

### Step 1: Synthesis of compound 9-1

A compound 7-3 (200.0 mg, 1.0 mmol, 1.0 eq.) and a compound SM10 (0.94 g, 1.0 mmol, 1.0 eq.) were dissolved in DMF (10.0 mL); and DIEA (516.0 mg, 4.0 mmol, 4.0 eq.) and HATU (494.0 mg, 1.3 mmol, 1.3 eq.) were added at 0°C. The mixture was stirred at room temperature for 16 hours; LCMS showed that the reaction was completed; and the reaction solution was concentrated, and then subjected to column purification (DCM/MeOH = 1/0-20/1) to obtain the yellow oily compound 9-1 (300.0 mg, crude). (MC20-1234-094).

**LCMS:** Rt: 0.693 min; MS m/z (ESI):335.1 [M+H] ⁺.

### Step 2: Synthesis of compound 9

A compound 9-1 (300.0 mg, 0.89 mmol, 1.0 eq.) and a compound SM11 (0.62 g, 0.89 mmol, 1.0 eq.) were dissolved in acetonitrile (10.0 mL); and K₂CO₃ (368.0 mg, 2.67 mmol, 3.0 eq.), Cs₂CO₃(86.0 mg, 0.267 mmol, 0.3 eq.) and NaI (13.0 mg, 0.089 mmol, 0.1 eq.) were added at 0°C. The mixture was stirred at 85°C for 16 hours. LCMS showed that the reaction was completed; and after concentration, it was purified by high-performance liquid chromatography to obtain the yellow oily compound 9 (21.0 mg, yield: 2.5%). (MC20-1234-096).

**¹H NMR (400 MHz, CDCl3) δ:**0.86-0.90 (m, 12H), 1.14-1.36 (m, 63H), 1.56-1.65 (m, 12H), 2.28-2.37 (m, 18H), 2.90-3.06 (m, 1H), 3.36-3.46 (m, 1H), 3.61-3.69 (m, 1H), 3.95-3.97 (m, 4H), 4.34-4.44 (m, 1H).

**LCMS:** Rt: 1.470 min; MS m/z (ESI): 948.8 [M+H]⁺.

### Step 3: Synthesis of compound SM11-1

A compound SM13 (10 g, 21.87 mmol, 1.0 eq.) was dissolved in acetonitrile (50 mL), and K₂CO₃ (3.02 g, 21.87 mmol, 1.0 eq.), Cs₂CO₃(2.38 g, 7.29 mmol, 0.3 eq.), NaI (0.2 g, 1.46 mmol, 0.07 eq.) and PMBNH₂ (1 g, 7.29 mmol, 0.3 eq.) were added. The reaction is stirred at 80°C for 10 h. The reaction mixture was poured into water (100 mL) and extracted with dichloromethane (3*100 mL). The organic phase is washed with saline, dried with anhydrous Na₂SO₄, and concentrated in vacuum. The crude was subjected to column chromatography (EtOAc:PE = 2:1) to obtain the yellow oily compound SM11-1 (5 g, yield: 84%). (MC19-89-111)

**LCMS:** Rt: 1.445 min; MS m/z (ESI): 814.6 [M+H]⁺.

### Step 4: Synthesis of compound SM11

A compound SM11-1 (5 g, 6.14 mmol, 1.0 eq.) was dissolved in ethyl acetate (100 mL); and Pd/C (1.0 g) was added, and stirred for 10 hours at room temperature under hydrogen protection. The reaction mixture was filtered and concentrated to obtain the yellow oily compound SM11 (4.0 g, yield: 94%) (MC19-89-112)

**LCMS:** Rt: 1.535 min; MS m/z (ESI): 694.6 [M+H]⁺.

### Example 10: Synthesis of compound 10.

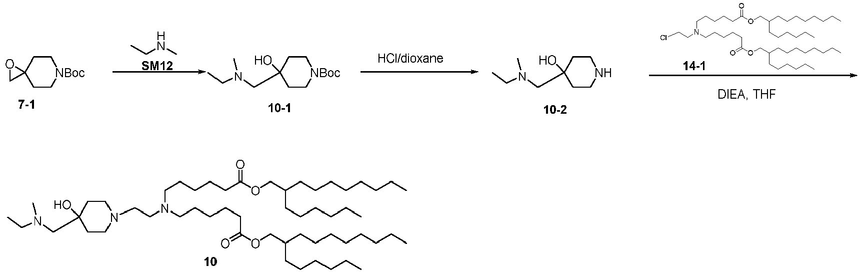

### Step 1: Synthesis of compound 10-1

A compound 7-1 (1.0 g, 4.7 mmol, 1.0 eq.) and a compound SM12 (11.7 mL, 23.5 mmol, 5.0 eq, 2M in THF) were dissolved in methanol (10.0 mL), and stirred for 16 hours at room temperature. The LCMS showed that the reaction was completed, and the mixture was concentrated to obtain the yellow oily compound 10-1 (1.2 g, crude). (MC20-1234-106)

**LCMS:** Rt: 0.714 min; MS m/z (ESI):273.2 [M+H] ⁺.

### Step 2: Synthesis of compound 10-2

A compound 10-1 (1.2 g, 4.4 mmol, 1.0 eq.) was dissolved in methylene chloride (10.0 mL); and dioxane hydrochloride (10.0 mL) was added at 0°C, and stirred for 16 hours. LCMS showed that the reaction was complete; and the mixture was concentrated to obtain the brown solid compound 10-2 (1.0 g, crude). (MC20-1234-110)

**LCMS:** Rt: 0.329 min; MS m/z (ESI):173.2 [M+H] ⁺.

### Step 3: Synthesis of compound 10

A compound 10-2 (100.0 mg, 0.57 mmol, 1.0 eq.) and a compound 14-1 (0.43 g, 0.57 mmol, 1.0 eq.) were dissolved in tetrahydrofuran (10.0 mL); and DIEA (220.0 mg, 1.71 mmol, 5.0 eq.) and NaI (8.0 mg, 0.057 mmol, 0.1 eq.) were added at 0°C. The mixture was stirred at 70°C for 16 hours. LCMS showed that the reaction was completed; and after concentration, it was purified by high-performance liquid chromatography to obtain the yellow oily compound 10 (70.0 mg, yield: 14%). (MC20-1234-111).

**¹H NMR (400 MHz, CDCl3) δ:**0.86-0.90 (m, 12H), 1.02-1.05 (m, 3H), 1.26-1.36 (m, 53H), 1.43-1.46 (m, 4H), 1.56-1.65 (m, 10H), 2.27-2.34 (m, 9H), 2.40-2.69 (m, 14H), 3.96 (d, *J=* 5.6 Hz, 4H).

**LCMS:** Rt: 1.247 min; MS m/z (ESI): 892.8 [M+H]⁺.

### Example 11: Synthesis of compound 11.

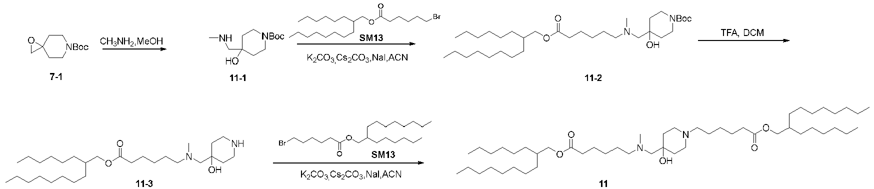

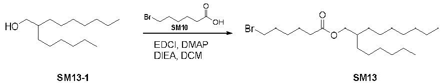

### Step 1: Synthesis of compound 11-1

A compound 7-1 (885 mg, 4.56 mmol, 1.1 eq.) and CH₃NH₂(1.0 g, 4.15 mmol, 1.0 eq.) were added to methanol (20 mL), and stirred under a sealed atmosphere at 60°C for 16 hours. LCMS showed that the reaction was completed. The mixture was concentrated and then purified by silica gel column chromatography (EA:PE = 0% ~ 5%) to obtain 11-1 as colorless oily compound (1.2 g, yield: 63%). (MC21-151-067)

**LCMS:** Rt: 0.710 min; MS m/z (ESI): 245.2 [M+H]⁺.

### Step 2: Synthesis of compound 11-2

A compound 11-1 (500 mg, 2.05 mmol, 1.0 eq.), a compound SM13 (1.03 g, 2.46 mmol, 1.1 eq.), K₂CO₃ (849 mg, 6.15 mmol, 3.0 eq.), Cs₂CO₃ (202 mg, 0.62 mmol, 0.3 eq.) and NaI (30 mg, 0.21 mmol, 0.1 eq.) were added to acetonitrile (20 mL), and stirred overnight at 85°C. The mixture was concentrated and purified by silica gel column chromatography (MeOH: DCM = 0% ~ 10%) to obtain 11-2 as yellow oily compound (1.0 g, yield: 83%). (MC21-151-068)

**LCMS:** Rt: 0.900 min; MS m/z (ESI): 583.5 [M+H]⁺.

### Step 3: Synthesis of compound 11-3

A compound 11-2 (500 mg, 0.86 mmol, 1.0 eq.) was dissolved in methylene chloride (10 mL); and TFA (2 mL) was added at room temperature, and stirred for 16 hours. LCMS showed that the reaction was completed, and the reaction solution was concentrated to obtain the yellow oily compound 11-3 (322 mg, yield: 78%). (MC21-151-072)

**LCMS:** Rt: 0.760 min; MS m/z (ESI): 483.4 [M+H]⁺.

### Step 4: Synthesis of compound 11

A compound 11-3 (320 mg, 0.66 mmol, 1.0 eq.), a compound SM13 (420 mg, 1.00 mmol, 1.5 eq.), K₂CO₃ (280 mg, 2.00 mmol, 3.0 eq.), Cs₂CO₃ (65 mg, 0.20 mmol, 0.3 eq.) and NaI (10 mg, 0.07 mmol, 0.1 eq.) were added to acetonitrile (10 mL), and stirred overnight. It was purified by high-performance liquid chromatography to obtain the yellow oily compound 11 (83 mg, yield: 15%). (MC21-151-073)

**¹H NMR (400 MHz, CDCl3) δ:**0.82- 0.94 (m, 12H), 1.14-1.39 (m, 53H), 1.40- 1.50 (m, 3H), 1.51-1.72 (m, 13H), 2.22- 2.39 (m, 9H), 2.40-2.53 (m, 5H), 2.65-2.84 (m, 1H), 3.99-3.89 (m, 4H).

**LCMS:** Rt: 1.190 min; MS m/z (ESI): 821.8 [M+H]⁺.

### Step 5: Synthesis of compound SM13

A compound SM13-1 (20.2 g, 83.3 mmol, 1.0 eq.) and a compound SM10 (19.5 g, 100 mol, 1.2 eq.) were dissolved in dichloromethane (300 mL); and EDCI (24.0 g, 125 mmol, 1.5 eq.), DMAP (2.0 g, 16.7 mmol, 0.2 eq.) and DIEA (27.0 g, 208 mmol, 2.5 eq.) were added. The mixture was stirred for 16 hours at room temperature. TLC showed that the reaction was completed. After the mixture was concentrated, it was purified by silica gel column chromatography (EA/PE=0-1%) to obtain the colorless oily compound SM13 (17 g, yield: 49%). (MC20-71-001)

### Example 12: Synthesis of compound 12.

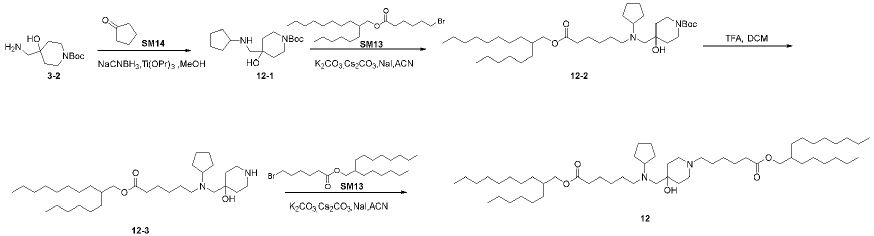

### Step 1: Synthesis of compound 12-1

A compound 3-2 (690 mg, 0.9 mmol, 1.0 eq.) was dissolved in methanol (10 mL); and a compounded SM14 (400 mg, 3.6 mmol, 1.2 eq.) and Ti(OPr)₄ (1.2 g, 4.5 mmol, 1.5 eq.) were added. The mixture was stirred to react for 5 hours at room temperature. Then NaCNBH₃ (408 mg, 6.0 mmol, 2.0 eq.) was added. The reaction was stirred to react for 10 hours at room temperature. Thin layer chromatography showed that the reaction was completed. The mixture was poured into water, and extracted with ethyl acetate, and the organic phase was separated, and dried with Na₂SO₄. After concentration, it was purified by high-performance liquid chromatography to obtain the yellow oily compound 12-1 (700 mg, yield: 78%). (MC21-151-064)

### Step 2: Synthesis of compound 12-2

A compound 12-1 (300 mg, 1.00 mmol, 1.0 eq.), a compound SM13 (420 mg, 1.00 mmol, 1.0 eq.), K₂CO₃ (414 mg, 3.00 mmol, 3.0 eq.), Cs₂CO₃(98 mg, 0.30 mmol, 0.3 eq.) and NaI (14 mg, 0.10 mmol, 0.1 eq.) were added into acetonitrile (10 mL), and stirred overnight at 85°C; and the mixture was concentrated and purified by silica gel column chromatography (MeOH:DCM = 0% ~ 10%) to obtain the yellow oily compound 12-2 (290 mg, yield: 83%). (MC21-151-066)

**LCMS:** Rt: 0.890 min; MS m/z (ESI): 637.5 [M+H]⁺.

### Step 3: Synthesis of compound 12-3

A compound 12-2 (290 mg, 0.46 mmol, 1.0 eq.) was dissolved in methylene chloride (10 mL); and TFA (2 mL) was added at room temperature, and stirred for 16 hours. LCMS showed that the reaction was completed, and the reaction solution was concentrated to obtain the yellow oily compound 12-3 (290 mg, yield: 80%). (MC21-151-072)

**LCMS:** Rt: 0.760 min; MS m/z (ESI): 537.4 [M+H]⁺.

### Step 4: Synthesis of compound 12

A compound 12-3 (290 mg, 0.54 mmol, 1.0 eq.), SM13 (273 mg, 0.65 mmol, 1.2 eq.), K₂CO₃ (222 mg, 1.59 mmol, 3.0 eq.), Cs₂CO₃(52 mg, 0.16 mmol, 0.3 eq.) and NaI (7 mg, 0.05 mmol, 0.1 eq.) were added to acetonitrile (10 mL), and stirred at 85°C overnight. It was purified by high-performance liquid chromatography to obtain the yellow oily compound 12 (96 mg, yield: 20%). (MC21-151-071)

**¹H NMR (400 MHz, CDCl3) δ:**0.78- 0.93 (m, 12H), 1.17- 1.40 (m, 54H), 1.41- 1.81 (m, 22H), 2.22- 2.45 (m, 9H), 2.46- 2.58 (m, 2H), 2.59-2.77 (m, 2H), 3.08-3.28 (m, 1H), 3.90-4.03 (m, 4H).

**LCMS:** Rt: 1.240 min; MS m/z (ESI): 875.8 [M+H]⁺.

### Example 13: Synthesis of compound 13.

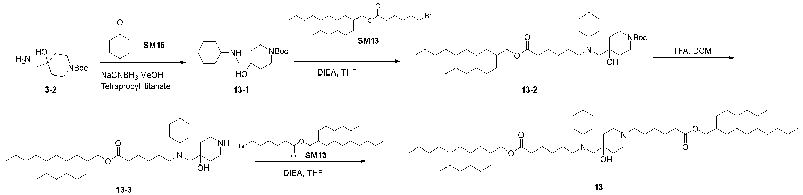

### Step 1: Synthesis of compound 13-1

A compound 3-2 (690.0 mg, 3.0 mmol, 1.0 eq.) and a compound SM15 (353.0 mg, 3.6 mmol, 1.2 eq.) were dissolved in methanol (10.0 mL); tetrapropyl titanate (1.2 g, 4.5 mmol, 1.5 eq.) was added at room temperature; the mixture was stirred at 50°C for 2 hours; and then NaCNBH₃ (408.0 mg, 6.0 mmol, 2.0 eq.)was added, and stirred at 50°C for 16 hours. The LCMS showed that the reaction was completed, H₂O (20.0 mL) was added, the mixture was concentrated and then purified by silica gel column chromatography (DCM/MeOH = 1/0-10/1) to obtain the yellow oily compound 13-1 (0.9 g, crude). (MC20-1234-081)

**LCMS:** Rt: 0.720 min; MS m/z (ESI): 313.2 [M+H] +.

### Step 2: Synthesis of compound 13-2

A compound 13-1 (0.9 g, 2.88 mmol, 1.0 eq.) and a compound SM13 (1.2 g, 2.88 mmol, 1.0 eq.) were added into THF (10.0 mL); and DIEA (1.8 g, 14.4 mmol, 5.0 eq.) and NaI (42.0 mg, 0.29 mmol, 0.1 eq.) were added at 0°C. The mixture was stirred at 70°C for 16 hours. LCMS showed that the reaction was completed, and the mixture was concentrated and then purified by silica gel column chromatography (DCM/MeOH = 1/0-20/1) to obtain the yellow oily compound 13-2 (0.6 g, yield: 32%). (MC20-1234-086)

### Step 3: Synthesis of compound 13-3

A compound 13-2 (0.48 g, 0.74 mmol, 1.0 eq.) was dissolved in methylene chloride (10.0 mL); and TFA (2.0 mL) was added at 0°C. The mixture was stirred at room temperature for 16 hours; the LCMS showed that the reaction was completed; and it was concentrated to obtain the brown oily compound 13-3 (0.42 g, crude). (MC20-1234-088)

**LCMS:** Rt: 0.794 min; MS m/z (ESI): 551.5 [M+H] ⁺.

### Step 4: Synthesis of compound 13

A compound 13-3 (200.0 mg, 0.36 mmol, 1.0 eq.) and a compound SM13 (228.0 mg, 0.54 mmol, 1.5 eq.) were dissolved in acetonitrile (15.0 mL); and K₂CO₃(149 mg, 1.08 mmol, 3.0 eq.), Cs₂CO₃(35.0 mg, 0.11 mmol, 0.3 eq.), and NaI (5.0 mg, 0.036 mmol, 0.1 eq.) were added at room temperature. The mixed solution was stirred at 80°C for 16 hours. LCMS showed that the reaction was completed, and the reaction solution was concentrated and then purified by high-performance liquid chromatography to obtain the colorless oily compound 13 (27.0 mg, yield: 8%). (MC20-30-090).

**¹H NMR (400 MHz, CDCl3) δ:** 0.86-0.90 (m, 12H), 1.14-1.36 (m, 59H), 1.45-1.70 (m, 22H), 2.21-2.41 (m, 11H), 3.89-3.90 (m, 4H).

**LCMS:** Rt:1.550 min; MS m/z (ESI): 889.8 [M+H]⁺.

### Example 14: Synthesis of compound 14.

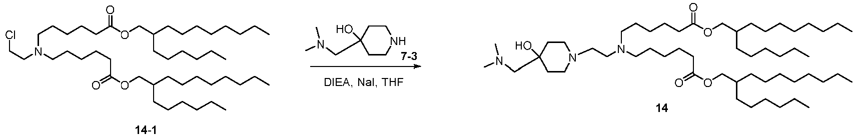

### Synthesis of compound 14

A compound 14-1 (0.75 g, 1.0 mmol, 1.0 eq.) and a compound 7-3 (0.2 g, 1.0 mmol, 1.0 eq.) were added into THF (10.0 mL); and DIEA (0.64 g, 5.0 mmol, 5.0 eq.) and NaI (14.6 mg, 0.1 mmol, 0.1 eq.) were added at 0°C. The mixture was stirred at 70°C for 16 hours. LCMS showed that the reaction was completed, and the reaction solution was concentrated and then purified by high-performance liquid chromatography to obtain the brown oily compound 14 (57.0 mg, yield: 6%). (MC20-1234-102)

**¹H NMR (400 MHz, CDCl3) δ:**0.86-0.90 (m, 12H), 1.26-1.32 (m, 53H), 1.43-1.45 (m, 4H), 1.59-1.65 (m, 10H), 2.28-2.32 (m, 6H), 2.36 (s, 6H), 2.41-2.61 (m, 12H), 3.96-3.97 (m, 4H).

**LCMS:** Rt: 1.580 min; MS m/z (ESI): 878.8 [M+H] ⁺.

### Example 15: Synthesis of compound 15.

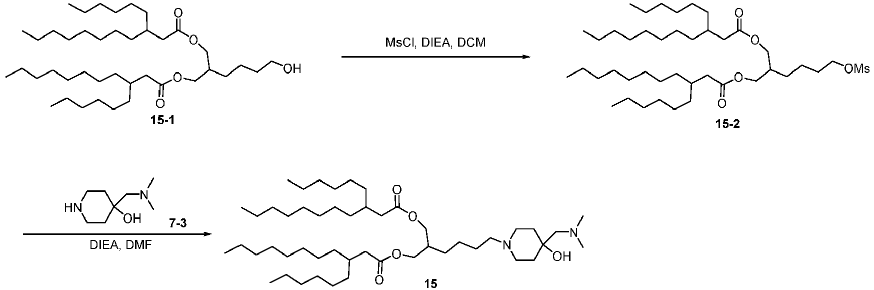

### Step 1: Synthesis of compound 15-2

A compound 15-1 (200 mg, 0.27 mmol, 1.0 eq.), MsCl (38 mg, 0.33 mmol, 1.2 eq.), and DIEA (106 mg, 0.82 mmol, 3.0 eq.) were added into methylene chloride (5 mL), and stirred for 1 hour. The mixture was quenched with water, and extracted with ethyl acetate, and the organic phase was dried and concentrated to obtain the yellow oily compound 15-2 (306 mg, crude product). (MC20-364-028)

### Step 2: Synthesis of compound 15

A compound 15-2 (137 mg, 0.19 mmol, 1.0 eq.), a compound 7-3 (36 mg, 0.22 mmol, 1.2 eq.), and DIEA (73 mg, 0.56 mmol, 3.0 eq.) were added to DMF (5 mL), and stirred at 80°C overnight. The mixture was diluted with water, extracted with ethyl acetate, washed with brine, dried over anhydrous sodium sulfate, and filtered; and the filtrate was concentrated and then purified by high-performance liquid chromatography to obtain the colorless oily compound 15 (19 mg, yield: 12.8%). (MC21-364-033)

**¹H NMR (400 MHz, CDCl3) δ:**0.82-0.92 (m, 12H), 1.19-1.32 (m, 56H), 1.33-1.38 (m, 3H), 1.76-1.87 (m, 2H), 1.93-2.07 (m, 2H), 2.18-2.29 (m, 6H), 2.30-2.38 (m, 9H), 2.58-2.68 (m, 2H), 3.97-4.11 (m, 4H).

**LCMS:** Rt: 0.080 min; MS m/z (ESI): 793.6 [M+H]⁺.

### Example 16: Preparation and characterization of lipid nanoparticles

Briefly, the cationic lipid, DSPC, cholesterol and PEG-lipid provided by the present invention were dissolved in ethanol at a molar ratio of 50:10:38.5:1.5, and mRNA was diluted in a citrate buffer at 10-50 mM, pH=4. The ethanolic lipid solution and the mRNA aqueous solution were mixed at a volume ratio of 1:3 by a microfluidic device at a flow rate of 9-30 mL/min, so as to prepare LNP at a weight ratio of total lipid to mRNA of about 10:1 to 30:1. PBS for dialysis was used instead of ethanol, thereby removing ethanol. Finally, the lipid nanoparticles were filtered through a 0.2µm sterile filter.

The size of the lipid nanoparticles was determined by dynamic light scattering using 173° backscatter detection mode of Malvern Zetasizer Nano ZS (Malvern UK). According to the description of the manufacturer, the encapsulation efficiency of the lipid nanoparticle was determined using a Quant-it Ribogreen RNA quantitative assay kit (Thermo Fisher Scientific, UK).

As reported in the literatures, the apparent pKa of LNP formulations was correlated with the LNP delivery efficiency of nucleic acids in vivo. The apparent pKa of each formulation was determined using a 2-(p-methylphenyl)-6-naphthalenesulfonic acid (TNS)-based fluorescence assay. The LNP formulation comprising cationic lipids/DSPC/cholesterol/DMG-PEG (50/10/38.5/1.5 mol%) was prepared as described above. TNS was made into a 300 uM distilled water stock solution. The LNP formulation was diluted to 0.1 mg/mL of total lipids in 3 mL of a buffer solution including 50 mM of sodium citrate, 50 mM of sodium phosphate, 50 mM of sodium borate, and 30 mM of sodium chloride, with the pH being 3 to 9 A TNS solution was added to reach a final concentration of 0.1 mg/mL; and after vortex mixing, the fluorescence intensity was measured at room temperature in a Molecular Devices Spectramax iD3 spectrometer using excitation wavelengths of 325 nm and 435 nm. The fluorescence data was subjected to a sigmoid best fit analysis, and the pKa value was measured when the pH reaches half of the maximum fluorescence intensity.

### Example 17: Research on animal

Lipid nanoparticles including human erythropoietin (hEPO) mRNA encapsulated by compound in the following table were administered at a dose of 0.5mg/kg to female ICR mice at 6-8 weeks of age (Xipuer-Bikai, Shanghai) via tail vein injection. Mouse blood samples were taken at specific time points (e.g., 6 hours) after administration. In addition to the above test groups, the lipid nanoparticles including dilinoleylmethyl-4-dimethylaminobutyrate (DLin-MC3-DMA, commonly abbreviated as MC3) encapsulated hEPO mRNA were administered at the same dose to mice of similar age and sex as positive controls.

After the last sampling time point, the mice were euthanized by excess CO₂. Serum was separated from whole blood by centrifugation at 5,000g for 10 minutes at 4°C, snap frozen and stored at -80°C for analysis. ELSA analysis was performed using a commercially available kit (DEP00, R&D system) according to the manufacturer instructions.

The following table lists the characteristics of the tested lipid nanoparticles, including the expression levels measured from the test group above MC3.

**Table 2.**

| Compound | Size (nm) | Polydispersity index (PDI) | Encapsulation efficiency (EE%) | EPO expression relative to MC3 |
|---|---|---|---|---|
| 1 | 68.14 | 0.257 | 99.1% | Not detected |
| 2 | 80.02 | 0.114 | 98.1% | Not detected |
| 3 | 78.45 | 0.248 | 97.3% | Not detected |
| 4 | 55.63 | 0.28 | 98.4% | Not detected |
| 5 | 44.61 | 0.167 | 97.7% | Not detected |
| 6 | 83.23 | 0.034 | 92.8% | A |
| 7 | 43.78 | 0.073 | 97.6% | Not detected |
| 8 | 54.23 | 0.16 | 93.7% | C |
| 9 | 35.26 | 0.212 | 97.6% | Not detected |
| 10 | 133.3 | 0.083 | 96.7% | Not detected |
| 11 | 86.19 | 0.045 | 96.8% | B |
| 12 | 109.1 | 0.084 | 81.5% | C |
| 13 | 117.7 | 0.049 | 83.9% | C |
| 14 | 97.33 | 0.139 | 97.3% | Not detected |
| 15 | 53.67 | 0.06 | 94.3% | Not detected |

| | | | | |
|---|---|---|---|---|
| A: ≥2 B: ≥1 and <2 C: ≥0.1 and <1 D:<0.1. | | | | |

## Claims

1. A compound represented by Formula (I): 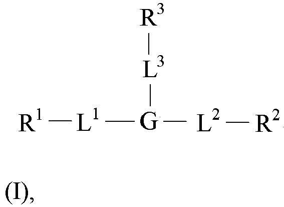 or pharmaceutically acceptable salt or stereoisomer thereof, wherein:
Gis N or CH;
L¹, L² and L³ each are independently selected from bonds or C₁-C₈ alkylene;
R¹ is selected from -C(O)OR⁴ or -OC(O)R⁹;
R² is selected from -C(O)OR⁵, C₃-C₈ cycloalkyl, C₁-C₈ alkyl, -OC(O)R¹⁰, -C(O)NHC₁₀-C₂₀ alkyl or -NHC(O)C₁₀-C₂₀ alkyl;
R³ is selected from 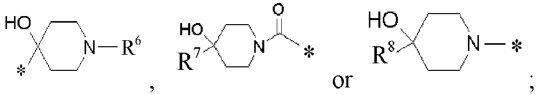 R⁴ is C₁₀-C₂₀ alkyl;
R⁵ is C₁₀-C₂₀ alkyl;
R⁶ is selected from -C(O)(CH₂)₁₋₈N(C₁-C₈ alkyl)C₁-C₈ alkyl, C₁-C₈ alkyl or -(CH₂)₁₋₈C(O)OR¹¹;
R⁷ is -(CH₂)₁₋₈N(C₁-C₈ alkyl)C₁-C₈ alkyl;
R⁸ is -(CH₂)₁₋₈N(C₁-C₈ alkyl)C₁-C₈ alkyl;
R⁹ is C₁₀-C₂₀ alkyl;
R¹⁰ is C₁₀-C₂₀ alkyl;
R¹¹ is C₁₀-C₂₀ alkyl; and
* denotes a ligation site.

2. The compound or pharmaceutically acceptable salt or stereoisomer thereof according to claim 1, wherein
GisN;
L¹, L² and L³ each are independently C₁-C₈ alkylene;
R¹ is -C(O)OR⁴;
R² is -C(O)OR⁵;
R³ is 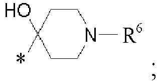
R⁴ is C₁₀-C₂₀ alkyl;
R⁵ is C₁₀-C₂₀ alkyl;
R⁶ is selected from -C(O)(CH₂)₁₋₈N(C₁-C₈ alkyl), C₁-C₈ alkyl or C₁-C₈ alkyl; and
* denotes a ligation site.

3. The compound or pharmaceutically acceptable salt or stereoisomer thereof according to claim 1, wherein
GisN;
L¹, L² and L³ each are independently C₁-C₈ alkylene;
R¹ is -C(O)OR⁴;
R² is -C(O)OR⁵;
R³ is selected from 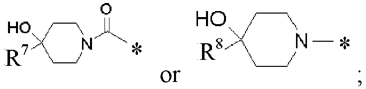
R⁴ is C₁₀-C₂₀ alkyl;
R⁵ is C₁₀-C₂₀ alkyl;
R⁷ is (CH₂)₁₋₈N(C₁-C₈ alkyl)C₁-C₈ alkyl;
R⁸ is -(CH₂)₁₋₈N(C₁-C₈ alkyl)C₁-C₈ alkyl; and
* denotes a ligation site.

4. The compound or pharmaceutically acceptable salt or stereoisomer thereof according to claim 1, wherein
GisN;
L¹ and L³ each are independently C₁-C₈ alkylene;
L² is a linkage;
R¹ is -C(O)OR⁴;
R² is selected from C₃-C₈cycloalkyl or C₁-C₈ alkyl;
R³ is 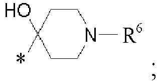
R⁴ is C₁₀-C₂₀ alkyl;
R⁶ is -(CH₂)₁₋₈C(O)OR¹¹;
R¹¹ is C₁₀-C₂₀ alkyl; and
* denotes a ligation site.

5. The compound or pharmaceutically acceptable salt or stereoisomer thereof according to claim 1, wherein
G is CH;
L¹, L² and L³ each are independently C₁-C₈ alkylene;
R¹ is -OC(O)R⁹;
R² is -OC(O)R¹⁰;
R³ is 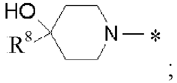
R⁸ is -(CH₂)₁₋₈N(C₁-C₈ alkyl)C₁-C₈ alkyl;
R⁹ is C₁₀-C₂₀ alkyl;
R¹⁰ is C₁₀-C₂₀ alkyl; and
* denotes a ligation site.

6. The compound or pharmaceutically acceptable salt or stereoisomer thereof according to any one of claims 1 to 5, wherein R⁴, R⁵, R⁹, R¹⁰ and R¹¹ are each independently selected from: - (CH₂)₁₋₃-CH(C₅-C₁₀ alkyl) (C₅-C₁₀ alkyl).

7. The compound or pharmaceutically acceptable salt or stereoisomer thereof according to 1, wherein the compound is selected from: 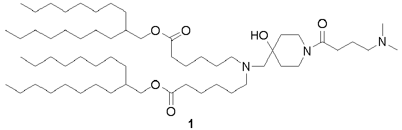 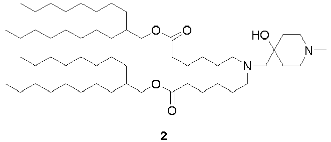 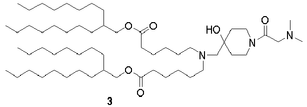 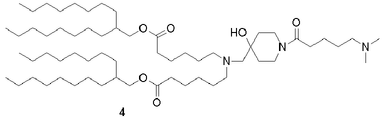 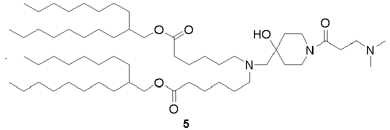 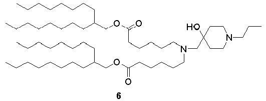 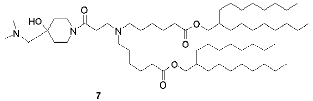 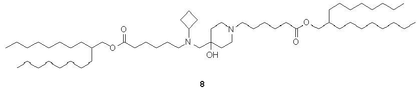 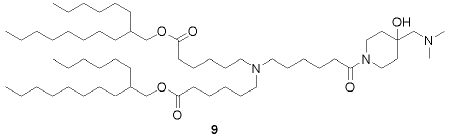 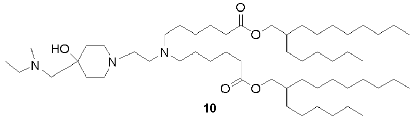 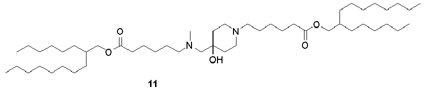 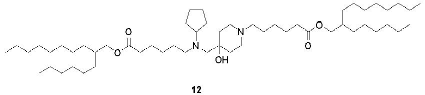 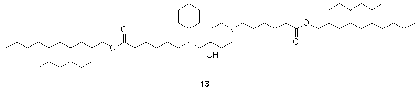 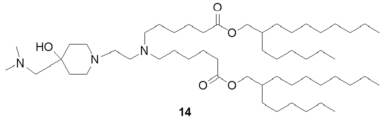 or

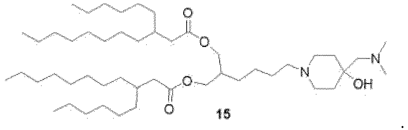

8. A composition, comprising the compound according to any one of claims 1 to 7 and a therapeutic or prophylactic agent, wherein preferably, the composition further comprises one or more structural lipids, preferably, the one or more structural lipids are DSPCs, more preferably, the molar ratio of the compound to the structural lipids is in a range of about 2:1 to about 8:1; preferably, the composition further comprises a steroid, preferably, the steroid is cholesterol, more preferably, the molar ratio of the compound to the steroid is in the range of about 5:1 to about 1:1; preferably, the composition further comprises one or more polymer-conjugated lipids, preferably, the polymer-conjugated lipids are DMG-PEG2000 or DMPE-PEG2000, and more preferably, the molar ratio of the compound to the polymer-conjugated lipids is in a range of about 100:1 to about 20:1; preferably, the therapeutic or prophylactic agent comprises at least one mRNA encoding an antigen or a fragment or epitope thereof; preferably, the mRNA is a monocistronic mRNA or a polycistronic mRNA; preferably, the antigen is a pathogenic antigen; preferably, the antigen is a tumor-associated antigen, preferably, the mRNA comprises one or more functional nucleotide analogs; and preferably, the functional nucleotide analogs are one or more selected from pseudouridine, 1-methyl-pseudouridine and 5-methylcytosine.

9. Lipid nanoparticles comprising the compound according to any one of claims 1 to 7 or the composition according to claim 8.

10. A pharmaceutical composition, comprising the compound according to any one of claims 1 to 7, the composition according to claim 8, or the lipid nanoparticlc according to claim 9 and a pharmaceutically acceptable excipient or diluent.
